# EUROPEAN PATENT APPLICATION

(11) **EP 4 066 895 A1**
(43) Date of publication of application: **05.10.2022**
(21) Application number: 20892362.3
(22) Date of filing: 27.11.2020
(51) Int. Cl.: A61P 7/00, A61P 43/00, C07D 207/34, C07D 409/04, C07D 409/12, C07D 471/04, C07D 495/04, C07D 513/04, C07D 209/42, C07D 209/52, A61K 31/401, A61K 31/403, A61K 31/4045, A61K 31/407, A61K 31/429, A61K 31/437

(54) **COMPOUND HAVING LYSOPHOSPHATIDIC ACID RECEPTOR AGONISTIC ACTIVITY AND PHARMACEUTICAL USE OF SAID COMPOUND**

(30) Priority: 29.11.2019 JP 2019216336
(71) Applicant: ONO Pharmaceutical Co., Ltd., Osaka-shi, Osaka 541-8526 (JP)
(72) Inventor: IWAKI, Yuzo, Mishima-gun, Osaka 618-8585 (JP); ENDO, Toshimitsu, Mishima-gun, Osaka 618-8585 (JP); KUSUMI, Kensuke, Mishima-gun, Osaka 618-8585 (JP)
(74) Representative: J A Kemp LLP
(86) International application number: PCT/JP2020/044318
(87) International publication number: WO 2021/107125

(57) **Abstract**

An object of the present invention is to provide a pharmaceutical containing a compound having an agonist activity for LPA3 as an active ingredient. As a result of intensive studies, the inventors of the present invention have found a compound represented by the following general formula (IY): 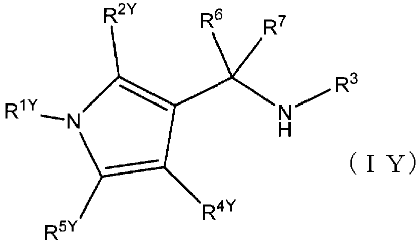 wherein all symbols represent the same meaning as described in the specification, having an agonist activity for LPA3, as a substance capable of solving such a problem and the like, and completed the present invention. Since the compound represented by general formula (IY) of the present invention and the like have an agonist activity for LPA3, it can be used as an active ingredient of a preventive and/or therapeutic agent for essential thrombocythemia or reactive thrombocytosis.

## Description

### TECHNICAL FIELD

The present invention relates to a compound represented by general formula (IY) described later, or a salt thereof, which has an agonist activity for a lysophosphatidic acid receptor, particularly a lysophosphatidic acid receptor subtype 3 (hereinafter, may be abbreviated as LPA3.), and a pharmaceutical use thereof.

### BACKGROUND ART

Lysophosphatidic acid (LPA) is a physiologically active lipid produced by phosphatidic acid (PA) present in a cell membrane or lysophosphatidylcholine (LPC) present extracellularly, and exhibits various physiological activities by acting on receptors of G protein-coupled receptors (GPCRs) (LPA1 to LPA3) belonging to an endothelial differentiation gene (EDG) type receptor family and GPCRs (LPA4 to LPA6) belonging to a purine receptor family (P2Y receptor family). LPA3 is a molecule identified as a third LPA receptor in 1999, and has been reported to be involved in platelet differentiation. In addition, Patent Literature 1 describes that a compound having LPA3 receptor agonist activity can be used as a drug for preventing and/or treating diseases in which platelets increase, for example, essential thrombocythemia, reactive thrombocytosis, and the like. Furthermore, Non Patent Literature 1 describes that an LPA3 agonist is observed to improve a pathological condition in a pulmonary fibrosis model mouse.

For example, Patent Literature 2 describes that a compound represented by general formula (A): 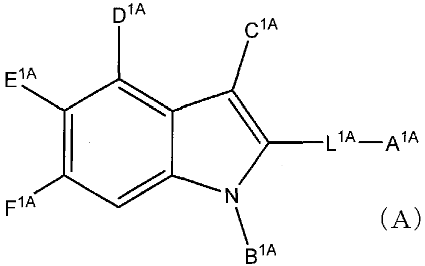 wherein L^{1A} is a bond or the like; A^{1A} is C(O)OH or a biological equivalent thereof, or the like; 1, 2, 3, 4 of B^{1A}, C^{1A}, D^{1A}, E^{1A}, and F^{1A}, or each is independently R^{1A}, R^{2A}, R^{3A}, R^{4A}, or the like, with the remainder being H, OH, CN, F, Cl, Br, or I; R^{4A} is alkyl, alkenyl, or alkynyl, each unsubstituted or substituted with 1, 2, 3, 4, or 5 independently selected R^{5A}, OR^{5A}, SR^{5A}, S(O)R^{5A}, SO₂R^{5A}, NH₂, NHR^{5a}, and the like; R^{5A} is R^{6A}, R^{7A}, R^{8A}, or the like; R^{6A} is phenyl that is unfused or fused with benzene, heteroallene or R^{6AA}; R^{7A} is heteroaryl that is unfused or fused with benzene, heteroallene or R^{7AA}; R^{8A} is cycloalkyl, cycloalkenyl, heterocyclic alkyl or heterocyclic alkenyl that is unfused or fused with benzene, heteroallene or R^{8AA}, respectively; and the required part was extracted for the definition of the group, or a therapeutically acceptable salt of the compound inhibits an activity of anti-apoptotic Mcl-1 protein, is useful for treatment of acute myeloid leukemia, cervical cancer, colorectal cancer, gastric cancer, multiple myeloma, non-Hodgkin's lymphoma, and the like.

Further, Patent Literature 3 describes that a compound represented by general formula (B): 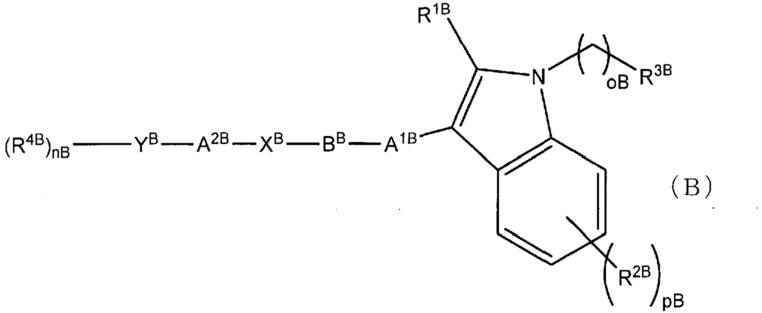 wherein A^{1B} and A^{2B} are (CH₂)_{mB} or the like; mB is 0 or an integer of 1 to 6; B^{B} is (CH₂)_{nB} or the like; nB is 0 or an integer of 1 to 6; X^{B} is NR^{5B} or the like; Y^{B} is R^{6B} or the like; oB is 0 or an integer of 1 to 3; pB is 0 or an integer of 1 to 4; R^{1B}, R^{2B}, R^{3B}, and R^{4B} are hydrogen, linear or branched alkyl having 1 to 12 carbon atoms, cycloalkyl having 3 to 6 carbon atoms, aryl, halo, C1 to C12 haloalkyl, hydroxy, C1 to C12 alkoxy, carboxy, or the like; R^{5B} is hydrogen or the like; R^{6B} is cycloalkyl having 3 to 6 carbon atoms or the like; and the required part was extracted for the definition of the group, is useful for treatment of diseases mediated by S1P receptors, such as glaucoma, dry eye, and angiogenesis.

However, the compounds disclosed in the present invention described later, or salts thereof, and pharmaceutical uses thereof are not described in any prior art.

### CITATIONS LIST

### Patent Literatures

Patent Literature 1: WO 2014/172705 A
Patent Literature 2: WO 2008/130970 A
Patent Literature 3: WO 2007/112322 A

### Non Patent Literature

Non Patent Literature 1: Program and Proceedings of the 93rd Annual Meeting of the Japan Biochemical Society, pages [P-088] (2020.09)

### SUMMARY OF INVENTION

### TECHNICAL PROBLEMS

An object of the present invention is to provide a pharmaceutical containing a compound having an agonist activity for LPA3 as an active ingredient.

### SOLUTIONS TO PROBLEMS

As a result of intensive investigations in order to solve the above problems, the present inventors have found that a compound represented by general formula (IY) or a salt thereof described later solves the above problems.

That is, the present invention relates to:
[1] A compound represented by general formula (IY): 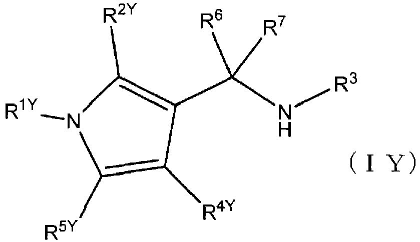 wherein R^{1Y} represents a C1 to 4 alkyl group or -CR⁸R⁹-ring 1, R⁸ and R⁹ each independently represent a hydrogen atom or a C1 to 4 alkyl group, ring 1 represents a 3 to 15-membered carbocyclic ring which may be substituted with 1 to 5 R^{13Y}s or a 3 to 15-membered heterocyclic ring which may be substituted with 1 to 5 R^{13Y}s, R^{13Y} represents halogen, a C1 to 4 alkyl group, a C1 to 4 alkoxy group, a C1 to 4 haloalkyl group, a C1 to 4 haloalkoxy group, a C3 to 6 cycloalkyl group, a hydroxyl group, a carboxyl group, an amino group, a (C1 to 4 alkyl)carbonylamino group, a (C1 to 4 alkyl)carbonyl group, a carbamoyl group, a (C1 to 4 alkyl)aminocarbonyl group, a di(C1 to 4 alkyl)aminocarbonyl group, a cyano group, a C1 to 4 alkylsulfonyl group, a C1 to 4 alkylthio group, a C1 to 4 haloalkylthio group, a nitro group, a 5 to 6-membered heterocyclic ring, or -O-(CH2)r_{Y}-R¹⁷, R¹⁷ represents a carboxyl group, a C1 to 4 alkoxycarbonyl group, -CONHSO₂R^{10Y}, -CONHR¹⁹, a sulfo group (-SO₃H group), - SO₂NHCOR²⁰, a 5-membered ring having highly acidic hydrogen, a cyano group, a di-(C1 to 4 alkyl)-hydroxymethyl group, a 3 to 15-membered carbocyclic ring which may be substituted with 1 to 3 R¹⁸s, or a 3 to 15-membered heterocyclic ring which may be substituted with 1 to 3 R¹⁸s, R¹⁸ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, an amino group, a C1 to 4 alkoxycarbonyl group, or an oxo group, a plurality of R^{13Y}s may be the same or different, r_{Y} represents an integer of 1 to 5, a plurality of R¹⁸s may be the same or different, R^{2Y} represents a carboxyl group, a C1 to 4 alkoxycarbonyl group, -CONHSO₂R^{10Y}, -CONHR¹⁹, a sulfo group (-SO₃H group), -SO₂NHCOR²⁰, or a 5-membered ring having highly acidic hydrogen, R^{10Y} represents a C1 to 4 alkyl group, a C1 to 4 haloalkyl group, a C3 to 6 cycloalkyl group, a di-(C1 to 4 alkyl)amino group, a 5 to 6-membered carbocyclic ring which may be substituted with 1 to 3 R¹⁶s, or a 5 to 6-membered heterocyclic ring which may be substituted with 1 to 3 R¹⁶s, and R¹⁶ represents halogen, a C1 to 4 alkyl group, or a C1 to 4 alkoxy group, a plurality of R¹⁶s may be the same or different, R¹⁹ represents a hydrogen atom, a hydroxyl group, a C1 to 4 alkyl group, or a C1 to 4 alkoxy group, R²⁰ represents a C1 to 4 alkyl group, a C1 to 4 haloalkyl group, a C3 to 6 cycloalkyl group, a di-(C1 to 4 alkyl)amino group, a 5 to 6-membered carbocyclic ring which may be substituted with 1 to 3 R²¹s, or a 5 to 6-membered heterocyclic ring which may be substituted with 1 to 3 R²¹s, R²¹ represents halogen, a C1 to 4 alkyl group, or a C1 to 4 alkoxy group, a plurality of R²¹s may be the same or different, R³ represents a 3 to 5-membered saturated carbocyclic ring which may be substituted with 1 to 5 R¹¹s or a 3 to 5-membered saturated heterocyclic ring which may be substituted with 1 to 5 R¹¹s, R¹¹ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, or an amino group, a plurality of R¹¹s may be the same or different, and two R¹¹s may be taken together to form C1 to 3 alkylene, and R^{4Y} and R^{5Y} each independently represent halogen, a C1 to 4 alkyl group, a C1 to 4 haloalkyl group, a C3 to 6 cycloalkyl group, a C2 to 4 alkenyl group, or a 5 to 6-membered carbocyclic ring which may be substituted with 1 to 3 R¹⁵s or a 5 to 6-membered heterocyclic ring which may be substituted with 1 to 3 R¹⁵s, R¹⁵ represents halogen, a C1 to 4 alkyl group, or a C1 to 4 alkoxy group, a plurality of R¹⁵s may be the same or different, and carbon atoms at position 4 and position 5 of the pyrrole ring, R^{4Y}, and R^{5Y} may be taken together to form a 5 to 6-membered carbocyclic ring which may be substituted with 1 to 3 R¹²s, or a 5 to 6-membered heterocyclic ring which may be substituted with 1 to 3 R¹²s, R¹² represents halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, a plurality of R¹²s may be the same or different, and R⁶ and R⁷ each independently represent a hydrogen atom, a C1 to 4 alkyl group, or a C1 to 4 haloalkyl group, and herein, each hydrogen atom may be a deuterium atom or a tritium atom; provided, however, 3-[(cyclopentylamino)methyl]-1 - [(3 -methylphenyl)methyl] -1H-indole-2-carboxylic acid, 3-[(cyclopentylamino)methyl]-1-[(2-fluorophenyl)methyl]-6-methyl-1H-indole-2-carboxylic acid, 3-[(cyclopentylamino)methyl]-6-methoxy-1-[(4-methylphenyl)methyl]-1H-indole-2-carboxylic acid, 3-[(cyclopentylamino)methyl]-1-ethyl-6-methoxy-1H-indole-2-carboxylic acid, 1 - [(2-chloro-4-fluorophenyl)methyl] -3 - [(cyclopentylamino)methyl] -6-methoxy-1H-indole-2-carboxylic acid, 1-[(2-chloro-4-fluorophenyl)methyl]-3-[(cyclopropylamino)methyl]-6-methoxy-1H-indole-2-carboxylic acid, 3-[(cyclopentylamino)methyl]-6-methoxy-1-[(4-methoxyphenyl)methyl]-1H-indole-2-carboxylic acid, 3-[(cyclopropylamino)methyl]-6-methoxy-1-[(4-methoxyphenyl)methyl]-1H-indole-2-carboxylic acid, 3-[(cyclopentylamino)methyl]-1-[(4-fluorophenyl)methyl]-6-methoxy-1H-indole-2-carboxylic acid, 3-[(cyclopentylamino)methyl]-6-methoxy-1-methyl-1H-indole-2-carboxylic acid, 3-[(cyclopentylamino)methyl]-1-[(4-methoxyphenyl)methyl]-1H-indole-2-carboxylic acid, 3-[(cyclopentylamino)methyl]-1-ethyl-6-methyl-1H-indole-2-carboxylic acid, 3-[(cyclopentylamino)methyl]-6-methoxy-1-[(2-methylphenyl)methyl]-1H-indole-2-carboxylic acid, 3-[(cyclopentylamino)methyl]-6-methyl-1-[(4-methylphenyl)methyl]-1H-indole-2-carboxylic acid, 3-[(cyclopropylamino)methyl]-6-methyl-1 - [(4-methylphenyl)methyl] -1 H-indole-2-carboxylic acid, 3 - [(cyclopentylamino)methyl] -1 - [(2-fluorophenyl)methyl]-6-methoxy-1H-indole-2-carboxylic acid, 3-[(cyclopentylamino)methyl]-1-[(4-fluorophenyl)methyl]-6-methyl-1H-indole-2-carboxylic acid, 3-[(cyclopropylamino)methyl]-1-[(4-fluorophenyl)methyl]-6-methyl-1H-indole-2-carboxylic acid, 3 - [(cyclopentylamino)methyl] -1 - [(3 -fluorophenyl)methyl]-6-methoxy-1H-indole-2-carboxylic acid, 3-[(cyclopentylamino)methyl]-6-methyl-1-[(2-methylphenyl)methyl]-1H-indole-2-carboxylic acid, 3-[(cyclopropylamino)methyl]-6-methyl-1-[(2-methylphenyl)methyl]-1H-indole-2-carboxylic acid, 3 - [(cyclopentylamino)methyl] -1-[(3-fluorophenyl)methyl]-6-methyl-1H-indole-2-carboxylic acid, 3-[(cyclopropylamino)methyl]-1-[(3-fluorophenyl)methyl]-6-methyl-1H-indole-2-carboxylic acid, 3 -[(cyclopentylamino)methyl] -1 - [(4-methoxyphenyl)methyl] -6-methyl-1H-indole-2-carboxylic acid, 3 - [(cyclopentylamino)methyl] -1 - [(4-ethenylphenyl)methyl] -6-methyl-1H-indole-2-carboxylic acid, 3 - [(cyclopentylamino)methyl] -1 -methyl-1H-indole-2-carboxylic acid, 3-[(cyclopentylamino)methyl]-1-(phenylmethyl)-1H-indole-2-carboxylic acid, 3-[(cyclopentylamino)methyl]-1 - [(2-methylphenyl)methyl] -1 H-indole-2-carboxylic acid, 3-[(cyclopentylamino)methyl]-1-[(4-methylphenyl)methyl]-1H-indole-2-carboxylic acid, 3-[(cyclopentylamino)methyl]-1 - [(4-fluorophenyl)methyl] -1H-indole-2-carboxylic acid, 3-[(cyclopentylamino)methyl]-1-[(2,5-dimethylphenyl)methyl]-1H-indole-2-carboxylic acid, 3-[(cyclopentylamino)methyl]-1-[(4-ethenylphenyl)methyl]-1H-indole-2-carboxylic acid, 3-[(cyclopropylamino)methyl]-1-(phenylmethyl)-1 H-indole-2-carboxylic acid, 3-[(cyclopropylamino)methyl]-1-[(2-methylphenyl)methyl]-1H-indole-2-carboxylic acid, 3-[(cyclopropylamino)methyl]-1-[(3-methylphenyl)methyl]-1H-indole-2-carboxylic acid, 3-[(cyclopropylamino)methyl] -1 - [(4-methylphenyl)methyl] -1H-indole-2-carboxylic acid, 3-[(cyclopropylamino)methyl]-1-[(4-ethenylphenyl)methyl]-1H-indole-2-carboxylic acid, 1-[(2-chlorophenyl)methyl]-3-[(cyclopentylamino)methyl]-1H-indole-2-carboxylic acid, 1-[(2-chlorophenyl)methyl]-3-[(cyclopropylamino)methyl]-1H-indole-2-carboxylic acid, 1-[(3-chlorophenyl)methyl]-3-[(cyclopentylamino)methyl]-1H-indole-2-carboxylic acid, 1-[(4-chlorophenyl)methyl]-3-[(cyclopentylamino)methyl]-1H-indole-2-carboxylic acid, 1-[(2-chloro-4-fluorophenyl)methyl] -3 - [(cyclopentylamino)methyl] -1H-indole-2-carboxylic acid, 1 - [(2-chloro-4-fluorophenyl)methyl] -3 - [(cyclopropylamino)methyl] -1 H-indole-2 carboxylic acid, and 3-[(cyclopentylamino)methyl]-1,6-dimethyl-1H-indole-2-carboxylic acid are excluded, or a salt thereof;
[2] The compound according to [1], represented by general formula (I): 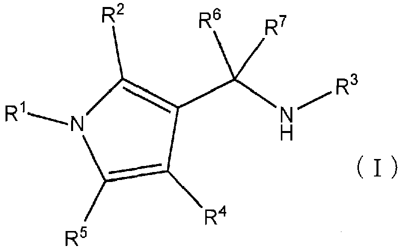 wherein R¹ represents a C1 to 4 alkyl group or -CR⁸R⁹-ring 1, R⁸ and R⁹ each independently represent a hydrogen atom or a C1 to 4 alkyl group, ring 1 represents a 3 to 15-membered carbocyclic ring which may be substituted with 1 to 5 R¹³s or a 3 to 15-membered heterocyclic ring which may be substituted with 1 to 5 R¹³s, R¹³ represents halogen, a C1 to 4 alkyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or - O-(CH₂)r-COOH group, a plurality of R¹³s may be the same or different, r represents an integer of 2 to 5, R² represents a carboxyl group, a C1 to 4 alkoxycarbonyl group, or - CONHSO₂R¹⁰, R¹⁰ represents a C1 to 4 alkyl group, a C3 to 6 cycloalkyl group, a 5 to 6-membered carbocyclic ring or 5 to 6-membered heterocyclic ring which may be substituted with 1 to 3 R¹⁶s, R¹⁶ represents halogen, a C1 to 4 alkyl group or a C1 to 4 alkoxy group, a plurality of R¹⁶s may be the same or different, R³ represents a 3 to 5-membered saturated carbocyclic ring which may be substituted with 1 to 5 R¹¹s, or a 3 to 5-membered saturated heterocyclic ring which may be substituted with 1 to 5 R¹¹s, R¹¹ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, or an amino group, a plurality of R¹¹s may be the same or different, two R¹¹s may be taken together to form C1 to 3 alkylene, R⁴ and R⁵ each independently represent halogen, a C1 to 4 alkyl group, a C2 to 4 alkenyl group, or a 5 to 6-membered carbocyclic ring which may be substituted with 1 to 3 R¹⁵s or a 5 to 6-membered heterocyclic ring which may be substituted with 1 to 3 R¹⁵s, R¹⁵ represents halogen, a C1 to 4 alkyl group, or a C1 to 4 alkoxy group, a plurality of R¹⁵s may be the same or different, and carbon atoms at position 4 and position 5 of the pyrrole ring, R⁴, and R⁵ may be taken together to form a 5 to 6-membered carbocyclic ring which may be substituted with 1 to 3 R¹²s, or a 5 to 6-membered heterocyclic ring which may be substituted with 1 to 3 R¹²s, R¹² represents halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, a plurality of R¹²s may be the same or different, and R⁶ and R⁷ each independently represent a hydrogen atom, a C1 to 4 alkyl group, or a C1 to 4 haloalkyl group, and herein, each hydrogen atom may be a deuterium atom or a tritium atom; provided, however, 3-[(cyclopentylamino)methyl]-1-[(3-methylphenyl)methyl]-1H-indole-2-carboxylic acid, 3-[(cyclopentylamino)methyl]-1-[(2-fluorophenyl)methyl]-6-methyl-1H-indole-2-carboxylic acid, 3-[(cyclopentylamino)methyl]-6-methoxy-1-[(4-methylphenyl)methyl]-1H-indole-2-carboxylic acid, 3-[(cyclopentylamino)methyl]-1-ethyl-6-methoxy-1H-indole-2-carboxylic acid, 1-[(2-chloro-4-fluorophenyl)methyl]-3-[(cyclopentylamino)methyl]-6-methoxy-1H-indole-2-carboxylic acid, 1-[(2-chloro-4-fluorophenyl)methyl]-3-[(cyclopropylamino)methyl]-6-methoxy-1H-indole-2-carboxylic acid, 3 - [(cyclopentylamino)methyl] -6-methoxy-1 - [(4-methoxyphenyl)methyl] -1H-indole-2-carboxylic acid, 3 - [(cyclopropylamino)methyl] -6-methoxy-1 - [(4-methoxyphenyl)methyl]-1H-indole-2-carboxylic acid, 3 - [(cyclopentylamino)methyl] -1 - [(4-fluorophenyl)methyl]-6-methoxy-1H-indole-2-carboxylic acid, 3-[(cyclopentylamino)methyl]-6-methoxy-1-methyl-1H-indole-2-carboxylic acid, 3-[(cyclopentylamino)methyl]-1-[(4-methoxyphenyl)methyl]-1H-indole-2-carboxylic acid, 3-[(cyclopentylamino)methyl]-1-ethyl-6-methyl-1H-indole-2-carboxylic acid, 3-[(cyclopentylamino)methyl]-6-methoxy-1-[(2-methylphenyl)methyl]-1H-indole-2-carboxylic acid, 3-[(cyclopentylamino)methyl]-6-methyl-1-[(4-methylphenyl)methyl]-1H-indole-2-carboxylic acid, 3 - [(cyclopropylamino)methyl] -6-methyl-1 - [(4-methylphenyl)methyl] -1H-indole-2-carboxylic acid, 3-[(cyclopentylamino)methyl]-1-[(2-fluorophenyl)methyl]-6-methoxy-1H-indole-2-carboxylic acid, 3 - [(cyclopentylamino)methyl] -1 - [(4-fluorophenyl)methyl]-6-methyl-1H-indole-2-carboxylic acid, 3-[(cyclopropylamino)methyl]-1-[(4-fluorophenyl)methyl]-6-methyl-1H-indole-2-carboxylic acid, 3-[(cyclopentylamino)methyl]-1-[(3 -fluorophenyl)methyl] -6-methoxy-1H-indole-2-carboxylic acid, 3-[(cyclopentylamino)methyl]-6-methyl-1-[(2-methylphenyl)methyl]-1H-indole-2-carboxylic acid, 3 - [(cyclopropylamino)methyl] -6-methyl-1 - [(2-methylphenyl)methyl] -1H-indole-2-carboxylic acid, 3 -[(cyclopentylamino)methyl]-1-[(3-fluorophenyl)methyl]-6-methyl-1H-indole-2-carboxylic acid, 3 - [(cyclopropylamino)methyl] -1 - [(3 - fluorophenyl)methyl] -6-methyl-1 H-indole-2-carboxylic acid, 3 - [(cyclopentylamino)methyl] - 1-[(4-methoxyphenyl)methyl]-6-methyl-1H-indole-2-carboxylic acid, 3-[(cyclopentylamino)methyl]-1-[(4-ethenylphenyl)methyl]-6-methyl-1H-indole-2-carboxylic acid, 3-[(cyclopentylamino)methyl]-1-methyl-1H-indole-2-carboxylic acid, 3-[(cyclopentylamino)methyl]-1-(phenylmethyl)-1H-indole-2-carboxylic acid, 3-[(cyclopentylamino)methyl]-1-[(2-methylphenyl)methyl]-1H-indole-2-carboxylic acid, 3-[(cyclopentylamino)methyl]-1-[(4-methylphenyl)methyl]-1H-indole-2-carboxylic acid, 3-[(cyclopentylamino)methyl]-1-[(4-fluorophenyl)methyl]-1H-indole-2-carboxylic acid, 3-[(cyclopentylamino)methyl]-1-[(2,5-dimethylphenyl)methyl]-1H-indole-2-carboxylic acid, 3-[(cyclopentylamino)methyl]-1 - [(4-ethenylphenyl)methyl] -1H-indole-2-carboxylic acid, 3-[(cyclopropylamino)methyl]-1-(phenylmethyl)-1H-indole-2-carboxylic acid, 3-[(cyclopropylamino)methyl]-1-[(2-methylphenyl)methyl]-1H-indole-2-carboxylic acid, 3-[(cyclopropylamino)methyl] -1 - [(3 -methylphenyl)methyl] -1H-indole-2-carboxylic acid, 3-[(cyclopropylamino)methyl]-1-[(4-methylphenyl)methyl]-1H-indole-2-carboxylic acid, 3-[(cyclopropylamino)methyl]-1-[(4-ethenylphenyl)methyl]-1H-indole-2-carboxylic acid, 1-[(2-chlorophenyl)methyl]-3-[(cyclopentylamino)methyl]-1H-indole-2-carboxylic acid, 1-[(2-chlorophenyl)methyl]-3-[(cyclopropylamino)methyl]-1H-indole-2-carboxylic acid, 1-[(3-chlorophenyl)methyl]-3-[(cyclopentylamino)methyl]-1H-indole-2-carboxylic acid, 1-[(4-chlorophenyl)methyl]-3-[(cyclopentylamino)methyl]-1H-indole-2-carboxylic acid, 1-[(2-chloro-4-fluorophenyl)methyl]-3-[(cyclopentylamino)methyl]-1H-indole-2-carboxylic acid, 1-[(2-chloro-4-fluorophenyl)methyl]-3-[(cyclopropylamino)methyl]-1H-indole-2 carboxylic acid, and 3-[(cyclopentylamino)methyl]-1,6-dimethyl-1H-indole-2-carboxylic acid are excluded, or a salt thereof;
[3] The compound according to [1] or [2], or a salt thereof, in which R³ is a cyclobutyl ring which may be substituted with 1 to 5 R¹¹s;
[4] The compound according to any one of [1] to [3], represented by general formula (I-1): 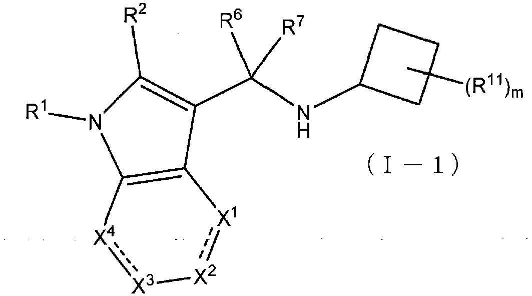
   wherein X¹ represents CR¹⁴⁻¹, NR¹⁴⁻¹, or a nitrogen atom, X² represents CR¹⁴⁻², NR¹⁴⁻², or a nitrogen atom, X³ represents CR¹⁴⁻³, NR¹⁴⁻³, or a nitrogen atom, X⁴ represents CR¹⁴⁻⁴, NR¹⁴⁻⁴, or a nitrogen atom, R¹⁴⁻¹, R¹⁴⁻², R¹⁴⁻³, and R¹⁴⁻⁴ each independently represent a hydrogen atom, halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, and m represents an integer of 0 to 5, 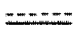
   represents a single bond or a double bond, and other symbols represent the same meaning as in [1] or [2], or a salt thereof;
[5] The compound according to any one of [1] to [4], represented by general formula (I-1-3): 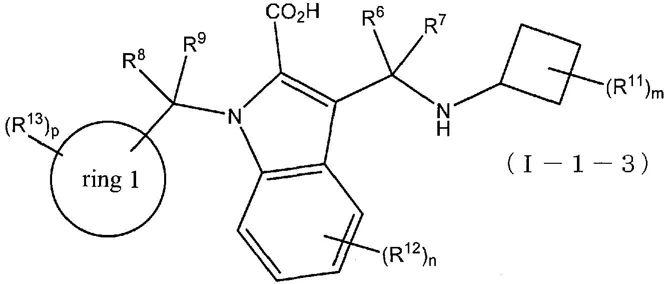 wherein p represents an integer of 0 to 5, n represents an integer of 0 to 3, and other symbols represent the same meaning as in [1], [2] or [4], or a salt thereof;
[5-1] The compound according to [5], in which ring 1 is a saturated 3 to 15-membered carbocyclic ring which may be substituted with 1 to 3 R¹³s, or a salt thereof;
[6] The compound according to any one of [1] to [5], represented by general formula (I-1-1): 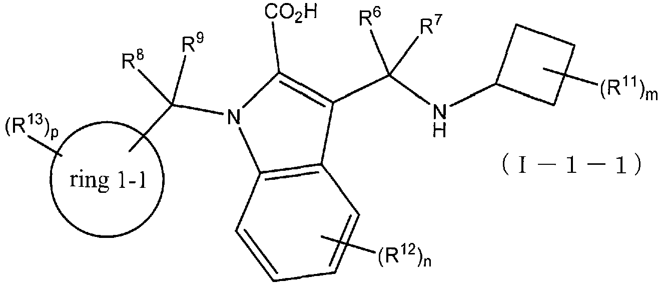 wherein ring 1-1 represents a 5 to 6-membered carbocyclic ring or a 5 to 6-membered heterocyclic ring, and other symbols represent the same meaning as in [1], [2], [4] or [5], or a salt thereof;
[7] The compound according to [6], in which ring 1-1 is a 5 to 6-membered carbocyclic ring, or a salt thereof;
[8] The compound according to any one of [1] to [3], represented by general formula (1-2): 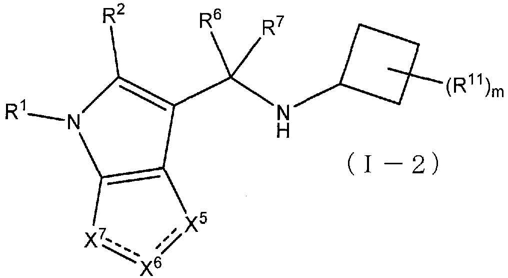 wherein X⁵ represents CR¹⁴⁻⁵, NR¹⁴⁻⁵, a nitrogen atom, or a sulfur atom which may be oxidized, X⁶ represents CR¹⁴⁻⁶, NR¹⁴⁻⁶, or a nitrogen atom, X⁷ represents CR¹⁴⁻⁷, NR¹⁴⁻⁷, a nitrogen atom, or a sulfur atom which may be oxidized, R¹⁴⁻⁵, R¹⁴⁻⁶, and R¹⁴⁻⁷ each independently represent a hydrogen atom, halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, m represents an integer of 0 to 5, and other symbols represent the same meaning as in [1] or [2]; provided that, 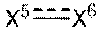 and 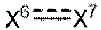 do not simultaneously represent a double bond, or a salt thereof;
[9] The compound according to any one of [1] to [3], represented by general formula (1-3): 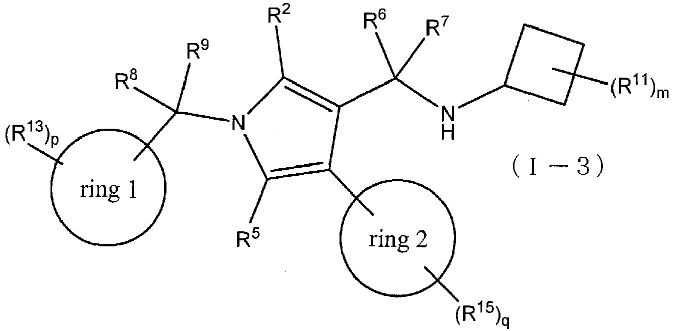 wherein ring 2 represents a 5 to 6-membered carbocyclic ring or a 5 to 6-membered heterocyclic ring, p represents an integer of 0 to 5, q represents an integer of 0 to 3, m represents an integer of 0 to 5, and other symbols represent the same meaning as in [1] or [2], or a salt thereof;
[10] The compound according to any one of [1] or [3], represented by general formula (1-4): 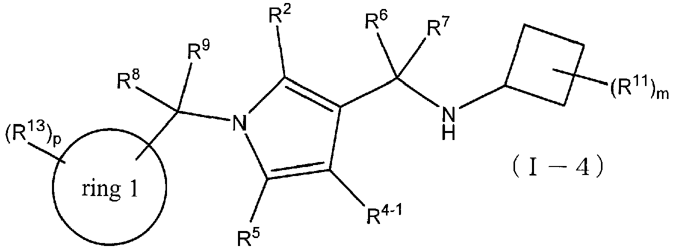 wherein R⁴⁻¹ represents halogen, a C1 to 4 alkyl group, or a C2 to 4 alkenyl group, p represents an integer of 0 to 5, m represents an integer of 0 to 5, and other symbols represent the same meaning as in [1] or [2], or a salt thereof;
[11] The compound according to [1] or [2], wherein the compound is
   (1) 3-[(cyclobutylamino)methyl]-1-(cyclopropylmethyl)-lH-indole-2-carboxylic acid,
   (2) 3-[(cyclobutylamino)methyl]-1-[(2,2-difluorocyclopropyl)methyl]-1H-indole-2-carboxylic acid,
   (3) 3-[(cyclobutylamino)methyl]-1-ethyl-1 H-indole-2-carboxylic acid,
   (4) 3 - [(cyclobutylamino)methyl] -1 -(4-methylbenzyl)-1H-indole-2-carboxylic acid,
   (5) 1-(2-chlorobenzyl)-3-[(cyclobutylamino)methyl]-1H-indole-2-carboxylic acid,
   (6) 5-bromo-3-[(cyclobutylamino)methyl]-4-methyl-1-(4-methylbenzyl)-1H-pyrrole-2-carboxylic acid,
   (7) 3-[(cyclobutylamino)methyl]-1-(3-cyclopropylbenzyl)-1H-indole-2-carboxylic acid,
   (8) 1-(4-methylbenzyl)-3-{[(2-methylcyclobutyl)amino]methyl}-1H-indole-2-carboxylic acid,
   (9) 3-[(cyclobutylamino)methyl]-1-(4-methylbenzyl)-1H-pyrrolo[2,3-b]pyridine-2-carboxylic acid,
   (10) 3 - [(cyclobutylamino)methyl] -1 - [2-(difluoromethoxy)benzyl] -1H-indole-2-carboxylic acid,
   (11) 6- [(cyclobutylamino)methyl] -4-(4-methylbenzyl)-4H-thieno[3,2-b]pyrrole-5-carboxylic acid,
   (12) 1 -(4-methylbenzyl)-3 - [(3 -thiethanylamino)methyl] -1H-indole-2-carboxylic acid,
   (13) 3-[1 -(cyclobutylamino)ethyl]-1 -(4-methylbenzyl)-1H-indole-2-carboxylic acid,
   (14) 3 - [(cyclobutylamino)methyl] -4-(3 -fluorophenyl)-5 -methyl-1 -(4-methylbenzyl)-1H-pyrrole-2-carboxylic acid,
   (15) 3-[(cyclobutylamino)methyl] -5 -methyl-1-(4-methylbenzyl)-4-vinyl-1H-pyrrole-2-carboxylic acid,
   (16) 3-[(cyclobutylamino)methyl]-1-(4-methylbenzyl)-4,5,6,7-tetrahydro-1H-indole-2-carboxylic acid, or
   (17) 7-cyano-3-[(cyclobutylamino)methyl]-1-(4-methylbenzyl)-1H-indole-2-carboxylic acid, or a salt thereof;
[12] A pharmaceutical composition containing the compound represented by general formula (IY) or the salt thereof according to [1] and a pharmaceutically acceptable carrier;
[12-1] The pharmaceutical composition according to [12], in which the compound represented by general formula (IY) or the salt thereof is a compound represented by general formula (I-1-2): 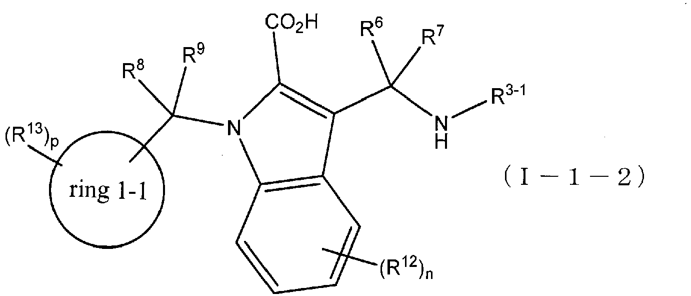 wherein R³⁻¹ represents a 3 to 5-membered saturated carbocyclic ring which may be substituted with 1 to 5 R¹¹s, and other symbols represent the same meaning as in [1], [2], [5] or [6], or a salt thereof;
[13] The pharmaceutical composition according to [12] or [12-1], which is an LPA3 agonist;
[14] The pharmaceutical composition according to [12], [12-1] or [13], which is a preventive and/or therapeutic agent for an LPA3-related disease;
[15] The pharmaceutical composition according to [14], wherein the LPA3-related disease is essential thrombocythemia, reactive thrombocytosis, aplastic anemia, myelodysplastic syndrome, or sepsis;
[16] A method for preventing and/or treating an LPA3-related disease, including administering an effective amount of the compound represented by general formula (IY) or the salt thereof according to the above [1] to a mammal;
[17] The compound represented by general formula (IY) or the salt thereof according the above [1], which is used for prevention and/or treatment of an LPA3-related disease; and
[18] A use of the compound represented by general formula (IY) or the salt thereof according to the above [1], for producing a preventive and/or therapeutic agent for an LPA3-related disease;
   and the like.

### ADVANTAGEOUS EFFECTS OF INVENTION

Since the compound of the present invention has an agonist activity for LPA3, it can be used as an active ingredient of a preventive and/or therapeutic agent for essential thrombocythemia, reactive thrombocytosis, and the like.

### DESCRIPTION OF EMBODIMENTS

In the present invention, the "halogen" includes, for example, fluorine, chlorine, bromine, and iodine atoms.

In the present invention, the "C1 to 4 alkyl group" includes methyl, ethyl, propyl, 1-methylethyl, butyl, 1-methylpropyl, 2-methylpropyl, and 2,2-dimethylethyl groups.

In the present invention, the "C2 to 4 alkenyl group" includes ethenyl, 1-propenyl, 2-propenyl, 1-methylethenyl, 1-butenyl, 2-butenyl, 3-butenyl, 1-ethyl-1-ethenyl, 1-methyl-1-propenyl, 2-methyl-1-propenyl, 1-methyl-2-propenyl, and 2-methyl-2-propenyl groups.

In the present invention, the "C2 to 4 alkynyl group" includes ethynyl, 1-propynyl, 2-propynyl, 1-butynyl, 2-butynyl, and 3-butynyl groups.

In the present invention, the "C1 to 4 haloalkyl group" includes fluoromethyl, fluoroethyl, fluoropropyl, fluoro-1-methylethyl, fluorobutyl, fluoro-1-methylpropyl, fluoro-2-methylpropyl, fluoro-2,2-dimethylethyl, difluoromethyl, difluoroethyl, difluoropropyl, difluoro-1-methylethyl, difluorobutyl, difluoro-1-methylpropyl, difluoro-2-methylpropyl, difluoro-2,2-dimethylethyl, trifluoromethyl, trifluoroethyl, trifluoropropyl, trifluoro-1-methylethyl, trifluorobutyl, trifluoro-1-methylpropyl, trifluoro-2-methylpropyl, trifluoro-2,2-dimethylethyl, chloromethyl, chloroethyl, chloropropyl, chloro-1-methylethyl, chlorobutyl, chloro-1-methylpropyl, chloro-2-methylpropyl, chloro-2,2-dimethylethyl, dichloromethyl, dichloroethyl, dichloropropyl, dichloro-1-methylethyl, dichlorobutyl, dichloro-1-methylpropyl, dichloro-2-methylpropyl, dichloro-2,2-dimethylethyl, trichloromethyl, trichloroethyl, trichloropropyl, trichloro-1-methylethyl, trichlorobutyl, trichloro-1-methylpropyl, trichloro-2-methylpropyl, and trichloro-2,2-dimethylethyl groups.

In the present invention, the "(C1 to 4 alkyl)carbonylamino group" includes methylcarbonylamino, ethylcarbonylamino, propylcarbonylamino, 1-methylethylcarbonylamino, butylcarbonylamino, 1-methylpropylcarbonylamino, 2-methylpropylcarbonylamino, and 2,2-dimethylethylcarbonylamino groups.

In the present invention, the "(C1 to 4 alkyl)carbonyl group" includes methylcarbonyl, ethylcarbonyl, propylcarbonyl, 1-methylethylcarbonyl, butylcarbonyl, 1-methylpropylcarbonyl, 2-methylpropylcarbonyl, and 2,2-dimethylethylcarbonyl groups.

In the present invention, the "(C1 to 4 alkyl)aminocarbonyl group" includes methylaminocarbonyl, ethylaminocarbonyl, propylaminocarbonyl, 1-methylethylaminocarbonyl, butylaminocarbonyl, 1-methylpropylaminocarbonyl, 2-methylpropylaminocarbonyl, and 2,2-dimethylethylaminocarbonyl groups.

In the present invention, the "di-(C1 to 4 alkyl)amino group" includes dimethylamino, diethylamino, dipropylamino, di(1-methylethyl)amino, dibutylamino, di(1-methylpropyl)amino, di(2-methylpropyl)amino, and di(2,2-dimethylethyl)amino groups.

In the present invention, the "di(C1 to 4 alkyl)aminocarbonyl group" includes dimethylaminocarbonyl, diethylaminocarbonyl, dipropylaminocarbonyl, di(1-methylethyl)aminocarbonyl, dibutylaminocarbonyl, di(1-methylpropyl)aminocarbonyl, di(2-methylpropyl)aminocarbonyl, and di(2,2-dimethylethyl)aminocarbonyl groups.

In the present invention, the "di-(C1 to 4 alkyl)-hydroxymethyl group" includes dimethylhydroxymethyl, diethylhydroxymethyl, dipropylhydroxymethyl, di(1-methylethyl)hydroxymethyl, dibutylhydroxymethyl, di(1-methylpropyl)hydroxymethyl, di(2-methylpropyl)hydroxymethyl, and di(2,2-dimethylethyl)hydroxymethyl groups.

In the present invention, the "C1 to 3 alkylene group" includes a methylene, ethylene, propylene, or methylethylene group.

In the present invention, the "C1 to 4 alkoxy group" includes methoxy, ethoxy, propoxy, 1-methylethoxy, butoxy, 1-methylpropoxy, 2-methylpropoxy, and 2,2-dimethylethoxy groups.

In the present invention, the "C1 to 4 alkoxycarbonyl group" includes methoxycarbonyl, ethoxycarbonyl, propoxycarbonyl, 1-methylethoxycarbonyl, butoxycarbonyl, 1-methylpropoxycarbonyl, 2-methylpropoxycarbonyl, and 2,2-dimethylethoxycarbonyl groups.

In the present invention, the "C1 to 4 haloalkoxy group" includes fluoromethoxy, fluoroethoxy, fluoropropoxy, fluoro-1-methylethoxy, fluorobutoxy, fluoro-1-methylpropoxy, fluoro-2-methylpropoxy, fluoro-2,2-dimethylethoxy, difluoromethoxy, difluoroethoxy, difluoropropoxy, difluoro-1-methylethoxy, difluorobutoxy, difluoro-1-methylpropoxy, difluoro-2-methylpropoxy, difluoro-2,2-dimethylethoxy, trifluoromethoxy, trifluoroethoxy, trifluoropropoxy, trifluoro-1-methylethoxy, trifluorobutoxy, trifluoro-1-methylpropoxy, trifluoro-2-methylpropoxy, trifluoro-2,2-dimethylethoxy, chloromethoxy, chloroethoxy, chloropropoxy, chloro-1-methylethoxy, chlorobutoxy, chloro-1-methylpropoxy, chloro-2-methylpropoxy, chloro-2,2-dimethylethoxy, dichloromethoxy, dichloroethoxy, dichloropropoxy, dichloro-1-methylethoxy, dichlorobutoxy, dichloro-1-methylpropoxy, dichloro-2-methylpropoxy, dichloro-2,2-dimethylethoxy, trichloromethoxy, trichloroethoxy, trichloropropoxy, trichloro-1-methylethoxy, trichlorobutoxy, trichloro-1-methylpropoxy, trichloro-2-methylpropoxy, and trichloro-2,2-dimethylethoxy groups.

In the present invention, the "C1 to 4 alkylthio group" includes methylthio, ethylthio, propylthio, 1-methylethylthio, butylthio, 1-methylpropylthio, 2-methylpropylthio, and 2,2-dimethylethylthio groups.

In the present invention, the "C1 to 4 haloalkylthio group" includes monofluoromethylthio, monofluoroethylthio, monofluoropropylthio, monofluoro-1-methylethylthio, monofluorobutylthio, monofluoro-1-methylpropylthio, monofluoro-2-methylpropylthio, monofluoro-2,2-dimethylethylthio, difluoromethylthio, difluoroethylthio, difluoropropylthio, difluoro-1-methylethylthio, difluorobutylthio, difluoro-1-methylpropylthio, difluoro-2-methylpropylthio, difluoro-2,2-dimethylethylthio, trifluoromethylthio, trifluoroethylthio, trifluoropropylthio, trifluoro-1-methylethylthio, trifluorobutylthio, trifluoro-1-methylpropylthio, trifluoro-2-methylpropylthio, trifluoro-2,2-dimethylethylthio, perfluoroethylthio, perfluoropropylthio, perfluoro-1-methylethylthio, perfluorobutylthio, perfluoro-1-methylpropylthio, perfluoro-2-methylpropylthio, perfluoro-2,2-dimethylethylthio, monochloromethylthio, monochloroethylthio, monochloropropylthio, monochloro-1-methylethylthio, monochlorobutylthio, monochloro-1-methylpropylthio, monochloro-2-methylpropylthio, monochloro-2,2-dimethylethylthio, dichloromethylthio, dichloroethylthio, dichloropropylthio, dichloro-1-methylethylthio, dichlorobutylthio, dichloro-1-methylpropylthio, dichloro-2-methylpropylthio, dichloro-2,2-dimethylethylthio, trichloromethylthio, trichloroethylthio, trichloropropylthio, trichloro-1-methylethylthio, trichlorobutylthio, trichloro-1-methylpropylthio, trichloro-2-methylpropylthio, trichloro-2,2-dimethylethylthio, perchloroethylthio, perchloropropylthio, perchloro-1-methylethylthio, perchlorobutylthio, perchloro-1-methylpropylthio, perchloro-2-methylpropylthio, and perchloro-2,2-dimethylethylthio groups.

In the present invention, the "3 to 15-membered carbocyclic ring" includes monocyclic, bicyclic or tricyclic unsaturated, partially saturated or saturated 3 to 15-membered carbocyclic rings. Examples of the monocyclic, bicyclic or tricyclic unsaturated 3 to 15-membered carbocyclic ring include benzene, pentalene, naphthalene, azulene, phenanthrene, anthracene, acenaphthylene, and biphenylene rings, and the like. Examples of the monocyclic, bicyclic or tricyclic, partially saturated or saturated 3 to 15-membered carbocyclic ring include cyclopropane, cyclobutane, cyclopentane, cyclohexane, cycloheptane, cyclopentene, cyclohexene, cyclopentadiene, cyclohexadiene, indene, fluorene, perhydropentalene, perhydroindene, perhydronaphthalene, perhydroazulene, perhydrofluorene, perhydrophenanthrene, perhydroanthracene, perhydroacenaphthylene, perhydrobiphenylene, and adamantane rings, and the like.

In the present invention, the "3 to 15-membered heterocyclic ring" includes monocyclic, bicyclic or tricyclic unsaturated, partially saturated or saturated 3 to 15-membered heterocyclic rings containing 1 to 4 nitrogen atoms, 1 to 2 oxygen atoms and/or 1 sulfur atom. Examples of the monocyclic, bicyclic or tricyclic unsaturated 3 to 15-membered heterocyclic ring containing 1 to 4 nitrogen atoms, 1 to 2 oxygen atoms and/or 1 sulfur atom include pyrrole, imidazole, triazole, tetrazole, pyrazole, pyridine, pyrazine, pyrimidine, pyridazine, azepine, diazepine, furan, pyran, oxepine, oxazepine, thiophene, thiain (thiopyran), thiepine, oxazole, isoxazole, thiazole, isothiazole, oxadiazole, oxazine, oxadiazine, oxazepine, oxadiazepine, thiadiazole, thiazine, thiadiazine, thiazepine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzoxadiazole, benzothiazole, benzimidazole, carbazole, and acridine rings, and the like. Examples of the monocyclic, bicyclic or tricyclic partially saturated or saturated 3 to 15-membered heterocyclic ring containing 1 to 4 nitrogen atoms, 1 to 2 oxygen atoms and/or 1 sulfur atom include aziridine, oxirane, azetidine, oxetane, thiirane, thietane, pyrroline, pyrrolidine, imidazoline, imidazolidine, triazoline, triazolidine, tetrazoline, tetrazolidine, pyrazoline, pyrazolidine, piperidine, piperazine, tetrahydropyridine, tetrahydropyrimidine, tetrahydropyridazine, dihydrofuran, tetrahydrofuran, dihydropyran, tetrahydropyran, dihydrothiophene, tetrahydrothiophene, dihydrothiaine (dihydrothiopyran), tetrahydrothiaine (tetrahydrothiopyran), oxazoline (dihydroxazole), oxazolidine (tetrahydroxazole), dihydroisoxazole, tetrahydroisoxazole, oxadiazoline (dihydroxadiazole), oxadiazolidine (tetrahydroxadiazole), thiazoline (dihydrothiazole), thiazolidine (tetrahydrothiazole), dihydroisothiazole, tetrahydroisothiazole, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole, perhydrobenzimidazole, benzoxazepine, benzoxadiazepine, benzothiazepine, benzothiadiazepine, benzoazepine, benzodiazepine, indolooxoazepine, indolotetrahydroxazepine, indolooxadiazepine, indolotetrahydroxadiazepine, indolothiazepine, indolotetrahydrothiazepine, indolothiadiazepine, indolotetrahydrothiadiazepine, indoloazepine, indolotetrahydroazepine, indolodiazepine, indolotetrahydrodiazepine, benzofurazan, benzothiadiazole, benzotriazole, camphor, imidazothiazole, dihydrocarbazole, tetrahydrocarbazole, perhydrocarbazole, dihydroacridine, tetrahydroacridine, perhydroacridine, dioxolane, and dioxazine rings, and the like.

In the present invention, the "5 to 6-membered carbocyclic ring" includes monocyclic unsaturated, partially saturated, or saturated 5 to 6-membered carbocyclic rings. Examples of the monocyclic unsaturated 5 to 6-membered carbocyclic ring include a benzene ring. Examples of the monocyclic partially saturated or saturated 5 to 6-membered carbocyclic ring include cyclopentane, cyclohexane, cyclopentene, cyclohexene, cyclopentadiene, and cyclohexadiene rings, and the like.

In the present invention, the "5 to 6-membered heterocyclic ring" includes monocyclic, unsaturated, partially saturated, or saturated 5 to 6-membered heterocyclic rings containing 1 to 4 nitrogen atoms, 1 to 2 oxygen atoms and/or 1 sulfur atom. Examples of the monocyclic unsaturated 5 to 6-membered heterocyclic ring containing 1 to 4 nitrogen atoms, 1 to 2 oxygen atoms and/or 1 sulfur atom include pyrrole, imidazole, triazole, tetrazole, pyrazole, pyridine, pyrazine, pyrimidine, pyridazine, furan, pyran, thiophene, thiain (thiopyran), oxazole, isoxazole, thiazole, isothiazole, oxadiazole, oxazine, oxadiazine, thiadiazole, thiazine, and thiadiazine rings, and the like. Examples of the monocyclic partially saturated or saturated 5 to 6-membered heterocyclic ring containing 1 to 4 nitrogen atoms, 1 to 2 oxygen atoms and/or 1 sulfur atom include pyrroline, pyrrolidine, imidazoline, imidazolidine, triazoline, triazolidine, tetrazoline, tetrazolidine, pyrazoline, pyrazolidine, piperazine, tetrahydropyridine, tetrahydropyrimidine, tetrahydropyridazine, dihydrofuran, tetrahydrofuran, dihydropyran, tetrahydropyran, dihydrothiophene, tetrahydrothiophene, dihydrothiane (dihydrothiopyran), tetrahydrothiaine (tetrahydrothiopyran), oxazoline (dihydroxazole), oxazolidine (tetrahydroxazole), dihydroisoxazole, tetrahydroisoxazole, oxadiazoline (dihydroxadiazole), oxadiazolidine (tetrahydroxadiazole), thiazoline (dihydrothiazole), thiazolidine (tetrahydrothiazole), dihydroisothiazole, tetrahydroisothiazole, morpholine, thiomorpholine, dioxolane, dioxane, and dioxazine rings, and the like.

In the present invention, the "3 to 5-membered saturated carbocyclic ring" includes cyclopropane, cyclobutane, and cyclopentane rings.

In the present invention, the "3 to 5-membered saturated carbocyclic ring" includes aziridine, oxirane, azetidine, oxetane, thiirane, thietane, pyrrolidine, imidazolidine, triazolidine, tetrazolidine, pyrazolidine, tetrahydrofuran, tetrahydrothiophene, oxazolidine (tetrahydroxazole), tetrahydroisoxazole, oxadiazolidine (tetrahydroxadiazole), thiazolidine (tetrahydrothiazole), tetrahydroisothiazole, and dioxolane rings.

In the present invention, the "C3 to 6 cycloalkyl group" includes cyclopropane, cyclobutane, cyclopentane, and cyclohexane rings.

In the present invention, the "CL to 4 alkylsulfonyl group" includes methylsulfonyl, ethylsulfonyl, propylsulfonyl, 1-methylethylsulfonyl, butylsulfonyl, 1-methylpropylsulfonyl, 2-methylpropylsulfonyl, and 2,2-dimethylethylsulfonyl groups.

In the present invention, the "C1 to 4 dialkylphosphoryl group" includes methylphosphoryl, ethylphosphoryl, propylphosphoryl, 1-methylethylphosphoryl, butylphosphoryl, 1-methylpropylphosphoryl, 2-methylpropylphosphoryl, and 2,2-dimethylethylphosphoryl groups.

In the present invention, the "sulfur atom which may be oxidized" includes -S-, - S(O)-, and -SO₂-.

In the present invention, examples of a 5 to 6-membered carbocyclic ring formed by carbon atoms at position 4 and position 5 of the pyrrole ring, R⁴ or R^{4Y}, and R⁵ or R^{5Y} taken together, described in general formula (I) or general formula (IY), include cyclopentene, cyclopentadiene, cyclohexene, cyclohexadiene, and benzene rings, and the like.

In the present invention, examples of a 5 to 6-membered heterocyclic ring formed by carbon atoms at position 4 and position 5 of the pyrrole ring, R⁴ or R^{4Y}, and R⁵ or R^{5Y} taken together, described in general formula (I) or general formula (IY), include pyridine, pyrimidine, thiophene, and thiazole rings, and the like.

In the present invention, examples of a bicyclic heterocyclic ring formed by the pyrrole ring described in general formula (I) or general formula (IY) and R⁴ or R^{4Y}, and R⁵ or R^{5Y} as substituents thereof taken together include indole, tetrahydrocyclopentapyrrole, tetrahydroindole, pyrrolopyridine, pyrrolopyrimidine, thienopyrrole, and pyrrolothiazole rings, and the like.

In the present invention, when two R¹¹s are taken together to form C1 to 3 alkylene, the two R¹¹s may be R¹¹s substituted with the same element or R¹¹s substituted with different elements. Examples of a cyclobutane ring in which two R¹¹s are taken together to form C1 to 3 alkylene include the following structures.

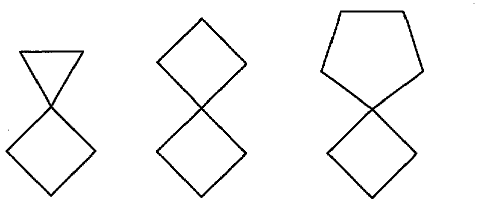

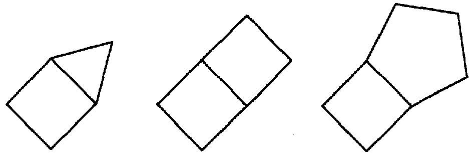

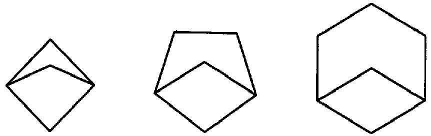

In the present invention, the 5-membered ring having highly acidic hydrogen includes a 5-membered ring having hydrogen with a pKa of 10 or less. Examples of the 5-membered ring having highly acidic hydrogen include a 4-hydroxy-1,2,5-oxadiazol-3-yl group, a 4-hydroxy-1,2,5-thiadiazol-3-yl group, a 5-oxo-4,5-dihydro-1H-tetrazol-1-yl group, a 3,5-dioxo-1,2,4-oxadiazolidin-2-yl group, a 5-oxo-4,5-dihydro-1,2,4-thiadiazol-3-yl group, a 5-thioxo-4,5-dihydro-1,3,4-oxadiazol-2-yl group, a 5-thioxo-4,5-dihydro-1H-1,2,4-triazol-3-yl group, a 5-thioxo-4,5-dihydro-1,2,4-oxadiazol-3-yl group, a 1H-1,2,3-triazol-5-yl group, a 2,4-dioxo-1,3-oxazolidin-5-yl group, a 2,4-dioxo-1,3-thiazolidin-5-yl group, a 3-hydroxy-1,2-oxazol-5-yl group, a 3-hydroxy-1,2-thiazol-5-yl group, a 3-hydroxy-1,2-oxazol-4-yl group, a 3-hydroxy-1,2-thiazol-4-yl group, a 4H-1,2,4-triazol-3-yl group, a 1-hydroxy-1H-1,2,3-triazol-5-yl group, a 3-hydroxy-1H-pyrazol-5-yl group, a 1-hydroxy-1H-pyrazol-5-yl group, a 1-hydroxy-1H-imidazol-2-yl group, a 1-hydroxy-1H-imidazol-5-yl group, a 1,1-dioxide-4-oxo-1,2,5-thiadiazolidin-2-yl group, a 1,1-dioxide-3-oxo-1,2,4-thiadiazolidin-4-yl group, a 4-hydroxy-2-oxo-2,5-dihydro-3-furanyl group, a 4-hydroxy-2-oxo-3-cyclopenten-1-yl group, a 1-oxide-4-oxo-3,4-dihydro-2H-1λ⁴,2,5-thiadiazol-1-yl group, a 5-tetrazolyl group, and a 5-oxo-4,5-dihydro-1,2,4-oxadiazol-3-yl group, and the like.

This 5-membered ring having highly acidic hydrogen may be substituted with 1 to 2 substituents. Examples of the substituent include methyl, ethyl, isopropyl, cyclopropyl, cyclopropylmethyl, isopropylmethyl, trifluoromethyl, and difluoromethyl groups, and the like. When the 5-membered ring having highly acidic hydrogen is substituted with two substituents, the two substituents may be the same or different. In addition, two substituents may be taken together to form a cyclopropyl, cyclobutyl, or cyclopentyl ring.

In the present invention, R^{1Y} is preferably R¹, further preferably or R¹ is preferably - CR⁸R⁹-ring 1, more preferably -CH2-ring 1, further preferably a benzyl group which may be substituted with 1 to 5 R^{13Y}s (preferably 1 to 5 R¹³s).

In the present invention, R⁸ is preferably a hydrogen atom.

In the present invention, R⁹ is preferably a hydrogen atom.

In the present invention, ring 1 is preferably a 5 to 6-membered carbocyclic ring or 5 to 6-membered heterocyclic ring which may be substituted with 1 to 5 R^{13Y}s (preferably 1 to 5 R¹³s), more preferably a 5 to 6-membered carbocyclic ring which may be substituted with 1 to 3 R^{13Y}s (preferably 1 to 3 R¹³s), and most preferably benzene which may be substituted with 1 to 5 R^{13Y}s (preferably 1 to 5 R¹³s). Also preferred as ring 1 is a 5 to 6-membered heterocyclic ring which may be substituted with 1 to 3 R^{13Y}s (preferably 1 to 3 R¹³s). Also preferred as ring 1 is a saturated 3 to 15-membered carbocyclic ring which may be substituted with 1 to 3 R^{13Y}s (preferably 1 to 3 R¹³s). The saturated 3 to 15-membered carbocyclic ring is more preferably cyclopropane, cyclobutane, cyclopentane, cyclohexane, or cycloheptane, and most preferably cyclopropane, cyclobutane, or cyclopentane.

In the present invention, ring 1-1 is preferably a 5 to 6-membered carbocyclic ring which may be substituted with 1 to 3 R¹³s, and most preferably benzene which may be substituted with 1 to 5 R¹³s.

In the present invention, R^{13Y} is preferably R¹³, further preferably or R¹³ is preferably halogen, a C1 to 4 alkyl group, a C1 to 4 haloalkyl group, a C1 to 4 haloalkoxy group, a C3 to 6 cycloalkyl group, or a cyano group.

In the present invention, R^{2Y} is preferably R², further preferably or R² is preferably a carboxyl group.

In the present invention, R³ is preferably a 3 to 5-membered saturated carbocyclic ring which may be substituted with 1 to 5 R¹¹s, and more preferably a cyclobutane ring which may be substituted with 1 to 5 R¹¹s. In one embodiment, a hydrogen atom on the 3 to 5-membered saturated carbocyclic ring may be a deuterium atom or a tritium atom.

In the present invention, R¹¹ is preferably halogen, a C1 to 4 alkyl group, a C1 to 4 alkoxy group, a C1 to 4 alkylthio group, or a cyano group.

In the present invention, R^{10Y} is preferably R¹⁰, further preferably or R¹⁰ is preferably a C1 to 4 alkyl group, a C3 to 6 cycloalkyl group, or a di-(C1 to 4 alkyl)amino group.

In the present invention, R¹⁹ is preferably a hydrogen atom or a C1 to 4 alkoxy group.

In the present invention, R²⁰ is preferably a C1 to 4 alkyl group, a C3 to 6 cycloalkyl group, or a di-(C1 to 4 alkyl)amino group.

In the present invention, R^{4Y} is preferably R⁴, further preferably or R⁴ is preferably halogen, a C1 to 4 alkyl group or a C2 to 4 alkenyl group, and more preferably halogen or a C1 to 4 alkyl group. Also preferred is a 5 to 6-membered carbocyclic ring or 5 to 6-membered heterocyclic ring which may be substituted with 1 to 3 R¹⁵s.

In the present invention, R^{5Y} is preferably R⁵, further preferably or R⁵ is preferably halogen or a C1 to 4 alkyl group.

In the present invention, R¹⁵ is preferably halogen or a C1 to 4 alkoxy group.

In the present invention, the 5 to 6-membered carbocyclic ring or 5 to 6-membered heterocyclic ring formed by R⁴ and R⁵ or R^{4Y} and R^{5Y} taken together is preferably a benzene, cyclopentane, cyclohexane, pyridine, pyrimidine, thiophene or thiazole ring, more preferably a benzene, cyclopentane, cyclohexane or thiophene ring, and most preferably a benzene ring.

In the present invention, the heterocyclic ring formed by the pyrrole ring described in general formula (I) or general formula (IY) and R⁴ and R⁵ or R^{4Y} and R^{5Y} as substituents thereof taken together is preferably indole, tetrahydrocyclopentapyrrole, tetrahydroindole or thienopyrrole, and more preferably an indole ring.

In the present invention, R¹² is preferably halogen, a C1 to 4 alkyl group, a C2 to 4 alkynyl group, a C1 to 4 alkoxy group, a cyano group, or a C1 to 4 alkylsulfonyl group.

In the present invention, R⁶ is preferably a hydrogen atom or a C1 to 4 alkyl group, and more preferably a hydrogen atom.

In the present invention, R⁷ is preferably a hydrogen atom or a C1 to 4 alkyl group, and more preferably a hydrogen atom.

In the present invention, R¹⁴⁻¹ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻² is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻³ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻⁴ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻⁵ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻⁶ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, R¹⁴⁻⁷ is preferably a hydrogen atom, halogen, a C1 to 4 alkyl group, a C2 to 4 alkynyl group, a C1 to 4 alkoxy group, a cyano group, or a C1 to 4 alkylsulfonyl group.

In the present invention, ring 2 is preferably a 5 to 6-membered carbocyclic ring which may be substituted with 1 to 3 R¹⁵s, and more preferably a benzene ring which may be substituted with 1 to 3 R¹⁵s. Also preferred as ring 2 is a 5 to 6-membered heterocyclic ring which may be substituted with 1 to 3 R¹⁵s.

In the present invention, p is preferably an integer of 0 to 3.

In the present invention, m is preferably an integer of 0 to 2.

In the present invention, n is preferably an integer of 0 to 1.

In the present invention, q is preferably an integer of 0 to 1.

In the present invention, r_{Y} is preferably an integer of 1 to 4.

In the present invention, the 5-membered ring having highly acidic hydrogen is preferably a 5-tetrazolyl group or a 5-oxo-4,5-dihydro-1,2,4-oxadiazol-3-yl group.

In the present invention, a compound represented by general formula (IY) is preferably general formula (I), further preferably a compound represented by general formula (I-1), a compound represented by general formula (1-2), a compound represented by general formula (1-3), or a compound represented by general formula (1-4), and more preferably a compound represented by general formula (I-1-1).

In the present invention, as the compound represented by general formula (IY), compounds represented by general formula (I-1-2): 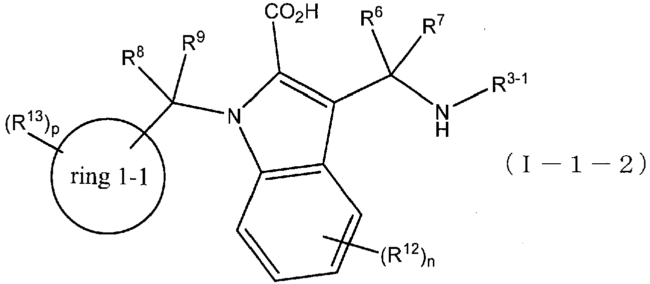 wherein R³⁻¹ represents a 3 to 5-membered saturated carbocyclic ring which may be substituted with 1 to 5 R¹¹s, and other symbols represent the same meaning as described above, are also preferred.

In the present invention, as the compound represented by general formula (IY), compounds represented by general formula (I-1-3): 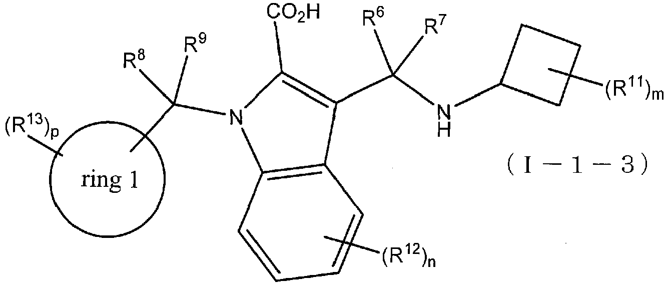 wherein all symbols represent the same meaning as described above, are also preferred.

In the present invention, the compound is preferably, for example,
(1) 3-[(cyclobutylamino)methyl] -1 - [(2,2-difluorocyclopropyl)methyl] -1 H-indole-2-carboxylic acid,
(2) 3-[(cyclobutylamino)methyl]-1-ethyl-1H-indole-2-carboxylic acid,
(3) 3-[(cyclobutylamino)methyl]-1 -(4-methylbenzyl)-1H-indole-2-carboxylic acid,
(4) 1 -(2-chlorobenzyl)-3 - [(cyclobutylamino)methyl] -1 H-indole-2-carboxylic acid,
(5) 3-[(cyclopentylamino)methyl]-1-(4-methylbenzyl)-1H-indole-2-carboxylic acid,
(6) 3-[(cyclopropylamino)methyl]-1-(4-methylbenzyl)-1H-indole-2-carboxylic acid,
(7) 5-bromo-3-[(cyclobutylamino)methyl]-4-methyl-1-(4-methylbenzyl)-1H-pyrrole-2-carboxylic acid,
(8) 3-[(cyclobutylamino)methyl] -1-(3 -cyclopropylbenzyl)-1H-indole-2-carboxylic acid,
(9) 1 -(4-methylbenzyl)-3 - { [(2-methylcyclobutyl)amino]methyl} -1H-indole-2-carboxylic acid,
(10) 3-[(cyclobutylamino)methyl]-1-(4-methylbenzyl)-1H-pyrrolo[2,3-b]pyridine-2-carboxylic acid,
(11) 3 - [(cyclobutylamino)methyl] -1 - [2-(difluoromethoxy)benzyl] -1H-indole-2-carboxylic acid,
(12) 6-[(cyclobutylamino)methyl]-4-(4-methylbenzyl)-4H-thieno[3,2-b]pyrrole-5-carboxylic acid,
(13) 1-(2-chlorobenzyl)-3-[(cyclopentylamino)methyl]-1H-indole-2-carboxylic acid,
(14) 1 -(4-methylbenzyl)-3- [(3 -thiethanylamino)methyl] -1H-indole-2-carboxylic acid,
(15) 1 -(2-chlorobenzyl)-3 - [(cyclopropylamino)methyl] -1H-indole-2-carboxylic acid,
(16) 3-[1-(cyclobutylamino)ethyl]-1-(4-methylbenzyl)-1H-indole-2-carboxylic acid,
(17) 3-[(cyclobutylamino)methyl]-4-(3-fluorophenyl)-5-methyl-1-(4-methylbenzyl)-1H-pyrrole-2-carboxylic acid,
(18) 3-[(cyclobutylamino)methyl]-5-methyl-1-(4-methylbenzyl)-4-vinyl-1H-pyrrole-2-carboxylic acid,
(19) 3-[(cyclobutylamino)methyl]-1-(4-methylbenzyl)-4,5,6,7-tetrahydro-1H-indole-2-carboxylic acid, or
(20) 7-cyano-3-[(cyclobutylamino)methyl]-1-(4-methylbenzyl)-1H-indole-2-carboxylic acid,
or a salt thereof.

In the present invention, the compound represented by general formula (I) or general formula (IY) is preferably 3-[(cyclobutylamino)methyl]-1-(cyclopropylmethyl)-1H-indole-2-carboxylic acid or a salt thereof.

In the present invention, as the compound represented by general formula (I) or general formula (IY), 3-[(cyclobutylamino)methyl]-1-[(2,2-difluorocyclopropyl)methyl]-1H-indole-2-carboxylic acid or a salt thereof is also preferred.

In the present invention, as the compound represented by general formula (I) or general formula (IY), 3-[(cyclobutylamino)methyl]-1-ethyl-1H-indole-2-carboxylic acid or a salt thereof is also preferred.

In the present invention, as the compound represented by general formula (I) or general formula (IY), 3-[(cyclobutylamino)methyl]-1-(4-methylbenzyl)-1H-indole-2-carboxylic acid or a salt thereof is also preferred.

In the present invention, as the compound represented by general formula (I) or general formula (IY), 1-(2-chlorobenzyl)-3-[(cyclobutylamino)methyl]-1H-indole-2-carboxylic acid or a salt thereof is also preferred.

In the present invention, as the compound represented by general formula (I) or general formula (IY), 5-bromo-3-[(cyclobutylamino)methyl]-4-methyl-1-(4-methylbenzyl)-1H-pyrrole-2-carboxylic acid or a salt thereof is also preferred.

In the present invention, as the compound represented by general formula (I) or general formula (IY), 3-[(cyclobutylamino)methyl]-1-(3-cyclopropylbenzyl)-1H-indole-2-carboxylic acid or a salt thereof is also preferred.

In the present invention, as the compound represented by general formula (I) or general formula (IY), 1-(4-methylbenzyl)-3-{[(2-methylcyclobutyl)amino]methyl}-1H-indole-2-carboxylic acid or a salt thereof is also preferred.

In the present invention, as the compound represented by general formula (I) or general formula (IY), 3-[(cyclobutylamino)methyl]-1-(4-methylbenzyl)-1H-pyrrolo[2,3-b]pyridine-2-carboxylic acid or a salt thereof is also preferred.

In the present invention, as the compound represented by general formula (I) or general formula (IY), 3-[(cyclobutylamino)methyl]-1-[2-(difluoromethoxy)benzyl]-1H-indole-2-carboxylic acid or a salt thereof is also preferred.

In the present invention, as the compound represented by general formula (I) or general formula (IY), 6-[(cyclobutylamino)methyl]-4-(4-methylbenzyl)-4H-thieno[3,2-b]pyrrole-5-carboxylic acid or a salt thereof is also preferred.

In the present invention, as the compound represented by general formula (I) or general formula (IY), 1-(4-methylbenzyl)-3-[(3-thiethanylamino)methyl]-1H-indole-2-carboxylic acid or a salt thereof is also preferred.

In the present invention, as the compound represented by general formula (I) or general formula (IY), 3-[1-(cyclobutylamino)ethyl]-1-(4-methylbenzyl)-1H-indole-2-carboxylic acid or a salt thereof is also preferred.

In the present invention, as the compound represented by general formula (I) or general formula (IY), 3-[(cyclobutylamino)methyl]-4-(3-fluorophenyl)-5-methyl-1-(4-methylbenzyl)-1H-pyrrole-2-carboxylic acid or a salt thereof is also preferred.

In the present invention, as the compound represented by general formula (I) or general formula (IY), 3-[(cyclobutylamino)methyl]-5-methyl-1-(4-methylbenzyl)-4-vinyl-1H-pyrrole-2-carboxylic acid or a salt thereof is also preferred.

In the present invention, as the compound represented by general formula (I) or general formula (IY), 3-[(cyclobutylamino)methyl]-1-(4-methylbenzyl)-4,5,6,7-tetrahydro-1H-indole-2-carboxylic acid or a salt thereof is also preferred.

In the present invention, as the compound represented by general formula (I) or general formula (IY), 7-cyano-3-[(cyclobutylamino)methyl]-1-(4-methylbenzyl)-1H-indole-2-carboxylic acid or a salt thereof is also preferred.

### [Isomer]

In the present invention, unless otherwise noted, isomers are all encompassed. For example, alkyl groups, alkoxy groups and the like include straight-chain and branched ones. Furthermore, isomers (E, Z, cis, trans isomers) in a double bond, ring or condensed ring, isomers due to the presence of asymmetric carbon or the like (R-, S-form, α-, β-configuration, enantiomers, diastereomers), optically active substances with racemic properties (D, L, d, l-forms), polar compounds obtained by chromatographic separation (high-polarity compounds, Low-polarity compounds), equilibrium compounds, rotational isomers, and mixtures and racemic mixtures thereof in any proportion are all encompassed by the present invention. Moreover, in the present invention, all isomers due to tautomerism are also included.

### [Salt, solvate]

Salts of the compounds represented by general formula (IY) disclosed in the present invention include all pharmacologically acceptable salts. The pharmacologically acceptable salt is preferably a water-soluble salt with low toxicity. Examples of suitable salts include acid addition salts (for example, inorganic acid salts [e.g., hydrochlorides, hydrobromides, hydroiodides, sulfates, phosphates, nitrates, etc.], organic acid salts [e.g., acetates, trifluoroacetates, lactates, tartrates, oxalates, fumarates, maleates, benzoates, citrates, methanesulfonates, ethanesulfonates, benzenesulfonates, toluenesulfonates, isethionates, glucuronates, gluconates, etc.], salts with acidic natural amino acids [e.g., aspartates, glutamates, etc.], etc.), and the like.

Furthermore, salts also include quaternary ammonium salts. The quaternary ammonium salt represents a salt obtained by quaternizing a nitrogen atom of the compound represented by general formula (IY) with an R⁰ group. Here, the R⁰ group represents, for example, a C1-8 alkyl group which may be substituted with a phenyl group.

The compound represented by general formula (IY) can be converted into the above-described salt, an N-oxide or a solvate by a known method.

The N-oxide of the compound represented by general formula (IY) represents a compound in which the nitrogen atom of the compound represented by general formula (IY) is oxidized. In addition, such an N-oxide may form a salt such as the above-described acid adduct salt.

The compound represented by general formula (IY), the salt thereof or the N-oxide thereof may form a solvate with, for example, water, an alcohol-based solvent (for example, ethanol, etc.), or the like. The solvate is preferably low-toxic and water-soluble.

The compound represented by general formula (IY) and the salt thereof may exist in an unsolvated form or may exist in a solvated form with a pharmaceutically acceptable solvent such as water or ethanol. The solvate is preferably a hydrate. The compound represented by general formula (IY) and the salt thereof can be converted into a solvate by a known method.

The compound represented by general formula (IY) and the salt thereof can form co-crystals with a suitable co-crystal forming agent. The co-crystal is preferably a pharmaceutically acceptable one formed with a pharmaceutically acceptable co-crystal forming agent. A co-crystal is typically defined as a crystal in which two or more different molecules are formed by intermolecular interactions that differ from ionic bonds. Further, the co-crystal may be a complex of a neutral molecule and a salt. The co-crystal can be prepared by known methods, such as by melt crystallization, by recrystallization from a solvent, or by physically pulverizing a component together. Suitable co-crystal forming agent includes those described in WO 2006/007448 A.

In the present invention, all references to the compound of the present invention include compounds represented by general formula (IY), salts thereof, solvates (for example, hydrates) thereof, N-oxides thereof, or co-crystals thereof, or solvates (for example, hydrates) of salts of compounds represented by general formula (IY), N-oxides thereof, or co-crystals thereof.

### [Prodrug]

The compound represented by general formula (IY) may be administered as a prodrug. A prodrug of the compound represented by general formula (IY) refers to a compound that is converted into the compound represented by general formula (IY) by a reaction with an enzyme, gastric acid or the like in vivo. Examples of the prodrug of the compound represented by general formula (IY) include compounds in which, when the compound represented by general formula (IY) has an amino group, the amino group is acylated, alkylated or phosphorylated (for example, compounds in which the amino group of the compound represented by general formula (IY) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated or tert-butylated, etc.), and the like; and compounds in which, when the compound represented by general formula (IY) has a hydroxyl group, the hydroxyl group is acylated, alkylated, phosphorylated or borated (for example, compounds in which the hydroxyl group of the compound represented by general formula (IY) is acetylated, palmitoylated, propanoylated, pivaloylated, succinylated, fumarylated, alanylated or dimethylaminomethylcarbonylated, etc.), and the like. In addition, the prodrug of the compound represented by general formula (IY) may be changed to the compound represented by general formula (IY) under physiological conditions described in "Development of Drug" by Hirokawa Shoten, Vol. 7, "Molecular Design", pp. 163-198 (published in 1990). The prodrugs of the compounds represented by general formula (IY) can be produced by a method known per se. Similarly to the compound represented by general formula (IY), the prodrug of the compound represented by general formula (IY) may form a salt such as the above-described acid adduct salt, or may form a solvate with, for example, water, an alcohol-based solvent (for example, ethanol, etc.), or the like.

### [Labeled compound]

In the present invention, the compound represented by general formula (IY) or the salt thereof also includes so-called labeled compounds in which some or all of the atoms constituting them are replaced by their isotopes. These labeled compounds can be produced by a method known per se. As isotopes used for labeling, for example, ²H, ³H, ¹³C, ¹⁴C, ¹⁵N, ¹⁶N, ¹⁷O, ¹⁸O, ³⁵S, ³⁶Cl, ⁷⁷Br, ¹²⁵I and the like can be suitably used, but are not limited thereto.

### [Production method]

### [Method for producing compound of present invention]

The compound represented by general formula (IY) or the salt thereof can be produced by appropriately improving a known method, for example, a method shown below, a method equivalent to these, a method shown in Examples, a method equivalent to Examples, or a method described in [Comprehensive Organic Transformations: A Guide to Functional Group Preparations, 2nd Edition (Richard C. Larock, John Wiley & Sons Inc, 1999)] and the like, and using a combination of them, but the method is not limited thereto. In each of the following production methods, a raw material compound forming a salt may be used. Examples of such a salt include those described as the salt of the compound represented by general formula (IY) described above.

Among the compounds of the present invention represented by general formula (IY), a compound wherein R^{1Y}, R^{4Y} and R^{5Y} are R¹, R⁴ and R⁵, respectively, and R^{2Y} is a carboxyl group, that is, a compound represented by general formula (IA) 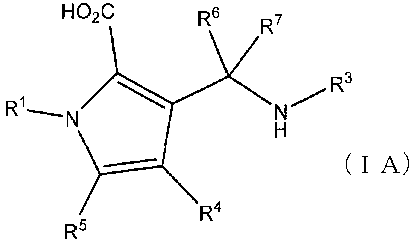
wherein all symbols represent the same meaning as described above, is a compound represented by the present invention wherein R^{2Y} is a C1 to 4 alkoxycarbonyl group, that is, a compound represented by general formula (IIA): 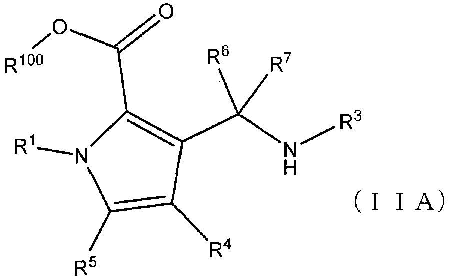
wherein R¹⁰⁰ represents a C1 to 4 alkyl group, and other symbols represent the same meaning as described above, can be produced by subjecting to hydrolysis reaction.

This hydrolysis reaction (deprotection reaction of a carboxyl group) is known, and examples thereof include alkaline hydrolysis and the like. Deprotection reaction by alkaline hydrolysis is carried out, for example, in an organic solvent (methanol, tetrahydrofuran, dioxane, etc.), by using an alkali metal hydroxide (sodium hydroxide, potassium hydroxide, lithium hydroxide, etc.), an alkaline earth metal hydroxide (barium hydroxide, calcium hydroxide, etc.) or a carbonate (sodium carbonate, potassium carbonate, etc.) or an aqueous solution thereof or a mixture thereof at a temperature of 0 to -100°C,

If further required, the reaction may be followed by an operation of converting into a desired salt by a known method.

Among the compounds of the present invention represented by general formula (IY), a compound wherein R^{1Y}, R^{10Y}, R^{4Y} and R^{5Y} are R¹, R¹⁰, R⁴ and R⁵, respectively, and R^{2Y} is -CONHSO₂R¹⁰, that is, a compound represented by general formula (IB): 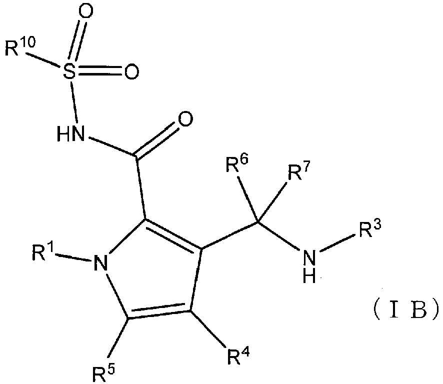
wherein all symbols represent the same meaning as described above, can be produced by subjecting to deprotection reaction a protecting group (P¹) of an amino group of a compound represented by general formula (IIB): 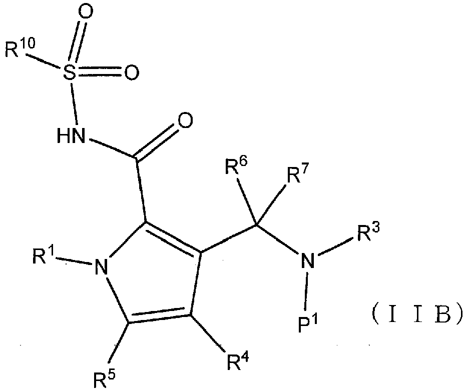
wherein P¹ represents a protecting group of the amino group, and other symbols represent the same meaning as described above.

Examples of the protecting group of the amino group include a benzyloxycarbonyl group, a tert-butoxycarbonyl group, an allyloxycarbonyl (Alloc) group, a 1-methyl-1-(4-biphenyl)ethoxycarbonyl (Bpoc) group, a trifluoroacetyl group, a 9-fluorenylmethoxycarbonyl group, a benzyl (Bn) group, a p-methoxybenzyl group, a benzyloxymethyl (BOM) group, a 2-(trimethylsilyl)ethoxymethyl (SEM) group, and the like.

Deprotection reaction of the protecting group of the amino group is known, and examples thereof include:
(1) deprotection reaction by alkaline hydrolysis,
(2) deprotection reaction under acidic conditions,
(3) deprotection reaction by hydrogenolysis,
(4) deprotection reaction of a silyl group,
(5) deprotection reaction using metal,
(6) deprotection reaction using a metal complex, and the like.

These methods are specifically described hereinbelow.
(1) Deprotection reaction by alkaline hydrolysis is carried out, for example, in an organic solvent (methanol, tetrahydrofuran, dioxane, etc.), by using an alkali metal hydroxide (sodium hydroxide, potassium hydroxide, lithium hydroxide, etc.), an alkaline earth metal hydroxide (barium hydroxide, calcium hydroxide, etc.) or a carbonate (sodium carbonate, potassium carbonate, etc.) or an aqueous solution thereof or a mixture thereof at a temperature of 0 to 40°C.
(2) Deprotection reaction under acidic conditions is carried out, for example, in an organic solvent (dichloromethane, chloroform, dioxane, ethyl acetate, anisole, etc.), in an organic acid (acetic acid, trifluoroacetic acid, methanesulfonic acid, p-tosyl acid, etc.) or an inorganic acid (hydrochloric acid, sulfuric acid, etc.) or a mixture thereof (hydrogen bromide/acetic acid, etc.) at a temperature of 0 to 100°C.
(3) Deprotection reaction by hydrogenolysis is carried out, for example, in a solvent (an ether-based solvent (tetrahydrofuran, dioxane, dimethoxyethane, diethyl ether, etc.), an alcohol-based solvent (methanol, ethanol, etc.), a benzene-based solvent (benzene, toluene, etc.), a ketone-based solvent (acetone, methyl ethyl ketone, etc.), a nitrile-based solvent (acetonitrile, etc.), an amide-based solvent (dimethylformamide, etc.), water, ethyl acetate, acetic acid or mixed solvents of two or more of thereof, etc.), in the presence of a catalyst (palladium-carbon, palladium black, palladium hydroxide, platinum oxide, Raney nickel, etc.), in a hydrogen atmosphere of normal or increased pressure or in the presence of ammonium formate at a temperature of 0 to 200°C.
(4) Deprotection reaction of a silyl group is carried out, for example, in a water-miscible organic solvent (tetrahydrofuran, acetonitrile, etc.), by using tetrabutylammonium fluoride at a temperature of 0 to 40°C.
(5) Deprotection reaction using metal is carried out, for example, in an acidic solvent (a mixed solvent of acetic acid, a buffer of pH 4.2 to 7.2 or a solution thereof with an organic solvent such as tetrahydrofuran), in the presence of zinc powder, with application of ultrasonic if necessary, at a temperature of 0 to 40°C.
(6) Deprotection reaction using a metal complex is carried out, for example, in an organic solvent (dichloromethane, dimethylformamide, tetrahydrofuran, ethyl acetate, acetonitrile, dioxane, ethanol, etc.), water or a mixed solvent thereof, in the presence of a trap reagent (tributyltin hydride, triethylsilane, dimedone, morpholine, diethylamine, pyrrolidine, etc.), an organic acid (acetic acid, formic acid, 2-ethylhexanoic acid, etc.) and/or a salt of an organic acid (sodium 2-ethylhexanoate, potassium 2-ethylhexanoate, etc.), in the presence or absence of a phosphine reagent (triphenylphosphine, etc.), by using a metal complex (tetrakistriphenylphosphine palladium(0), bis(triphenylphosphine)palladium(II) dichloride, palladium(II) acetate, tris(triphenylphosphine)rhodium(I) chloride, etc.) at a temperature of 0 to 40°C.

In addition to the above, for example, deprotection reaction can be carried out by the methods described in "Comprehensive Organic Transformations: A Guide to Functional Group Preparations 2nd Edition (Richard C. Larock, John Wiley & Sons Inc, 1999)" and "P. G. M. Wuts, T. W. Greene, Green's Protective Groups in Organic Synthesis, Wiley, Fourth Edition, New York, 2007".

As can be easily understood by those skilled in the art, the target compound represented by general formula (IB) can be easily produced by properly using these deprotection reactions.

If further required, the reaction may be followed by an operation of converting into a desired salt by a known method.

Among the compounds of the present invention represented by general formula (IIA), a compound in which R⁷ is a hydrogen atom or a deuterium atom, that is, a compound represented by general formula (IIA-1) can be produced by the method described in the following reaction step formula 1. In reaction step formula 1, X¹ represents a halogen atom, mesylate, tosylate, triflate or hydroxyl group, and other symbols represent the same meaning as described above.

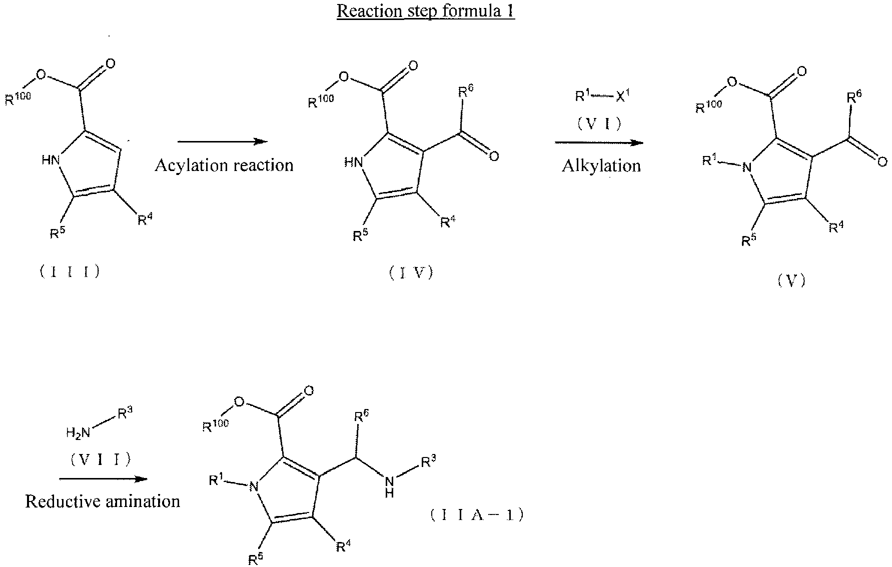

A compound represented by general formula (IV) can be produced by subjecting a compound represented by general formula (III) to acylation reaction.

When R⁶ is a hydrogen atom or a deuterium atom, the compound represented by general formula (IV) can be produced, for example, by adding a formylating agent (e.g., N,N-dimethylformamide, N-methylformanilide, or a corresponding deuterium substitution reagent) in an organic solvent (e.g., 1,2-dichloroethane, tetrahydrofuran, or mixed solvents thereof, etc.), in the presence of a dehydrating chlorinating agent (e.g., phosphorus oxychloride, thionyl chloride, etc.), and reacting the mixture at 0°C to reflux temperature.

When R⁶ is a C1 to 4 alkyl group or a C1 to 4 haloalkyl group, for example, the compound represented by general formula (IV) can be produced, for example, by adding a corresponding carboxylic acid in an organic solvent (e.g., dichloromethane, acetonitrile, tetrahydrofuran, or mixed solvents thereof, etc.), in the presence of an activating agent (e.g., trifluoroacetic anhydride, polyphosphoric acid, etc.), and reacting the mixture at 0°C to reflux temperature.

A compound represented by general formula (V) can be produced by subjecting the compound represented by general formula (IV) and the compound represented by general formula (VI) to alkylation reaction.

This alkylation reaction is known, and can be carried out by any of the following methods: (1) a substitution reaction when X¹ is a halogen atom, mesylate, tosylate, or triflate; and (2) a Mitsunobu reaction when X¹ is a hydroxyl group.
(1) The substitution reaction is known and is carried out by reacting at 0 to 100°C, for example, in an organic solvent (for example, dimethylformamide, dimethylacetamide, dimethylsulfoxide, chloroform, dichloromethane, diethyl ether, tetrahydrofuran, methyl t-butyl ether), in the presence of an alkali metal hydride (for example, sodium hydride), or a carbonate (for example, cesium carbonate, potassium carbonate, sodium carbonate) or an aqueous solution thereof, or a mixture thereof.
(2) The Mitsunobu reaction is known, and is carried out by reacting at 0 to 100°C, for example, in an organic solvent (for example, dichloromethane, diethyl ether, tetrahydrofuran, acetonitrile, benzene, toluene), in the presence of (1) an azo compound (for example, diethyl azodicarboxylic acid (DEAD), diisopropyl azodicarboxylate, 1,1'-(azodicarbonyl)dipiperidine (ADDP), 1,1'-azobis(N,N-dimethylformamide)) and a phosphine compound (for example, triphenylphosphine, tributylphosphine, trimethylphosphine, polymer support triphenylphosphine) or in the presence of (2) an ylide compound (cyanomethylene trimethylphosphorane, cyanomethylene tributylphosphorane).

A compound represented by general formula (IIA-1) can be produced by subjecting the compound represented by general formula (V) and a compound represented by general formula (VII) to reductive amination reaction.

This reductive amination reaction is known, and an imine produced in the reaction may be isolated and then reduced, or the imine may be produced in the reaction system and reduced without isolation (in one pot). This imine production reaction is known, and can be carried out, for example, in an organic solvent (e.g., methanol, ethanol, dichloromethane, chloroform, dichloroethane, benzene, toluene, or a mixed solvent thereof, or the like), in the presence or absence of a dehydrating agent (e.g., anhydrous magnesium sulfate, molecular sieve (trade name), or the like), in the presence or absence of an acid (e.g., hydrochloric acid, acetic acid, or the like), at 20°C to reflux temperature. The reduction reaction of imine is also known, and can be carried out, for example, in an organic solvent (e.g., tetrahydrofuran, diethyl ether, dichloroethane, dichloromethane, dimethylformamide, acetic acid, methanol, ethanol, or a mixture thereof, or the like), in the presence of a reducing agent (e.g., sodium triacetoxyborohydride, sodium cyanoborohydride, sodium borohydride, zinc borohydride, diisobutylaluminum hydride, 2-picoline borane complex, or the like, or a corresponding deuterium substitution reagent) at a temperature of 0 to 40°C, or can be carried out in a solvent (e.g., ether-based (e.g., tetrahydrofuran, dioxane, dimethoxyethane, diethyl ether, or the like), alcohol-based (e.g., methanol, ethanol, or the like), benzene-based (e.g., benzene, toluene, or the like), nitrile-based (e.g., acetonitrile or the like), amide-based (e.g., dimethylformamide or the like), water, ethyl acetate, acetic acid, or a mixed solvent thereof, or the like), in the presence of a catalyst (e.g., palladium-carbon, palladium black, palladium hydroxide, platinum oxide, Raney nickel, or the like) under normal pressure or pressure of hydrogen, or a deuterium atmosphere at a temperature of 0 to 200°C. In addition, a reductive amination reaction that is carried out without isolating imine is known, and for example, the reductive amination reaction can be carried out in an organic solvent (e.g., dichloroethane, dichloromethane, dimethylformamide, acetic acid, or a mixture thereof, or the like), in the presence of a reducing agent (e.g., sodium triacetoxyborohydride, sodium cyanoborohydride, sodium borohydride, 2-picoline borane complex, or the like, or a corresponding deuterium substitution reagent) at a temperature of 0 to 40°C.

The reaction can also be carried out, for example, in an organic solvent (e.g., dichloroethane, acetonitrile, or a mixed solvent thereof, or the like), in the presence or absence of a tertiary amine (e.g., triethylamine, diisopropylethylamine, or the like) and a Lewis acid (e.g., titanium tetrachloride or the like) at -50°C to 80°C, and further in the presence of a reducing agent (e.g., sodium triacetoxyborohydride, sodium cyanoborohydride, 2-picoline borane complex, or the like, or a corresponding deuterium substitution reagent) at a temperature of 0 to 80°C.

If further required, the reaction may be followed by an operation of converting into a desired salt by a known method.

Among the compounds of the present invention represented by general formula (IIA), a compound in which R⁷ is a C1 to 4 alkyl group or a C1 to 4 haloalkyl group, that is, a compound represented by general formula (IIA-2) can be produced by the method described in the following reaction step formula 2. In reaction step formula 2, R⁷⁻¹ represents a C1 to 4 alkyl group or a C1 to 4 haloalkyl group, M represents an organic metal (for example, magnesium halide, lithium, or the like) or organosilicon (for example, trimethylsilane or the like), and other symbols represent the same meaning as described above.

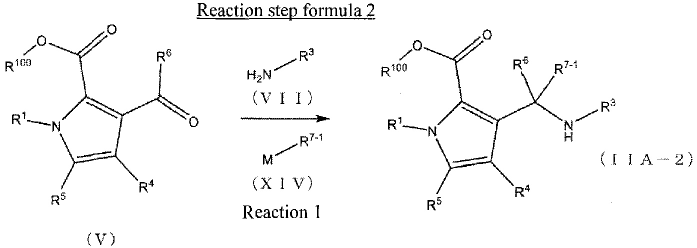

The compound represented by general formula (IIA-2) can be produced by reaction 1, using the compound represented by general formula (V), the compound represented by general formula (VII), and a compound represented by general formula (XIV).

Reaction 1 is a reaction for producing an imine and a subsequent nucleophilic reaction of R⁷⁻¹-M to an imine, and the imine produced in the reaction may be isolated and then subjected to nucleophilic reaction, or an imine may be produced in the reaction system and then subjected to nucleophilic reaction without isolation (in one pot). This imine production reaction is known, and can be carried out, for example, in an organic solvent (e.g., methanol, ethanol, dichloromethane, chloroform, dichloroethane, benzene, toluene, or a mixed solvent thereof, or the like), in the presence or absence of a dehydrating agent (e.g., anhydrous magnesium sulfate, molecular sieve (trade name), or the like), in the presence or absence of an acid (e.g., hydrochloric acid, acetic acid, or the like), at 20°C to reflux temperature.

The reaction can also be carried out, for example, by reacting at 0 to 40°C in an organic solvent (e.g., dichloroethane, dichloromethane, or a mixed solvent thereof, or the like), in the presence of a tertiary amine (e.g., triethylamine, diisopropylethylamine, or the like), by using a Lewis acid (e.g., titanium tetrachloride or the like).

The nucleophilic reaction of R⁷⁻¹-M with imine is also known, and is carried out, for example, by reacting an organometallic reagent corresponding to R⁷⁻¹, for example, alkyl (R⁷⁻¹) magnesium bromide, alkyl (R⁷⁻¹) lithium or the like at -78°C to reflux temperature in an organic solvent (e.g., tetrahydrofuran, diethyl ether, dichloroethane, dichloromethane, dimethylformamide, or a mixture thereof, or the like), in the presence or absence of an activating agent (e.g., boron trifluoride diethyl ether complex, trifluoroacetic acid, potassium hydrogen fluoride, or the like). It is also carried out by reacting an organosilicon reagent corresponding to R⁷⁻¹, for example, a Rupert reagent (trifluoromethyltrimethylsilane) at -78°C to reflux temperature.

If further required, the reaction may be followed by an operation of converting into a desired salt by a known method.

In addition, the compound represented by general formula (IIA) can also be produced by reaction step formula 3, using the compound represented by general formula (V).

In reaction step formula 3, LG represents a leaving group (for example, halogen, a p-toluenesulfonyloxy group, a methanesulfonyloxy group, or the like), and other symbols represent the same meaning as described above.

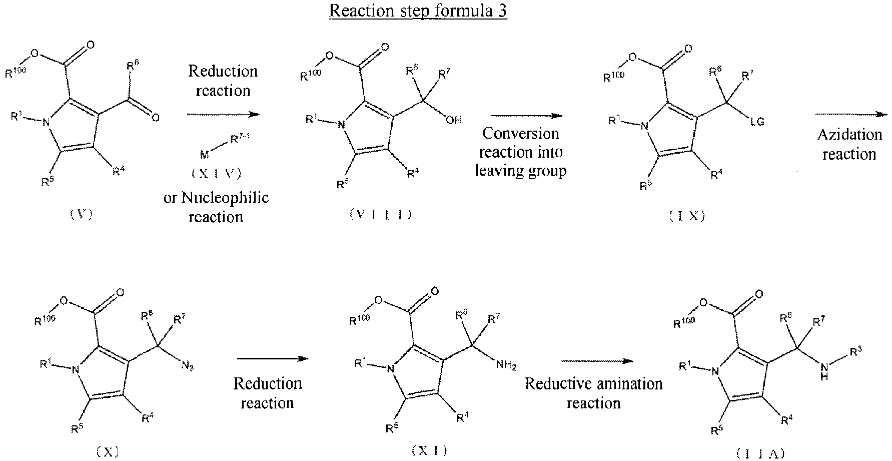

A compound represented by general formula (VIII) can be produced by subjecting the compound represented by general formula (V) to reduction reaction or a nucleophilic reaction to a carbonyl group of R⁷⁻¹-M.

This reduction reaction is known, and can be carried out, for example, by reacting a reducing agent (sodium borohydride, lithium borohydride, lithium aluminum hydride, diisobutylaluminum hydride, or the like) in an organic solvent (methanol, ethanol, tetrahydrofuran, hexane, or the like) at a temperature of -78 to 80°C.

This nucleophilic reaction to the carbonyl group of R⁷⁻¹-M is also known, and is carried out, for example, by reacting an organometallic reagent corresponding to R⁷⁻¹, for example, alkyl (R⁷⁻¹) magnesium bromide, alkyl (R⁷⁻¹) lithium or the like, in an organic solvent (tetrahydrofuran, diethyl ether, or the like), in the presence or absence of cerium chloride at -78°C to room temperature. In addition, the reaction can be carried out by reacting an organosilicon reagent corresponding to R⁷⁻¹, for example, a Rupert reagent (trifluoromethyltrimethylsilane) in an organic solvent (tetrahydrofuran, diethyl ether, or the like), in the presence of tetrabutylammonium fluoride at -78°C to room temperature.

A compound represented by general formula (IX) can be produced by subjecting the compound represented by general formula (VIII) to a conversion reaction into a leaving group.

This conversion reaction into a leaving group is known, and when LG is halogen, it can be carried out, for example, by adding a halogenating agent (for example, phosphorus tribromide, thionyl chloride, or the like) in an organic solvent (for example, dichloromethane, acetonitrile, toluene), and reacting the mixture at -50°C to reflux temperature. In addition, the reaction can also be carried out, for example, by adding a halogenating agent (for example, iodine, carbon tetrabromide, carbon tetrachloride, or the like) in an organic solvent (for example, dichloromethane, acetonitrile, tetrahydrofuran), in the presence of triphenylphosphine, and reacting the mixture at -50°C to reflux temperature.

When LG is a p-toluenesulfonyloxy group, a methanesulfonyloxy group, or the like, the reaction can be carried out, for example, by adding sulfonyl chloride (for example, methanesulfonyl chloride, p-toluenesulfonyl chloride, or the like) in an organic solvent (for example, tetrahydrofuran, dichloromethane, toluene), in the presence or absence of a base [alkylamine (for example, triethylamine, diisopropylethylamine, or the like), aromatic amine (for example, pyridine, 4-dimethylaminopyridine, or the like), or alkali metal hydride (for example, sodium hydride, potassium hydride, or the like)], and reacting the mixture at -78 to 50°C.

The compound represented by general formula (X) can be produced by subjecting the compound represented by general formula (IX) to azidation reaction.

This azidation reaction is known, and can be carried out, for example, by adding an azidation agent (for example, sodium azide, potassium azide) in an organic solvent (for example, tetrahydrofuran, dimethylsulfoxide, N,N-dimethylformamide, acetonitrile), in the presence or absence of an activating agent (for example, sodium iodide), and reacting the mixture at -50°C to reflux temperature.

The compound represented by general formula (XI) can be produced by subjecting the compound represented by general formula (X) to reduction reaction.

This reduction reaction is known, and examples thereof include (1) reduction reaction using hydrogen, (2) hydride reduction reaction, (3) reduction reaction using silane, (4) Staudinger reaction, and (5) reduction reaction using metal.

These methods are specifically described hereinbelow.
(1) The reduction reaction using hydrogen is carried out, for example, in the presence of a catalyst (for example, palladium-carbon) by adding hydrogen gas in an organic solvent (for example, methanol, dioxane, tetrahydrofuran) at 0 to 100°C.
(2) The hydride reduction reaction is carried out, for example, by reacting at a temperature of 0 to 100°C in an organic solvent (for example, methanol, dimethyl ether), by using a hydride reducing agent (for example, sodium borohydride, zinc borohydride, sodium cyanohydride, lithium triethylborohydride).
(3) The reduction reaction using silane is carried out, for example, by reacting at a temperature of 0 to 100°C in an organic solvent (for example, dioxane, toluene), by using a silane compound (for example, phenylsilane, triethylsilane, tris(trimethylsilyl)silane).
(4) The Staudinger reaction is carried out in an organic solvent (for example, methanol, dichloromethane, tetrahydrofuran), by using a phosphine compound (for example, triphenylphosphine, tributylphosphine, trimethylphosphine) at 0 to 100°C.
(5) The reduction reaction using metal is carried out, for example, by reacting at a temperature of 0 to 100°C in an acidic solvent (for example, a mixed solution of acetic acid, ammonium acetate, a buffer of pH 4.2 to 7.2 or a solution thereof with an organic solvent such as tetrahydrofuran), by using metal (for example, copper, zinc).

The compound represented by general formula (IIA) can be produced by subjecting the compound represented by general formula (XI) to reductive amination reaction.

This reductive amination reaction is known, and can be carried out by using a carbonyl compound corresponding to R³. The imine produced in the reaction may be isolated and then reduced, or the imine may be produced in the reaction system and reduced without isolation (in one pot). This imine production reaction is known, and can be carried out, for example, in an organic solvent (e.g., methanol, ethanol, dichloromethane, chloroform, dichloroethane, benzene, toluene, or a mixed solvent thereof, or the like), in the presence or absence of a dehydrating agent (e.g., anhydrous magnesium sulfate, molecular sieve (trade name), or the like), in the presence or absence of an acid (e.g., hydrochloric acid, acetic acid, or the like), at 20°C to reflux temperature. The reduction reaction of imine is also known, and can be carried out, for example, in an organic solvent (e.g., tetrahydrofuran, diethyl ether, dichloroethane, dichloromethane, dimethylformamide, acetic acid, methanol, ethanol, or a mixture thereof, or the like), in the presence of a reducing agent (e.g., sodium triacetoxyborohydride, sodium cyanoborohydride, sodium borohydride, zinc borohydride, diisobutylaluminum hydride, 2-picoline borane complex, or the like, or a corresponding deuterium substitution reagent) at a temperature of 0 to 40°C, or can be carried out in a solvent (e.g., ether-based (e.g., tetrahydrofuran, dioxane, dimethoxyethane, diethyl ether, or the like), alcohol-based (e.g., methanol, ethanol, or the like), benzene-based (e.g., benzene, toluene, or the like), nitrile-based (e.g., acetonitrile or the like), amide-based (e.g., dimethylformamide or the like), water, ethyl acetate, acetic acid, or a mixed solvent thereof, or the like), in the presence of a catalyst (e.g., palladium-carbon, palladium black, palladium hydroxide, platinum oxide, Raney nickel, or the like) under normal pressure or pressure of hydrogen, or a deuterium atmosphere at a temperature of 0 to 200°C. In addition, a reductive amination reaction that is carried out without isolating imine is known, and for example, the reductive amination reaction can be carried out in an organic solvent (e.g., dichloroethane, dichloromethane, dimethylformamide, acetic acid, or a mixture thereof, or the like), in the presence of a reducing agent (e.g., sodium triacetoxyborohydride, sodium cyanoborohydride, sodium borohydride, 2-picoline borane complex, or the like, or a corresponding deuterium substitution reagent) at a temperature of 0 to 40°C.

The reaction can also be carried out, for example, in an organic solvent (e.g., dichloroethane, acetonitrile, or a mixed solvent thereof, or the like), in the presence or absence of a tertiary amine (e.g., triethylamine, diisopropylethylamine, or the like) and a Lewis acid (e.g., titanium tetrachloride or the like) at -50°C to 80°C, and further in the presence of a reducing agent (e.g., sodium triacetoxyborohydride, sodium cyanoborohydride, 2-picoline borane complex, or the like, or a corresponding deuterium substitution reagent) at a temperature of 0 to 80°C.

A compound represented by general formula (IIB) can be produced by the method described in the following reaction step formula 4. In reaction step formula 4, X² represents, for example, a halogen atom, mesylate, tosylate, triflate, tert-butyloxycarbonyloxy, or the like, and other symbols represent the same meaning as described above.

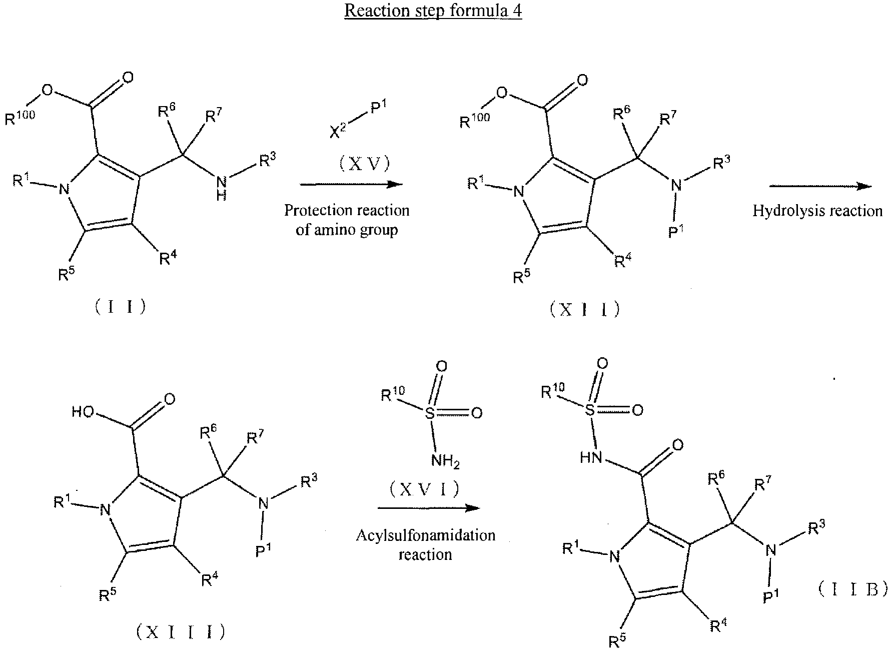

A compound represented by general formula (XII) can be produced by subjecting a compound represented by general formula (II) and a compound represented by general formula (XV) to a protection reaction of an amino group.

The protection reaction of an amino group is known, and for example, the protecting group of an amino group can be introduced using the protecting group introduction reaction described in "P. G. M. Wuts, T. W. Greene, Green's Protective Groups in Organic Synthesis, Wiley, Fourth Edition, New York, 2007".

The compound represented by general formula (XIII) can be produced by subjecting the compound represented by general formula (XII) to hydrolysis reaction.

This hydrolysis reaction is known, and is, for example, a reaction having the same contents as the "hydrolysis reaction" described as the method for producing the compound represented by general formula (IA) described above.

The compound represented by general formula (IIB) can be produced by subjecting the compound represented by general formula (XIII) and a compound represented by general formula (XVI) to acylsulfonamidation reaction.

This acylsulfonamidation reaction is known, and examples thereof include:
(1) a method using an acid halide,
(2) a method using a mixed acid anhydride,
(3) a method using a condensing agent, and the like.

These methods are specifically described hereinbelow.
(1) The method using an acid halide is carried out, for example, by reacting carboxylic acid with an acid halogenating agent (oxalyl chloride, thionyl chloride, or the like) in an organic solvent (chloroform, dichloromethane, diethylether, tetrahydrofuran, dimethoxyethane, or the like) or in the absence of a solvent at about -20°C to reflux temperature, and reacting the obtained acid halide with a sulfonamide corresponding to R¹⁰ (NH₂SO₂R¹⁰), in the presence of a base (pyridine, triethylamine, dimethylaniline, dimethylaminopyridine, diisopropylethylamine, or the like) in an organic solvent (chloroform, dichloromethane, diethylether, tetrahydrofuran, acetonitrile, ethyl acetate, or the like) at a temperature of about 0 to 40°C. Furthermore, the reaction can also be performed by reacting the obtained acid halide with a sulfonamide corresponding to R¹⁰ (NH₂SO₂R¹⁰) at about 0 to 40°C, by using an aqueous alkali solution (sodium bicarbonate solution, sodium hydroxide solution, or the like) in an organic solvent (dioxane, tetrahydrofuran, dichloromethane, or the like), in the presence or absence of a phase transfer catalyst (a quaternary ammonium salt such as tetrabutylammonium chloride, triethylbenzylammonium chloride, tri-n-octylmethylammonium chloride, trimethyldecylammonium chloride or tetramethylammonium bromide, or the like).
(2) The method using a mixed acid anhydride is carried out, for example, by reacting carboxylic acid with an acid halide (pivaloyl chloride, tosyl chloride, mesyl chloride, or the like) or an acid derivative (ethyl chloroformate, isobutyl chloroformate, or the like) at about 0 to 40°C, in the presence of a base (pyridine, triethylamine, dimethylaniline, dimethylaminopyridine, diisopropylethylamine, or the like) in an organic solvent (chloroform, dichloromethane, diethylether, tetrahydrofuran, or the like) or in the absence of a solvent, and reacting the obtained mixed acid anhydride with a sulfonamide corresponding to R¹⁰ (NH₂SO₂R¹⁰) at about 0 to 40°C in an organic solvent (chloroform, dichloromethane, diethylether, tetrahydrofuran, or the like).
(3) The method using a condensing agent is carried out, for example, by reacting carboxylic acid with a sulfonamide corresponding to R¹⁰ (NH₂SO₂R¹⁰) at about 0 to 40°C in an organic solvent (chloroform, dichloromethane, dimethyl formamide, diethylether, tetrahydrofuran, or the like) or in the absence of a solvent, in the presence or absence of a base (pyridine, triethylamine, dimethylaniline, dimethylaminopyridine, or the like), by using a condensing agent (1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridiniumiodine, 1-propylphosphonic acid cyclic anhydride (1-propanephosphonic acid cyclic anhydride, PPA), or the like), by using or not using 1-hydroxybenztriazole (HOBt).

Even when R¹, R¹⁰, R⁴, and R⁵ as substituents in the compounds represented by general formula (IA), general formula (IIA), general formula (IB), and general formula (IIB) and the compounds represented by reaction step formula 1, reaction step formula 2, reaction step formula 3, and reaction step formula 4 are R^{1Y}, R^{10Y}, R^{4Y}, and R^{5Y}, respectively, all the reactions described above can be performed in the same manner.

Among the compounds of the present invention, a compound having optical activity can be produced by using a starting material or reagent having optical activity, optically resolving a racemic production intermediate, then leading to the compound of the present invention, or optically resolving a racemic compound of the present invention.

The method of optical resolution is known, and examples thereof include a method of forming a salt, a complex or the like with another optically active compound, and performing recrystallization, then isolating a target compound or directly separating using a chiral column or the like.

The compounds represented by general formulae (III), (VI), (VII), (XIV), (XV) and (XVI) used as raw material compounds are known per se, or can be easily produced by using, for example, a method described in [Comprehensive Organic Transformations: A Guide to Functional Group Preparations, 2nd Edition (Richard C. Larock, John Wiley & Sons Inc, 1999)] and the like in combination.

In the reactions exemplified in the present specification, any heating method such as a water bath, an oil bath, a sand bath or microwave may be used at the time of heating.

In the reactions exemplified in the present specification, a solid phase supported reagent which is supported on a high molecular polymer (for example, polystyrene, polyacrylamide, polypropylene, polyethylene glycol, etc.) may be used.

Products of the reactions exemplified in the present specification can be purified by a method of conventional purification means, for example, distillation at normal or reduced pressure, various chromatography using silica gel, an ion exchange resin, a scavenger resin or magnesium silicate (for example, high performance liquid chromatography, thin layer chromatography, or column chromatography, etc.) or washing, re-crystallization or the like. Purification may be carried out after each reaction or after completing few reactions.

### [Toxicity]

The compound of the present invention has low toxicity and can be safely used as a pharmaceutical.

### [Application to pharmaceuticals]

Since the compound of the present invention has an agonist activity for LPA3, in mammals, especially humans, it can be formulated as a preventive and/or therapeutic agent for a disease associated with LPA3. As the disease associated with LPA3, a disease in which platelets increase can be exemplified. Examples of the disease in which platelets increase include essential thrombocythemia, reactive thrombocytosis, and the like.

As the disease associated with LPA3, a disease in which red blood cells decrease can also be exemplified. Examples of the disease in which red blood cells decrease include aplastic anemia, myelodysplastic syndrome, and the like.

As the disease associated with LPA3, idiopathic interstitial pneumonia can also be exemplified. Examples of the idiopathic interstitial pneumonia include idiopathic pulmonary fibrosis, and the like.

As the disease associated with LPA3, sepsis can also be exemplified.

In the pharmaceutical application of the compound of the present invention, the compound of the present invention may be used not only as a single agent but also as a combined medicine in combination with other active ingredients, for example, drugs and the like as listed below, as a combination drug, for example, for (1) complementation and/or enhancement of preventive, therapeutic and/or symptom ameliorating effect thereof, (2) improvement of kinetics and absorption thereof, reduction of dose, and/or (3) reduction of side effects thereof.

The combined medicine of the compound of the present invention with the other agent(s) may be administered in the form of a compounding agent in which both ingredients are compounded in a preparation or may be administered in the form of separate preparations by the same route of administration or different routes of administration. When separate preparations are administered, simultaneous administration is not necessarily required, and a time difference may be provided in administration as necessary. In addition, in the case of providing a time difference in administration, the order of administration is not particularly limited, and may be appropriately adjusted so as to obtain desired drug efficacy.

The dose of the other agent(s) which is used in combination with the compound of the present invention can be appropriately increased or decreased based on the clinically used dose of the agent(s) or an agent similar thereto. In addition, the compounded ratio of the compound of the present invention and other agent(s) can be appropriately adjusted by considering the age and body weight of the subject of administration, the method for administration, the duration of administration, the target disease, the symptom and the like. Approximately, 1 part by weight of the compound of the present invention may be combined with 0.01 to 100 parts by weight of other agent(s). A plurality of other agents may be used. In addition, other agent(s) may be agent(s) having the same mechanism as those listed above. Such an agent includes not only those found so far but also those found in the future.

The dose of the compound of the present invention varies depending on the age, the body weight, the symptom, the therapeutic effect, the method for administration, the duration of the treatment and the like. However, normally, the dose per adult is in the range of from 0.1 mg to 300 mg per administration, from one to several oral administrations per day or the dose per adult is in the range of from 0.1 mg to 150 mg per administration, from one to several parenteral administrations per day. Alternatively, the dose is continuously administrated intravenously for a period of time in the range of 1 to 24 hours per day.

As described above, since the dose varies depending on various conditions, a dose smaller than the above dose may be sufficient, or administration beyond the range may be necessary.

In order to use the compound of the present invention as a single agent or a combined medicine in combination of the compound of the present invention with other agent(s) as a concomitant drug, for the purpose of prevention and/or treatment of the above diseases, the substance as an active ingredient is usually formulated with various additives or a pharmaceutically acceptable carrier such as a solvent and then administered systemically or topically in oral or parenteral form. Here, the pharmaceutically acceptable carrier means a substance other than the active ingredient, which is generally used in the preparation of pharmaceuticals. The pharmaceutically acceptable carrier preferably has no pharmacological action at a dose of the preparation, is harmless, and does not interfere with the therapeutic effect of the active ingredient. In addition, the pharmaceutically acceptable carrier can also be used for the purpose of enhancing usefulness of the active ingredient and the preparation, facilitating formulation, stabilizing quality, improving usability, and the like. Specifically, substances such as those described in "Encyclopedia of Pharmaceutical Additives" published by Yakuji Nippo, Limited in 2000 (edited by International Pharmaceutical Excipients Council Japan) or the like may be appropriately selected according to the purpose.

### [Preparation]

When the compound of the present invention or the like or a combined medicine of the compound of the present invention and other agent(s) is administered, it is used as a solid preparation or liquid preparation for oral administration, a sustained release preparation or controlled release preparation for oral administration, or an injection, infusion solution, external preparation, inhalant, suppository or the like for parenteral administration.

Examples of the solid preparation for oral administration include tablets, pills, capsules, powders, granules, and the like, and examples of the capsules include hard capsules, soft capsules, and the like.

The solid preparation may be obtained by, for example, formulating the compound of the present invention together with a pharmaceutically acceptable carrier. Here, examples of the pharmaceutically acceptable carrier used for formulating the solid preparation include excipients (for example, lactose, mannitol, glucose, microcrystalline cellulose, starch, and the like), binders (for example, hydroxypropyl cellulose, polyvinylpyrrolidone, magnesium aluminate metasilicate, and the like), disintegrants (for example, calcium fiber glycolate and the like), lubricants (for example, magnesium stearate, and the like), stabilizers, solubilizing agents (for example, glutamic acid, aspartic acid, and the like), and the like. If necessary, the film may be coated with a coating agent (for example, white sugar, gelatin, hydroxypropyl cellulose, hydroxypropyl methylcellulose phthalate, or the like), or may be coated with two or more layers. Furthermore, it may be contained in a capsule containing gelatin.

The liquid preparation for oral administration may be in any form of a solution, a suspension, an emulsion, a syrup, an elixir and the like, and for example, the compound of the present invention may be dissolved, suspended or emulsified in a diluent (for example, purified water, ethanol, or a mixture thereof, or the like) for formulation. Furthermore, the liquid preparation may contain a wetting agent, a suspending agent, an emulsifying agent, a sweetening agent, a flavoring agent, a fragrance, a preservative, a buffering agent, or the like.

The sustained release preparation for oral administration may contain, for example, a gel-forming substance, and examples of the gel-forming substance include gum arabic, agar, polyvinylpyrrolidone, sodium alginate, propylene glycol alginate, carboxyvinyl polymer, carboxymethyl cellulose, sodium carboxymethyl cellulose, guar gum, gelatin, hydroxypropyl methyl cellulose, hydroxypropyl cellulose, polyvinyl alcohol, methyl cellulose, hydroxyethyl methyl cellulose, and the like.

The injection or infusion solution for parenteral administration may be in the form of either an aqueous solution, a suspension or an emulsion, and may be formulated as a solid preparation together with a pharmaceutically acceptable carrier so as to be dissolved, suspended or emulsified for use by adding a solvent (for example, distilled water for injection, physiological saline, glucose solution, isotonic solution (for example, a solution of sodium chloride, potassium chloride, glycerin, mannitol, sorbitol, boric acid, borax, propylene glycol, or the like), or the like) at the point of use. Here, examples of the "pharmaceutically acceptable carrier" include stabilizers (for example, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene, and the like), solubilizing agents (for example, alcohols (for example, ethanol and the like), polyalcohols (for example, propylene glycol, polyethylene glycol, and the like), and nonionic surfactants (for example, polysorbate 20 (registered trademark), polysorbate 80 (registered trademark), HCO-50, and the like), and the like), suspending agents (for example, glyceryl monostearate, aluminum monostearate, methyl cellulose, carboxymethyl cellulose, hydroxymethyl cellulose, sodium lauryl sulfate, and the like), emulsifiers (for example, gum arabic, sodium alginate, tragacanth, and the like), soothing agents (for example, benzyl alcohol, chlorobutanol, sorbitol, and the like), buffering agents (for example, phosphate buffer, acetate buffer, borate buffer, carbonate buffer, citrate buffer, tris buffer, glutamate buffer, epsilon aminocaproate buffer, and the like), preservatives (for example, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edetate, boric acid, borax, and the like), antiseptics (for example, benzalkonium chloride, paraoxybenzoic acid, chlorobutanol, and the like), pH adjusting agents (for example, hydrochloric acid, sodium hydroxide, phosphoric acid, acetic acid, and the like), antioxidants, and the like. As the antioxidant, for example, (1) water-soluble antioxidants such as ascorbic acid, cysteine hydrochloride, sodium bisulfate, sodium metabisulfite, and sodium sulfite, (2) oil-soluble antioxidants such as ascorbyl palmitate, butylated hydroxyanisole, butylated hydroxytoluene, lecithin, propyl gallate, and α-tocopherol, (3) metal chelating agents such as citric acid, ethylenediaminetetraacetic acid, sorbitol, tartaric acid, and phosphoric acid, and the like can be used.

The injection or infusion solution can be produced by sterilization in the final process or by a sterile operation method, for example, filtration with a filter or the like to be sterilized, and then filling into a sterile container. In addition, the injection or infusion solution can also be used by dissolving sterile powder (which may contain a powder of a pharmaceutically acceptable carrier.) obtained by vacuum drying and freeze drying in an appropriate solvent at the point of use.

Examples of the dosage form of the external preparation for parenteral administration include propellant, inhalant, spray, aerosol, ointment, gel, cream, fomentation, patch, liniment, nasal drop, and the like.

The propellant, inhalant, and spray may contain, in addition to a diluent used commonly, a stabilizer such as sodium bisulfite and a buffer capable of imparting isotonicity, for example, an isotonic agent such as sodium chloride, sodium citrate or citric acid. The method for producing a spray is described in detail in US 2,868,691 and US 3,095,355.

Examples of the inhalant include an inhalation solution or an inhalation powder, and the solution may be such a configuration that it is used after dissolving or suspending in water or other suitable medium at the point of use. These inhalants are produced in accordance with a known method. For example, an inhalation liquid is prepared by appropriately mixing an antiseptic (for example, benzalkonium chloride, paraben, or the like), a colorant, a buffering agent (for example, sodium phosphate, sodium acetate, or the like), an isotonizing agent (for example, sodium chloride, concentrated glycerin, or the like), a thickener (for example, a carboxyvinyl polymer or the like), an absorption accelerator, and the like as necessary. On the other hand, an inhalation powder is prepared by appropriately mixing a lubricant (for example, stearic acid, a salt thereof, or the like), a binder (for example, starch, dextrin, or the like), an excipient (for example, lactose, cellulose, or the like), a colorant, an antiseptic (for example, benzalkonium chloride, paraben, or the like), an absorption accelerator, and the like as necessary. In case of administering the inhalation solution, a spraying apparatus (for example, an atomizer, a nebulizer, or the like) is commonly used, and on the other hand, in case of administering the inhalation powder, an inhalation administration device for powder medicine is commonly used.

The ointment is prepared by a known or commonly used formulation, and is prepared, for example, by mixing or melting the compound of the present invention in an ointment base. Here, the ointment base is selected from known or commonly used ones, and for example, one or more selected from higher fatty acids or higher fatty acid esters (for example, adipic acid, myristic acid, palmitic acid, stearic acid, oleic acid, adipic acid ester, myristic acid ester, palmitic acid ester, stearic acid ester, oleic acid ester, and the like), waxes (for example, beeswax, spermaceti wax, ceresin, and the like.), surfactants (for example, polyoxyethylene alkyl ether phosphate esters and the like), higher alcohols (for example, cetanol, stearyl alcohol, cetostearyl alcohol, and the like), silicone oils (for example, dimethylpolysiloxane, and the like), hydrocarbons (for example, hydrophilic petrolatum, white petrolatum, purified lanolin, liquid paraffin and the like), glycols (for example, ethylene glycol, diethylene glycol, propylene glycol, polyethylene glycol, macrogol, and the like), vegetable oils (for example, castor oil, olive oil, sesame oil, turpentine oil, and the like), animal oils (for example, mink oil, egg yolk oil, squalane, squalene, and the like), water, absorption accelerators and anti-rash agents are mixed and used. Furthermore, the ointment base may contain a humectant, a preservative, a stabilizer, an antioxidant, a flavoring agent, or the like.

The gel is prepared by a known or commonly used formulation, and is prepared, for example, by melting the compound of the present invention into a gel base. Here, the gel base is selected from known or commonly used ones, and for example, one or more selected from lower alcohols (for example, ethanol, isopropyl alcohol, and the like), gelling agents (for example, carboxymethyl cellulose, hydroxyethyl cellulose, hydroxypropyl cellulose, ethyl cellulose, and the like), neutralizing agents (for example, triethanolamine, diisopropanolamine, and the like), surfactants (for example, polyethylene glycol monostearate, and the like), gums, water, absorption accelerators and anti-rash agents are mixed and used. Furthermore, the gel base may contain a preservative, an antioxidant, a flavoring agent, or the like.

The cream is prepared according to a known or commonly used formulation, and is produced, for example, by melting or emulsifying the compound of the present invention in a cream base. Here, the cream base is selected from known or commonly used ones, and for example, one or more selected from higher fatty acid esters, lower alcohols, hydrocarbons, polyhydric alcohols (for example, propylene glycol, 1,3-butylene glycol, and the like), higher alcohols (for example, 2-hexyl decanol, cetanol, and the like), emulsifiers (for example, polyoxyethylene alkyl ethers, fatty acid esters, and the like), water, absorption accelerators and anti-rash agents are mixed and used. Furthermore, the cream base may contain a preservative, an antioxidant, a flavoring agent, or the like.

The fomentation is prepared according to a known or commonly used formulation, and is prepared, for example, by melting the compound of the present invention in a fomentation base to obtain a kneaded mixture and spreading and applying it onto a support. Here, the fomentation base is selected from known or commonly used ones, and for example, one or more selected from thickeners (for example, polyacrylic acid, polyvinylpyrrolidone, gum arabic, starch, gelatin, methyl cellulose, and the like), wetting agents (for example, urea, glycerin, propylene glycol, and the like), fillers (for example, kaolin, zinc oxide, talc, calcium, magnesium, and the like), water, solubilizing agents, tackifiers, and anti-rash agents are mixed and used. Furthermore, the fomentation base may contain a preservative, an antioxidant, a flavoring agent, or the like.

The patch is prepared by a known or commonly used formulation, and is prepared, for example, by melting the compound of the present invention in a patch base and spreading and applying it onto a support. Here, the patch base is selected from known or commonly used ones, and for example, one or more selected from polymer bases, fats and oils, higher fatty acids, tackifiers, and anti-rash agents are mixed and used. Furthermore, the patch base may contain a preservative, an antioxidant, a flavoring agent, or the like.

The liniment is prepared by a known or commonly used formulation, and is prepared, for example, by dissolving, suspending or emulsifying the compound of the present invention in one or more kinds selected from water, alcohols (for example, ethanol, polyethylene glycol, and the like), higher fatty acids, glycerin, soap, emulsifiers, suspending agents, and the like. Furthermore, the liniment may contain a preservative, an antioxidant, a flavoring agent, or the like.

Unless otherwise defined, all technical and scientific terms used herein have the same meaning as commonly understood by one of ordinary skill in the art to which this invention belongs.

In addition, contents of all patent literatures and non patent literatures or references explicitly cited herein may be entirely incorporated herein as a part of the present specification.

The present invention will be described in more detail by the following examples, but the scope of the present invention is not limited thereto. Various changes or modifications can be made by those skilled in the art based on the description of the present invention, and these changes or modifications are also included in the present invention.

### EXAMPLES

The present invention will be described in detail with reference to examples and biological examples hereinbelow, but the present invention is not limited thereto. The compound names of the present invention and the compound names shown in the examples were named by ACD/Name (version 6.00, manufactured by Advanced Chemistry Development Inc.) or Chemdraw Ultra (version 12.0, manufactured by Cambridge Soft).

LCMS was performed using a Waters i-class (A) or Shimadzu Nexera X2 (B) system under the following conditions.
Column: YMC Triart C18 2.0 mm × 30 mm, 1.9 µm; flow rate: 1.0 mL/min; temperature: 30°C; mobile phase A: 0.1% trifluoroacetic acid (hereinafter, abbreviated as TFA) aqueous solution; mobile phase B: 0.1% TFA acetonitrile solution: gradient (ratios of mobile phase (A) : mobile phase (B) are described):
0 to 0.10 min: (95% : 5%); 0.10 to 1.20 min: (95% : 5%) to (5%: 95%); 1.20 to 1.50 min: (5% : 95%).

The numerical values shown in NMR portions are measured values (chemical shift values) of ¹H-NMR when the described measurement solvent is used.

### Reference Example 1: Ethyl 3-formyl-1H-indole-2-carboxylate

A solution of phosphorus oxychloride (2.7 mL) in dimethylformamide (hereinafter, abbreviated as DMF) (8 mL) was stirred under ice bath for 30 minutes, then a solution of ethyl indole-2-carboxylate (5.0 g) in DMF (8.5 mL) was added thereto, and the reaction mixture was stirred at room temperature for 30 minutes and at 60°C for 4 hours. After cooling the reaction mixture to room temperature, water (30 mL) was poured thereto, and subsequently, the mixture was neutralized by adding 2 N sodium hydroxide. The obtained solid was filtered, washed with DMF/water (2/1), and then dried to obtain the title compound (5.5 g) having the following physical property values.
TLC: Rf 0.23 (hexane : ethyl acetate = 4 : 1);
¹H-NMR (CDCl₃): δ 1.48, 4.53, 7.31-7.50, 8.45-8.53, 9.31, 10.76.

### Reference Example 2: Ethyl 3-[(cyclobutylamino)methyl]-1H-indole-2-carboxylate

To a solution of the compound (2.0 g) produced in Reference Example 1 in 1,2-dichloroethane (hereinafter, abbreviated as DCE) (15 mL), cyclobutylamine (1.3 g) and acetic acid (2 mL) were added, and the mixture was stirred at 45°C overnight. The reaction mixture was cooled to room temperature, then sodium triacetoxyborohydride (3.9 g) was added thereto, and the mixture was stirred at room temperature for 4 hours. Ethyl acetate and a saturated aqueous sodium bicarbonate solution were added to the reaction mixture, and then the obtained solid was filtered and dried to obtain the title compound (1.3 g) having the following physical property values.
LCMS (B) Retention time (min): 0.88;
MS (ESI, Pos.): 273 (M+H)⁺.
¹H-NMR (DMSO-d₆): δ 1.37, 1.46-1.69, 1.96-2.07, 3.15-3.25, 4.05, 4.36, 7.06, 7.25, 7.42, 7.78, 11.57.

### Example 1: 3-[(Cyclobutylamino)methyl]-1-(cyclopropylmethyl)-1H-indole-2-carboxylic acid

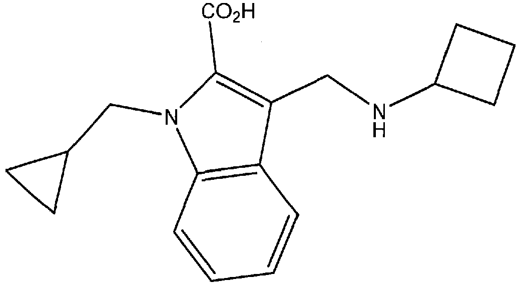

To a solution of the compound (20 mg) produced in Reference Example 2 in DMF (1 mL), sodium hydride (60%, 2.9 mg) was added under ice bath, and the mixture was stirred at room temperature for 1 hour. 1-(Bromomethyl)cyclopropane (9.9 mg) was added to the reaction mixture, and the mixture was stirred at 60°C overnight. The reaction mixture was cooled to room temperature, then ethyl acetate and water were added thereto, and the mixture was extracted. The organic layer was dried over sodium sulfate and then concentrated under reduced pressure, methanol (0.5 mL), tetrahydrofuran (hereinafter, abbreviated as THF) (0.5 mL) and 5 N sodium hydroxide (0.2 mL) were added to the resulting residue, and the mixture was stirred at 60°C for 3 hours. The reaction mixture was cooled to room temperature, then 1 N hydrochloric acid (1 mL) and ethyl acetate were added thereto, and the mixture was extracted. The organic layer was dried over sodium sulfate and then concentrated under reduced pressure, and the resulting residue was purified by HPLC (column: YMC Triart C18 30 mm × 75 mm, 5 µm; flow rate: 40 mL/min; mobile phase A: 0.1% aqueous TFA solution; mobile phase B: 0.1% TFA acetonitrile solution: gradient (ratios of mobile phase (A): mobile phase (B) are described): 0 to 0.50 min: (90% : 10%); 0.50 to 9.00 min: (90% : 10%) to (20%: 80%); 9.01 to 11.00 min: (0%: 100%)) to obtain the title compound (8.5 mg) having the following physical property values.
LCMS (A) Retention time (min): 0.76;
MS (ESI, Pos.): 299 (M+H)⁺.
¹H-NMR (DMSO-d₆): δ 1.19-1.28, 1.72-1.84, 2.12-2.25, 3.69-3.79, 4.41, 4.54, 7.22, 7.37, 7.67, 7.84, 8.95.

### Example 1(1) to Example 1(4)

The same procedure as in Reference Example 2 to Example 1 was carried out using a corresponding amino compound in place of cyclobutylamine and a corresponding bromo compound or iodo compound in place of 1-(bromomethyl)cyclopropane to obtain the following compounds.

### Example 1(1): 3-[(Cyclopentylamino)methyl]-1-ethyl-1H-indole-2-carboxylic acid

LCMS (A) Retention time (min): 0.69;
MS (ESI, Pos.): 287 (M+H)⁺.
¹H-NMR (DMSO-d₆): δ 1.22, 1.51-1.57, 1.64-1.74, 1.87-1.94, 3.37-3.44, 4.23, 4.67, 7.09, 7.21, 7.48, 7.71, 10.88.

### Example 1(2): 1-Butyl-3-[(cyclobutylamino)methyl]-1H-indole-2-carboxylic acid

LCMS (A) Retention time (min): 0.81;
MS (ESI, Pos.): 301 (M+H)⁺.
¹H-NMR (DMSO-d₆): δ 1.23-1.30, 1.62-1.69, 1.72-1.84, 2.12-2.24, 3.69-3.79, 4.40, 4.61, 7.22, 7.38, 7.64, 7.83, 8.94.

### Example 1(3): 3-[(Cyclobutylamino)methyl]-1-[(2,2-difluorocyclopropyl)methyl]-1H-indole-2-carboxylic acid

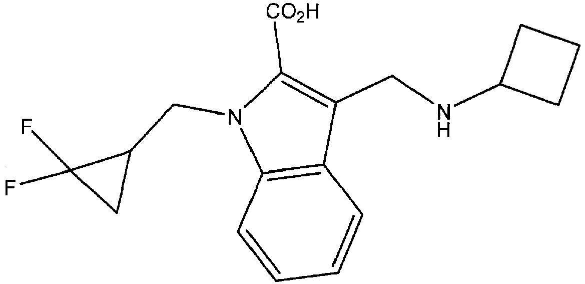

LCMS (A) Retention time (min): 0.76;
MS (ESI, Pos.): 335 (M+H)⁺.
¹H-NMR (DMSO-d₆): δ 1.44-1.52, 1.55-1.63, 1.72-1.84, 2.12-2.25, 3.69-3.78, 4.40, 4.70, 4.89, 7.24, 7.40, 7.66, 7.85, 9.13.

### Example 1(4): 3-[(Cyclobutylamino)methyl]-1-ethyl-1H-indole-2-carboxylic acid

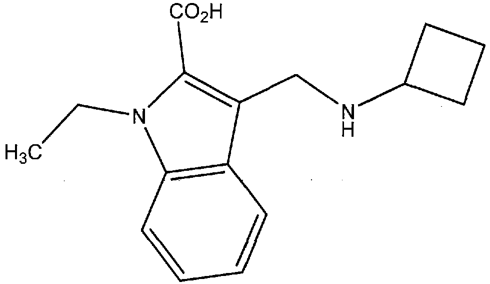

LCMS (A) Retention time (min): 0.70;
MS (ESI, Pos.): 273 (M+H)⁺.
¹H-NMR (DMSO-d₆): δ1.21, 1.65-1.84, 2.01-2.20, 3.50-3.64, 4.10, 4.66, 7.07, 7.20, 7.47, 7.67, 11.08.

### Reference Example 3: Ethyl 3-formyl-1-(4-methylbenzyl)-1H-indole-2-carboxylate

Potassium carbonate (1.9 g) and 1-(bromomethyl)-4-methylbenzene (940 mg) were added to a solution of the compound (1.0 g) produced in Reference Example 1 in DMF (10 mL), and the reaction mixture was stirred at room temperature for 1 hour. Water was added to the reaction mixture, and the mixture was extracted with ethyl acetate. The organic layer was dried over sodium sulfate, and then concentrated under reduced pressure. The resulting residue was purified by silica gel column chromatography (hexane : ethyl acetate = 19 : 1 → 9 : 1 → 4 : 1) to obtain the title compound (1.41 g) having the following physical property values.
TLC: Rf 0.38 (hexane : ethyl acetate = 4 : 1);
¹H-NMR (CDCl₃): δ 1.39, 2.29, 4.45, 5.78, 6.96, 7.08, 7.32-7.43, 8.50-8.57, 10.64.

### Reference Example 4: Ethyl 3-[(cyclobutylamino)methyl]-1-(4-methylbenzyl)-1H-indole-2-carboxylate

To a solution of the compound (350 mg) produced in Reference Example 3 in DCE (5 mL), cyclobutylamine (155 mg) and acetic acid (0.5 mL) were added, and the mixture was stirred at 45°C for 5 hours. The reaction mixture was cooled to room temperature, then sodium triacetoxyborohydride (462 mg) was added thereto, and the mixture was stirred at room temperature overnight. Water and a saturated aqueous sodium bicarbonate solution were added to the reaction mixture, and the mixture was extracted with ethyl acetate. The organic layer was dried over sodium sulfate, and then concentrated under reduced pressure. The resulting residue was purified by silica gel column chromatography (hexane : ethyl acetate = 1 : 1 → 0 : 1) to obtain the title compound (330 mg) having the following physical property values.
TLC: Rf 0.59 (chloroform : methanol = 9 : 1);
¹H-NMR (CDCl₃): δ 1.36, 1.52-1.80, 2.07-2.20, 3.30-3.44, 4.14, 4.36, 5.73, 6.89, 7.03, 7.13-7.22, 7.25-7.37, 7.78.

### Example 2: 3-[(Cyclobutylamino)methyl]-1-(4-methylbenzyl)-1H-indole-2-carboxylic acid

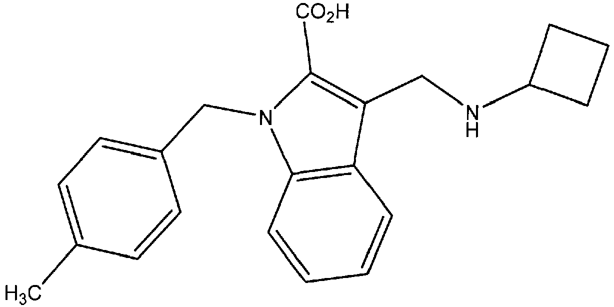

A 2 N aqueous sodium hydroxide solution (4.5 mL) was added to a solution of the compound (330 mg) produced in Reference Example 4 in methanol (9 mL) and 1,2-dimethoxyethane (4.5 mL), and the mixture was stirred at 50°C for 3 hours. The reaction mixture was cooled to room temperature, then neutralized with 2 N hydrochloric acid (4.5 mL), and water was added thereto, then the obtained solid was filtered and dried to obtain the title compound (250 mg) having the following physical property values.
LCMS (A) Retention time (min): 0.79;
MS (ESI, Pos.): 349 (M+H)⁺.
¹H-NMR (DMSO-d₆): δ 1.70-1.87, 2.13-2.25, 3.60-3.75, 4.32, 5.91, 6.99, 7.05, 7.16, 7.26, 7.51, 7.81, 10.00.

### Example 2(1) to Example 2(82)

The same procedure as in Reference Example 1 to Reference Example 3 to Reference Example 4 to Example 2 was carried out by using a corresponding indole compound or pyrrole compound in place of ethyl indole-2-carboxylate, a corresponding bromo compound or chloro compound in place of 1-(bromomethyl)-4-methylbenzene, and a corresponding amino compound in place of cyclobutylamine to obtain the following compounds.

### Example 2(1): 1-(2-Chlorobenzyl)-3-[(cyclopentylamino)methyl]-4-fluoro-1H-indole-2-carboxylic acid

LCMS (A) Retention time (min): 0.86;
MS (ESI, Pos.): 401 (M+H)⁺.
¹H-NMR (DMSO-d₆): δ 1.49-1.79, 1.85-2.03, 3.41-3.57, 4.40, 6.06, 6.19, 6.83-6.94, 7.02-7.27, 7.46, 10.75.

### Example 2(2): 1-(2-Chlorobenzyl)-3-[(cyclopentylamino)methyl]-5-methyl-1H-indole-2-carboxylic acid

LCMS (A) Retention time (min): 0.88;
MS (ESI, Pos.): 397 (M+H)⁺.
¹H-NMR (DMSO-d₆): δ 1.46-1.79, 1.83-2.00, 2.37, 3.38-3.51, 4.28, 6.02, 6.12, 6.95-7.13, 7.15-7.23, 7.45, 7.56, 10.83.

### Example 2(3): 1-(2-Chlorobenzyl)-3-((cyclopentylamino)methyl]-7-fluoro-1H-indole-2-carboxylic acid

LCMS (A) Retention time (min): 0.86;
MS (ESI, Pos.): 401 (M+H)⁺.
¹H-NMR (DMSO-d₆): δ 1.52-1.61, 1.67-1.77, 1.89-2.00, 3.43-3.51, 4.33, 6.11, 6.20, 6.94, 7.04-7.11, 7.21, 7.47, 7.65, 10.68.

### Example 2(4): 1-(2-Chlorobenzyl)-3-[(cyclopentylamino)methyl]-4-methyl-1H-indole-2-carboxylic acid

LCMS (A) Retention time (min): 0.87;
MS (ESI, Pos.): 397 (M+H)⁺.
¹H-NMR (DMSO-d₆): δ 1.53-1.61, 1.66-1.76, 1.93-2.10, 2.70, 3.50-3.57, 4.45, 6.02, 6.19, 6.85, 7.00-7.08, 7.21, 7.47, 10.64.

### Example 2(5): 1-(2-Chlorobenzyl)-3-(cyclopentylamino)methyl]-7-methyl-1H-indole-2-carboxylic acid

LCMS (A) Retention time (min): 0.88;
MS (ESI, Pos.): 397 (M+H)⁺.
¹H-NMR (DMSO-d₆): δ 1.51-1.60, 1.66-1.76, 1.88-1.97, 2.34, 3.40-3.48, 4.30, 5.96, 6.88, 7.00, 7.06, 7.22, 7.48, 7.65, 10.71.

### Example 2(6): 1-(2-Chlorobenzyl)-3-[(cyclobutylamino)methyl]-1H-indole-2-carboxylic acid

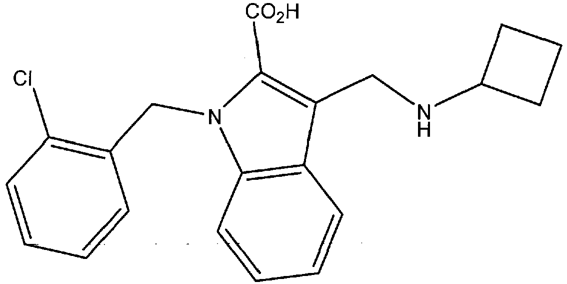

LCMS (A) Retention time (min): 0.81;
MS (ESI, Pos.): 369 (M+H)⁺.
¹H-NMR (DMSO-d₆): δ 1.70-1.88, 2.10-2.24, 3.60-3.70, 4.23, 6.08, 6.18, 7.07, 7.10-7.26, 7.48, 7.78, 11.10.

### Example 2(7): 3-[(Cyclopentylamino)methyl]-1-(2-methylbenzyl)-1H-indole-2-carboxylic acid

LCMS (A) Retention time (min): 0.81;
MS (ESI, Pos.): 363 (M+H)⁺.
¹H-NMR (DMSO-d₆): δ 1.50-1.60, 1.66-1.75, 1.88-1.97, 2.39, 3.39-3.48, 4.32, 5.98, 6.86, 7.04, 7.08-7.18, 7.24, 7.78, 10.90.

### Example 2(8): 3-[(Cyclopentylamino)methyl]-1-(4-methylbenzyl)-1H-indole-2-carboxylic acid

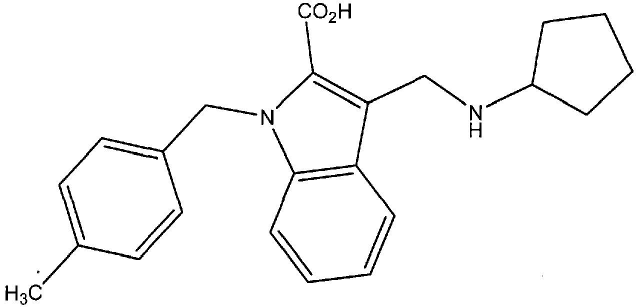

LCMS (A) Retention time (min): 0.82;
MS (ESI, Pos.): 397 (M+H)⁺.
¹H-NMR (DMSO-d₆): δ 1.50-1.59, 1.66-1.74, 1.87-1.94, 2.19, 3.39-3.45, 4.27, 5.95, 7.00-7.04, 7.08, 7.15, 7.41, 7.72, 10.84.

### Example 2(9): 1-(3-Chlorobenzyl)-3-[(cyclopentylamino)methyl]-1H-indole-2-carboxylic acid

LCMS (A) Retention time (min): 0.82;
MS (ESI, Pos.): 383 (M+H)⁺.
¹H-NMR (DMSO-d₆): δ 1.50-1.59, 1.65-1.75, 1.86-1.95, 3.38-3.46, 4.30, 6.01, 7.08-7.15, 7.18, 7.21-7.30, 7.43, 7.76, 10.81.

### Example 2(10): 1-(4-Chlorobenzyl)-3-[(cyclopentylamino)methyl]-1H-indole-2-carboxylic acid

LCMS (A) Retention time (min): 0.83;
MS (ESI, Pos.): 383 (M+H)⁺.
¹H-NMR (DMSO-d₆): δ 1.50-1.59, 1.65-1.74, 1.87-1.96, 3.38-3.46, 4.28, 5.98, 7.10, 7.12-7.19, 7.29, 7.41, 7.74, 10.81.

### Example 2(11): 3-[(Cyclopentylamino)methyl]-1-(2-methoxybenzyl)-1H-indole-2-carboxylic acid

LCMS (A) Retention time (min): 0.80;
MS (ESI, Pos.): 379 (M+H)⁺.
¹H-NMR (DMSO-d₆): δ 1.51-1.59, 1.67-1.74, 1.88-1.96, 3.40-3.47, 3.88, 4.30, 5.95, 6.23, 6.65, 6.99, 7.09, 7.11-7.16, 7.22, 7.76, 10.87.

### Example 2(12): 3-[(Cyclopentylamino)methyl]-1-(3-methoxybenzyl)-1H-indole-2-carboxylic acid

LCMS (A) Retention time (min): 0.79;
MS (ESI, Pos.): 379 (M+H)⁺.
¹H-NMR (DMSO-d₆): δ 1.49-1.59, 1.64-1.75, 1.86-1.96, 3.38-3.47, 3.65, 4.29, 5.97, 6.63-6.69, 6.73, 7.06-7.19, 7.41, 7.74, 10.78.

### Example 2(13): 1-Benzyl-3-[(cyclopentylamino)methyl]-1H-indole-2-carboxylic acid

LCMS (A) Retention time (min): 0.78;
MS (ESI, Pos.): 349 (M+H)⁺.
¹H-NMR (DMSO-d₆): δ 1.49-1.60, 1.64-1.75, 1.87-1.97, 3.38-3.48, 4.29, 6.00, 7.07-7.12, 7.13-7.18, 7.19-7.24, 7.42, 7.74, 10.75.

### Example 2(14): 3-[(Cyclopentylamino)methyl]-1-(3-methylbenzyl)-1H-indole-2-carboxylic acid

LCMS (A) Retention time (min): 0.81;
MS (ESI, Neg.): 361 (M-H)⁻.
¹H-NMR (CDCl₃): δ 1.30-1.52, 1.56-1.96, 2.22, 3.16-3.32, 4.45, 5.94, 6.81-7.00, 7.03-7.35, 7.60, 11.00.

### Example 2(15): 4-Chloro-1-(2-chlorobenzyl)-3-[(cyclopentylamino)methyl]-1H-indole-2-carboxylic acid

LCMS (A) Retention time (min): 0.90;
MS (ESI, Pos.): 417 (M+H)⁺.
¹H-NMR (DMSO-d₆): δ 1.51-1.61, 1.63-1.75, 1.92-2.02, 3.45-3.54, 4.71, 6.06, 6.17, 7.08, 7.12-7.18, 7.22, 7.26, 7.48, 10.58.

### Example 2(16): 3-[(Cyclobutylamino)methyl]-1-(3,4-dimethylbenzyl)-1H-indole-2-carboxylic acid

LCMS (A) Retention time (min): 0.83;
MS (ESI, Pos.): 363 (M+H)⁺.
¹H-NMR (DMSO-d₆): δ 1.67-1.86, 2.07-2.19, 3.53-3.67, 4.18, 5.93, 6.81, 6.93, 6.97, 7.08, 7.15, 7.40, 7.70, 11.12.

### Example 2(17): 1-(4-Methylbenzyl)-3-[(3-oxetanylamino)methyl)-1H-indole-2-carboxylic acid

LCMS (A) Retention time (min): 0.77;
MS (ESI, Pos.): 351 (M+H)⁺.
¹H-NMR (DMSO-d₆): δ 2.20, 4.23, 4.25-4.31, 4.62-4.70, 5.94, 6.98-7.04, 7.09, 7.18, 7.44, 7.71, 11.70.

### Example 2(18): 3-[(Cyclopropylamino)methyl]-1-(4-methylbenzyl)-1H-indole-2-carboxylic acid

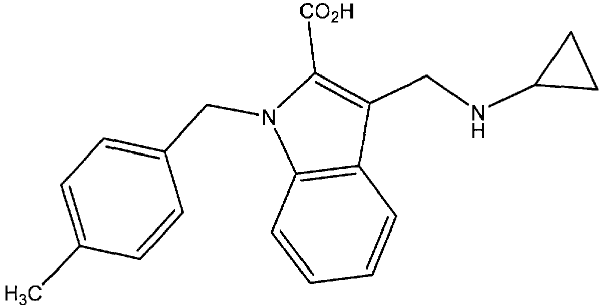

LCMS (A) Retention time (min): 0.82;
MS (ESI, Pos.): 335 (M+H)⁺.
¹H-NMR (DMSO-d₆): δ 0.73-0.84, 2.20, 2.70-2.77, 4.62, 5.87, 6.95, 7.04, 7.20, 7.32, 7.56, 7.88, 9.20.

### Example 2(19): 1-(4-Methylbenzyl)-3-{[(3-methylcyclobutyl)amino]methyl}-1H-indole-2-carboxylic acid

LCMS (A) Retention time (min): 0.88;
MS (ESI, Pos.): 363 (M+H)⁺.
¹H-NMR (DMSO-d₆): δ 1.71-1.93, 2.06-2.14, 2.21, 2.30-2.45, 3.51-3.89, 4.39-4.45, 5.87, 6.93-6.98, 7.02-7.07, 7.19-7.24, 7.33, 7.58, 7.82-7.87, 8.90-9.22.

### Example 2(20): 3-[(Cyclobutylamino)methyl]-1-(2-fluoro-4-methylbenzyl)-1H-indole-2-carboxylic acid

LCMS (A) Retention time (min): 0.86;
MS (ESI, Pos.): 367 (M+H)⁺.
¹H-NMR (DMSO-d₆): δ 1.72-1.84, 2.11-2.25, 3.63-3.72, 4.30, 5.96, 6.48, 6.79, 7.01, 7.17, 7.26, 7.44, 7.80, 10.10.

### Example 2(21): 3-[(Cyclobutylamino)methyl]-1-[4-methyl-3-(trifluoromethyl)benzyl]-1H-indole-2-carboxylic acid

LCMS (A) Retention time (min): 0.91;
MS (ESI, Pos.): 417 (M+H)⁺.
¹H-NMR (DMSO-d₆): δ 1.70-1.84, 2.10-2.22, 2.36, 3.66-3.76, 4.40, 5.95, 7.16, 7.22, 7.29-7.36, 7.48, 7.60, 7.85, 9.41.

### Example 2(22): 3-[(Cyclobutylamino)methyl]-1-(2,4-dimethylbenzyl)-1H-indole-2-carboxylic acid

LCMS (A) Retention time (min): 0.88;
MS (ESI, Pos.): 363 (M+H)⁺.
¹H-NMR (DMSO-d₆): δ 1.73-1.85, 2.14-2.26, 2.37, 3.67-3.82, 4.36-4.48, 5.81-5.97, 6.68-6.71, 6.96-7.08, 7.14-7.26, 7.29-7.34, 7.39-7.43, 7.81-7.92, 9.12.

### Example 2(23): 1-(3-Chloro-4-methylbenzyl)-3-[cyclobutylamino)methyl]-1H-indole-2-carboxylic acid

LCMS (A) Retention time (min): 0.89;
MS (ESI, Pos.): 383 (M+H)⁺.
¹H-NMR (DMSO-d₆): δ 1.72-1.84, 2.12-2.25, 2.37-2.48, 3.69-3.80, 4.37-4.48, 5.86-5.99, 6.80-6.83, 6.91-7.00, 7.08-7.12, 7.19-7.41, 7.44-7.59, 7.83-7.92, 9.30.

### Example 2(24): 3-[(Cyclobutylamino)methyl]-1-(2-methylbenzyl)-1H-indole-2-carboxylic acid

LCMS (A) Retention time (min): 0.84;
MS (ESI, Pos.): 349 (M+H)⁺.
¹H-NMR (DMSO-d₆): δ 1.75-1.85, 2.15-2.26, 2.42, 3.74-3.82, 4.47, 5.88, 5.96, 6.90, 7.08, 7.21, 7.25, 7.33, 7.45, 7.91, 9.06.

### Example 2(25): 3-[(Cyclobutylamino)methyl]-1-(3-methylbenzyl)-1H-indole-2-carboxylic acid

LCMS (A) Retention time (min): 0.84;
MS (ESI, Pos.): 349 (M+H)⁺.
¹H-NMR (DMSO-d₆): δ 1.72-1.84, 2.12-2.23, 3.70-3.76, 4.41, 5.89, 6.79, 6.92, 7.01, 7.12, 7.21, 7.32, 7.55, 7.85, 9.27.

### Example 2(26): 1-Benzyl-3-[(cyclobutylamino-1H-indole-2-carboxylic acid

LCMS (A) Retention time (min): 0.80;
MS (ESI, Pos.): 335 (M+H)⁺.
¹H-NMR (DMSO-d₆): δ 1.72-1.84, 2.12-2.24, 3.69-3.78, 4.40, 5.94, 7.06, 7.17-7.27, 7.32, 7.57, 7.85, 9.29.

### Example 2(27): 3-1(Cyclobutylamino)methyl]-1-(2-fluorobenzyl)-1H-indole-2-carboxylic acid

LCMS (A) Retention time (min): 0.81;
MS (ESI, Pos.): 353 (M+H)⁺.
¹H-NMR (DMSO-d₆): δ 1.73-1.85, 2.12-2.25, 3.69-3.79, 4.41, 5.98, 6.52, 6.99, 7.18-7.29, 7.34, 7.54, 7.87, 9.35.

### Example 2(28): 3-[(Cyclobutylamino)methyl]-1-(3-fluorobenzyl)-1H-indole-2-carboxylic acid

LCMS (A) Retention time (min): 0.81;
MS (ESI, Pos.): 353 (M+H)⁺.
¹H-NMR (DMSO-d₆): δ 1.73-1.85, 2.12-2.25, 3.70-3.80, 4.42, 5.95, 6.82, 6.92, 7.04, 7.22, 7.28-7.36, 7.57, 7.86, 9.27.

### Example 2(29): 3-[(Cyclobutylamino)methyl]-1-(4-fluorobenzyl)-1H-indole-2-carboxylic acid

LCMS (A) Retention time (min): 0.81;
MS (ESI, Pos.): 353 (M+H)⁺.
¹H-NMR (DMSO-d₆): δ 1.72-1.85, 2.12-2.24, 3.69-3.78, 4.40, 5.91, 7.07-7.15, 7.21, 7.33, 7.59, 7.85, 9.30.

### Example 2(30): 4-Bromo-3-[(cyclopentylamino)methyl]-1-(3-methylbenzyl)-1H-indole-2-carboxylic acid

LCMS (A) Retention time (min): 0.91;
MS (ESI, Pos.): 441 (M+H)⁺.
¹H-NMR (DMSO-d₆): δ 1.47-1.78, 1.85-2.05, 2.18, 3.33-3.50, 4.72, 5.94, 6.83, 6.92-7.14,7.28, 7.46, 10.59.

### Example 2(31): 3-[(Cyclopentylamino)methyl]-4,5-dimethyl-1-(4-methylbenzyl)-1H-pyrrole-2-carboxylic acid

LCMS (A) Retention time (min): 0.84;
MS (ESI, Pos.): 341 (M+H)⁺.
¹H-NMR (DMSO-d₆): δ 1.45-1.74, 1.76-1.98,2.22, 3.33-3.44, 3.78, 5.69, 6.83, 7.03, 11.26.

### Example 2(32): 3-[(Cyclobutylamino)methyl]-4,5-dimethyl-1-(4-methylbenzyl)-1H-pyrrole-2-carboxylic acid

LCMS (A) Retention time (min): 0.81;
MS (ESI, Pos.): 327 (M+H)⁺.
¹H-NMR (DMSO-d₆): δ 1.68-1.81, 1.88, 1.94, 1.99-2.16, 2.23, 3.44-3.51, 3.69, 5.68, 6.83, 7.05, 11.38.

### Example 2(33): 4-Bromo-3-[(cyclobutylamino)methyl]-5-methyl-1-(4-methylbenzyl)-1H-pyrrole-2-carboxylic acid

LCMS (A) Retention time (min): 0.85;
MS (ESI, Pos.): 391 (M+H)⁺.
¹H-NMR (DMSO-d₆): δ 1.65-1.84, 1.92-2.28, 3.40-3.57, 3.76, 5.78, 6.86, 7.06, 11.25.

### Example 2(34): 5-Bromo-3-[(cyclobutylamino)methyl]-4-methyl-1-(4-methylbenzyl)-1H-pyrrole-2-carboxylic acid

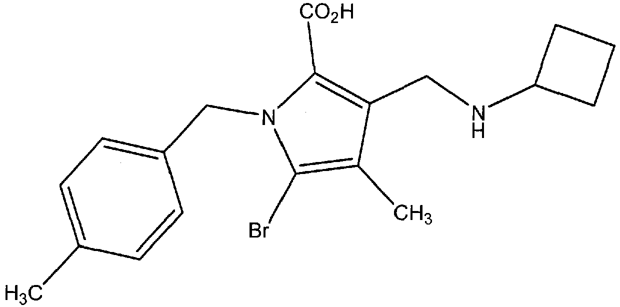

LCMS (A) Retention time (min): 0.81;
MS (ESI, Neg.): 389 (M-H)⁻.
¹H-NMR (CD₃OD): δ 1.80-1.97, 2.05, 2.12-2.37, 3.64-3.79, 3.96, 5.79, 6.93, 7.03.

### Example 2(35): 3-[(Cyclobutylamino)methyl]-4-fluoro-1-(4-methylbenzyl)-1H-indole-2-carboxylic acid

LCMS (A) Retention time (min): 0.83;
MS (ESI, Pos.): 367 (M+H)⁺.
¹H-NMR (DMSO-d₆): δ 1.70-1.83, 2.07-2.19, 2.20, 3.60-3.67, 4.25, 5.94, 6.83, 7.00-7.05, 7.08-7.13, 7.27, 10.87.

### Example 2(36): 3-[(Cyclobutylamino)methyl]-1-(2,6-difluorobenzyl)-1H-indole-2-carboxylic acid

LCMS (B) Retention time (min): 0.95;
MS (ESI, Pos.): 371 (M+H)⁺.
¹H-NMR (CDCl₃): δ 1.43-1.88, 2.02-2.14, 2.38-2.54, 3.44-3.58, 4.46, 6.32, 6.75-6.87, 7.09-7.28, 7.40, 7.57, 11.33.

### Example 2(37): 3-[(Cyclobutylamino)methyl]-1-[2-(trifluoromethoxy)benzyl]-1H-indole-2-carboxylic acid

LCMS (B) Retention time (min): 1.02;
MS (ESI, Pos.): 419 (M+H)⁺.
¹H-NMR (CDCl₃): δ 1.43-1.82, 2.01-2.13, 2.23-2.40, 3.40-3.53, 4.37, 6.01, 6.58, 6.95-7.03, 7.12-7.32, 7.54-7.62, 11.14.

### Example 2(38): 3-[(Cyclobutylamino)methyl]-1-[4-(difluoromethyl)benzyl]-1H-indole-2-carboxylic acid

LCMS (B) Retention time (min): 0.97;
MS (ESI, Pos.): 385 (M+H)⁺.
¹H-NMR (CDCl₃): δ 1.45-2.14, 2.15-2.32, 3.37-3.53, 4.35, 5.96, 6.50, 7.09-7.62, 10.70.

### Example 2(39): 3-[(Cyclobulylamino)methyl]-1-[3-(trifluoromethoxy)benzyl]-1H-indole-2-carboxylic acid

LCMS (B) Retention time (min): 1.02;
MS (ESI, Pos.): 419 (M+H)⁺.
¹H-NMR (CDCl₃): δ 1.45-1.78, 1.98-2.11, 2.24-2.38, 3.40-3.52, 4.39, 5.99, 6.94-7.09, 7.13-7.32, 7.58, 11.21.

### Example 2(40): 3-[(Cyclobutylamino)methyl]-1-[4-(trifluoromethoxy)benzyl]-1H-indole-2-carboxylic acid

LCMS (B) Retention time (min): 1.02;
MS (ESI, Pos.): 419 (M+H)⁺.
¹H-NMR (DMSO-d₆): δ 1.37-1.83, 2.00-2.14, 2.23-2.39, 3.41-3.54, 4.39, 5.93, 7.00-7.08, 7.11-7.28, 7.57, 11.12.

### Example 2(41): 3-[(Cyclobutylamino)methyl]-1-(3-cyclopropylbenzyl)-1H-indole-2-carboxylic acid

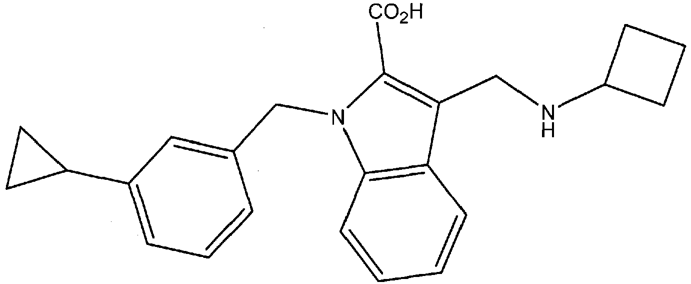

LCMS (B) Retention time (min): 1.05;
MS (ESI, Pos.): 375 (M+H)⁺.
¹H-NMR (CDCl₃): δ 0.52-0.61, 0.79-0.91, 1.45-1.90, 1.95-2.12, 2.17-2.34, 3.33-3.47, 4.40, 5.97, 6.76-6.92, 6.89, 7.06, 7.16, 7.24, 7.32, 7.54, 10.82.

### Example 2(42): 3-[(Cyclobutylamino)methyl]-5-fluoro-1-(4-methylbenzyl)-1H-indole-2-carboxylic acid

LCMS (B) Retention time (min): 1.00;
MS (ESI, Pos.): 367 (M+H)⁺.
¹H-NMR (CDCl₃): δ 1.50-1.82, 1.98-2.11, 2.23, 2.27-2.40, 3.38-3.50, 4.24, 5.93, 6.93, 6.97-7.04, 7.14, 7.20, 11.12.

### Example 2(43): 5-Bromo-3-[(cyclobutylamino)methyl]-1-(4-methylbenzyl)-1H-indole-2-carboxylic acid

LCMS (B) Retention time (min): 1.05;
MS (ESI, Pos.): 427 (M+H)⁺.
¹H-NMR (DMSO-d₆): δ 1.70-1.86, 2.10-2.20, 3.56-3.68, 4.18, 5.95, 6.98-7.07, 7.26, 7.41, 7.98, 10.87.

### Example 2(44): 3-[(Bicyclo[1.1.1]pent-1-ylamino)methyl]-1-(4-methylbenzyl)-1H-indole-2-carboxylic acid

LCMS (B) Retention time (min): 0.99;
MS (ESI, Pos.): 361 (M+H)⁺.
¹H-NMR (DMSO-d₆): δ 1.86, 2.21, 2.53-2.58, 4.23, 5.95, 6.98, 7.03, 7.11, 7.20, 7.46, 7.77, 12.05.

### Example 2(45): 1-(4-Methylbenzyl)-3-{[(1-methylcyclobutyl)amino]methyl}-1H-indole-2-carboxylic acid

LCMS (B) Retention time (min): 0.98;
MS (ESI, Pos.): 363 (M+H)⁺.
¹H-NMR (DMSO-d₆): δ 1.52, 1.82-1.96, 2.21, 2.33-2.45, 4.17, 5.97, 7.00-7.07, 7.09, 7.18, 7.44, 7.77, 11.14.

### Example 2(46): 1-(4-Methylbenzyl)-3-{[(2-methylcyclobutyl)amino]methyl}-1H-indole-2-carboxylic acid

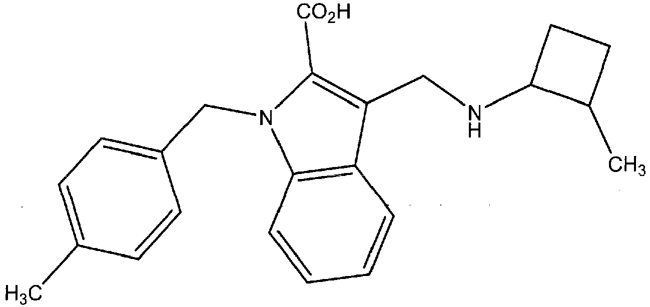

LCMS (B) Retention time (min): 1.00;
MS (ESI, Pos.): 363 (M+H)⁺.
¹H-NMR (DMSO-d₆): δ 1.07, 1.18, 1.28-1.40, 1.43-1.55, 1.90-2.35, 2.42-2.72, 3.10-3.22, 3.61-3.72, 4.19, 5.88-6.05, 7.01-7.05, 7.06-7.12, 7.13-7.20, 7.39-7.46, 7.69-7.74, 11.20.

### Example 2(47): 3-{[(3,3-Dimethylcyclobutyl)amino]methyl}-1-(4-methylbenzyl)-1H-indole-2-carboxylic acid

LCMS (B) Retention time (min): 1.03;
MS (ESI, Pos.): 377 (M+H)⁺.
¹H-NMR (DMSO-d₆): δ 1.11, 1.91-2.06, 2.20, 3.57-3.71, 4.16, 5.96, 7.02, 7.08, 7.16, 7.42, 7.69, 11.03.

### Example 2(48): 1-(4-Methylbenzyl)-3-[(spiro[3.3]hept-2-ylamino)methyl]-1H-indole-2-carboxylic acid

LCMS (B) Retention time (min): 1.04;
MS (ESI, Pos.): 389 (M+H)⁺.
¹H-NMR (DMSO-d₆): δ 1.72-1.82, 1.88-2.01, 2.04-2.14, 2.18-2.28, 3.41-3.53, 4.15, 5.95, 7.02, 7.08, 7.16, 7.42, 7.68, 11.04.

### Example 2(49): 3-[(Cyclobutylamino)methyl]-1-(4-methylbenzyl)-1H-pyrrolo[2,3-b]pyridine-2-carboxylic acid

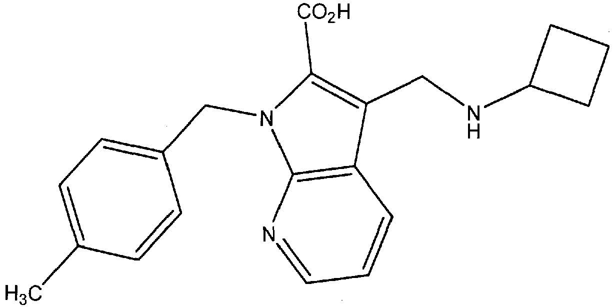

LCMS (B) Retention time (min): 0.89;
MS (ESI, Pos.): 350 (M+H)⁺.
¹H-NMR (DMSO-d₆): δ 1.69-1.85, 2.05-2.19, 2.20, 3.56-3.67, 4.18, 6.00, 7.00, 7.06, 7.18, 8.17, 8.35, 10.96.

### Example 2(50): 3-[(Cyclobutylamino)methyl]-1-(4-methylbenzyl)-1H-pyrrolo[2,3-c]pyridine-2-carboxylic acid

LCMS (B) Retention time (min): 0.70;
MS (ESI, Pos.): 350 (M+H)⁺.
¹H-NMR (DMSO-d₆): δ 1.68-1.85, 2.08-2.19, 2.22, 3.57-3.67, 4.19, 6.03, 7.06, 7.11, 7.68, 8.16, 8.83, 10.64.

### Example 2(51): 3-[(Cyclobutylamino)methyl]-1-(4-cyclopropylbenzyl)-1H-indole-2-carboxylic acid

LCMS (B) Retention time (min): 1.00;
MS (ESI, Pos.): 375 (M+H)⁺.
¹H-NMR (CDCl₃): δ 0.52-0.60, 0.82-0.90, 1.45-1.82, 1.97-2.10, 2.25-2.40, 3.39-3.50, 4.35, 5.95, 6.89, 7.02, 7.13, 7.22, 7.32, 7.55, 11.15.

### Example 2(52): 3-[(Cyclobutylamino)methyl]-1-[3-(difluoromethoxy)benzyl]-1H-indole-2-carboxylic acid

LCMS (B) Retention time (min): 0.98;
MS (ESI, Pos.): 401 (M+H)⁺.
¹H-NMR (CDCl₃): δ 1.54-1.85, 2.00-2.12, 2.24-2.37, 3.41-3.53, 4.38, 5.97, 6.39, 6.84-7.00, 7.14-7.32, 7.58, 11.03.

### Example 2(53): 3-[(Cyclobutylamino)methy]-1-[2-(difluoromethoxy)benzyl]-1H-indole-2-carboxylic acid

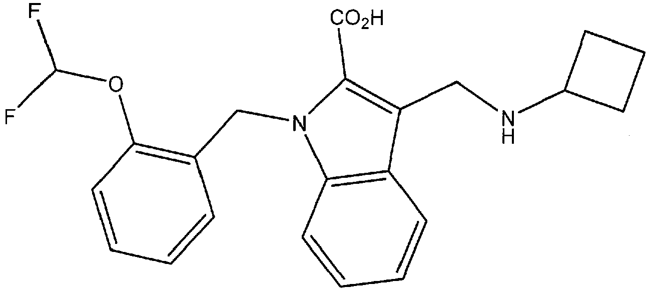

LCMS (B) Retention time (min): 0.98;
MS (ESI, Pos.): 401 (M+H)⁺.
¹H-NMR (CDCl₃): δ 1.42-1.82, 2.02-2.13, 2.25-2.39, 3.42-3.52, 4.35, 5.95, 6.57, 6.71, 6.92, 7.10-7.28, 7.58, 11.12.

### Example 2(54): 3-[(Cyclobutylamino)methyl]-1-[3-(difluoromethyl)benzyl]-1H-indole-2-carboxylic acid

LCMS (B) Retention time (min): 0.96;
MS (ESI, Pos.): 385 (M+H)⁺.
¹H-NMR (CDCl₃): δ 1.45-1.78, 1.97-2.09, 2.23-2.37, 3.40-3.52, 4.38, 6.02, 6.52, 7.17, 7.20-7.33, 7.58, 11.18.

### Example 2(55): 3-[(Cyclobutylamino)methyl]-1-(2-cyclopropylbenzyl)-1H-indole-2-carboxylic acid

LCMS (B) Retention time (min): 1.00;
MS (ESI, Pos.): 375 (M+H)⁺.
¹H-NMR (CDCl₃): δ 0.76-0.86, 0.99-1.09, 1.48-1.85, 1.96-2.13, 2.18-2.33, 3.38-3.51, 4.34, 5.30, 6.15-6.25, 6.87, 7.03-7.24, 7.57, 10.99.

### Example 2(56): 3-[(Cyclobutylamino)methyl]-1-[2-(difluoromethyl)benzyl]-1H-indole-2-carboxylic acid

LCMS (B) Retention time (min): 0.97;
MS (ESI, Pos.): 385 (M+H)⁺.
¹H-NMR (CDCl₃): δ 1.47-1.82, 1.96-2.08, 2.17-2.32, 3.37-3.48, 4.31, 6.02, 6.49, 7.07, 7.11-7.30, 7.52-7.62, 11.06.

### Example 2(57): 3-[(Cyclobutylamino)methyl]-1-[4-(difluoromethoxy)benzyl]-1H-indole-2-carboxylic acid

LCMS (B) Retention time (min): 1.02;
MS (ESI, Pos.): 401 (M+H)⁺.
¹H-NMR (CDCl₃): δ 1.42-1.82, 2.04-2.14, 2.25-2.39, 3.42-3.53, 4.37, 5.92, 6.38, 6.94, 7.13, 7.14-7.20, 7.22-7.32, 7.57, 11.12.

### Example 2(58): 6-Bromo-3-[(cyclobutylamino)methyl]-1-(4-methylbenzyl)-1H-indole-2-carboxylic acid

LCMS (B) Retention time (min): 1.03;
MS (ESI, Pos.): 427 (M+H)⁺.
¹H-NMR (DMSO-d₆): δ 1.70-1.85, 2.09-2.19, 2.22, 3.55-3.68, 4.18, 5.96, 7.01, 7.05, 7.22, 7.66, 7.69, 10.95.

### Example 2(59): 7-Bromo-3-[(cyclobutylamino)methyl]-1-(4-methylbenzyl)-1H-indole-2-carboxylic acid

LCMS (B) Retention time (min): 1.03;
MS (ESI, Pos.): 427 (M+H)⁺.
¹H-NMR (DMSO-d₆): δ 1.70-1.84, 2.09-2.19, 2.20, 3.55-3.67, 4.18, 6.44, 6.72, 7.00, 7.04, 7.38, 7.79, 10.88.

### Example 2(60): 6-[(Cyclobutylamino)methyl]-4-(4-methylbenzyl)-4H-thieno[3,2-b]pyrrole-5-carboxylic acid

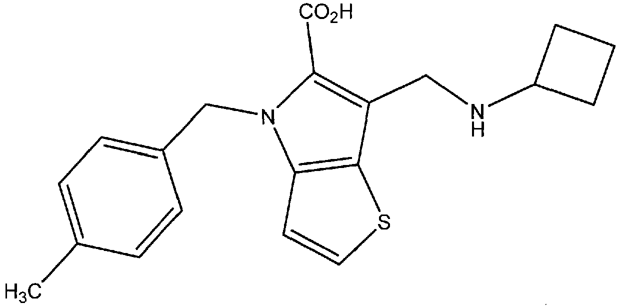

LCMS (B) Retention time (min): 0.95;
MS (ESI, Pos.): 355 (M+H)⁺.
¹H-NMR (DMSO-d₆): δ 1.68-1.84, 2.02-2.20, 2.22, 3.51-3.62, 3.95, 5.85, 7.05, 7.11, 7.12, 7.34, 11.38.

### Example 2(61): 5-Chloro-3 [(cyclobutylamino)methyl]-1-(4-methylbenzyl)-1H-indole-2-carboxylic acid

LCMS (B) Retention time (min): 1.04;
MS (ESI, Pos.): 383 (M+H)⁺.
¹H-NMR (CDCl₃): δ 1.50-1.82, 2.00-2.11, 2.23, 2.25-2.39, 3.38-3.49, 4.23, 5.91, 6.97, 7.01, 7.12, 7.18, 7.47, 11.10.

### Example 2(62): 3-[(Cyclobutylamino)methyl]-1-[4-(methylsulfonyl)benzyl]-1H-indole-2-carboxylic acid

LCMS (B) Retention time (min): 0.87;
MS (ESI, Pos.): 413 (M+H)⁺.
¹H-NMR (DMSO-d₆): δ 1.71-1.86, 2.10-2.21, 3.15, 3.58-3.68, 4.21, 6.13, 7.12, 7.19, 7.31,7.42, 7.74, 7.80, 11.06.

### Example 2(63): 6-Chloro-3-[(cyclobutylamino)methyl]-1-(4-methylbenzyl)-1H-indole-2-carboxylic acid

LCMS (B) Retention time (min): 1.02;
MS (ESI, Pos.): 383 (M+H)⁺.
¹H-NMR (DMSO-d₆): δ 1.69-1.86, 2.09-2.19, 2.22, 3.55-3.67, 4.18, 5.96, 7.02, 7.05, 7.10, 7.53, 7.74, 10.96.

### Example 2(64): 3-[(Cyclobutylamino)methyl]-1-[2-(methylsulfonyl)benzyl]-1H-indole-2-carboxylic acid

LCMS (B) Retention time (min): 0.91;
MS (ESI, Pos.): 413 (M+H)⁺.
¹H-NMR (CDCl₃): δ 1.45-1.80, 2.02-2.12, 2.15-2.28, 3.40, 3.40-3.52, 4.31, 6.26, 6.33, 7.19-7.39, 7.57, 8.07, 10.97.

### Example 2(65): 3-[(Cyclobutylamino)methyl]-6-fluoro-1-(4-methylbenzyl)-1H-indole-2-carboxylic acid

LCMS (B) Retention time (min): 0.98;
MS (ESI, Pos.): 367 (M+H)⁺.
¹H-NMR (CDCl₃): δ 1.48-1.82, 2.00-2.11, 2.23, 2.25-2.40, 3.40-3.50, 4.28, 5.88, 6.88, 6.95, 7.01, 7.45, 11.15.

### Example 2(66): 7-Chloro-3-[(cyclobutylamino)methyl]-1-(4-methylbenzyl)-1H-indole-2-carboxylic acid

LCMS (B) Retention time (min): 1.02;
MS (ESI, Pos.): 383 (M+H)⁺.
¹H-NMR (CDCl₃): δ 1.45-1.80, 1.90-2.03, 2.17-2.31, 3.31-3.42, 4.26, 6.45, 6.87, 6.97, 7.04, 7.20, 7.44, 11.08.

### Example 2(67): 3-[(Cyclobutylamino)methyl]-7-fluoro-1-(4-methylbenzyl)-1H-indole-2-carboxylic acid

LCMS (B) Retention time (min): 0.98;
MS (ESI, Pos.): 367 (M+H)⁺.
¹H-NMR (CDCl₃): δ 1.47-1.80, 1.95-2.07, 2.22, 2.23-2.38, 3.35-3.46, 4.31, 6.13, 6.90, 6.96-7.08, 7.29, 11.14.

### Example 2(68): 4-Chloro-3-[(cyclobutylamino)methyl]-1-(4-methylbenzyl)-1H-indole-2-carboxylic acid

LCMS (B) Retention time (min): 1.02;
MS (ESI, Pos.): 383 (M+H)⁺.
¹H-NMR (CDCl₃): δ 1.55-1.83, 2.06-2.18, 2.23, 2.24-2.37, 3.54-3.65, 4.84, 5.87, 6.96-7.02, 7.03-7.10, 7.15-7.19, 10.84.

### Example 2(69): 3-[(Cyclobutylamino)methyl]-1-(4-methylbenzyl)-1,4,5,6-tetrahydrocyclopenta[b]pyrrole-2-carboxylic acid

LCMS (B) Retention time (min): 1.04;
MS (ESI, Pos.): 339 (M+H)⁺.
¹H-NMR (CDCl₃): δ 1.50-1.71, 1.73-1.89, 1.91-2.04, 2.18, 2.21-2.44, 2.46-2.71, 3.32-3.43, 3.94, 5.33, 6.87, 6.98, 7.16-7.22, 9.33.

### Example 2(70): 1-(2-Chlorobenzyl)-3-[(cyclopentylamino)methyl]-1H-indole-2-carboxylic acid

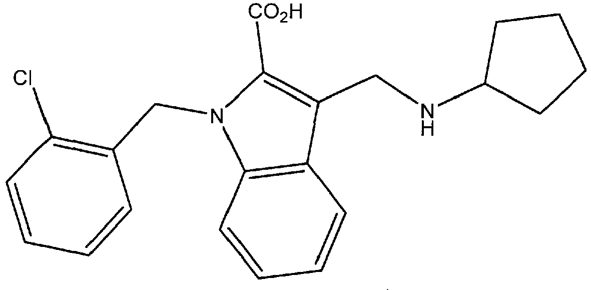

LCMS (A) Retention time (min): 0.84;
MS (ESI, Pos.): 383 (M+H)⁺.
¹H-NMR (DMSO-d₆): δ 1.48-1.80, 1.86-2.02, 3.40-3.55, 4.37, 6.06, 6.16, 7.06, 7.10-7.28, 7.47, 7.82, 10.56.

### Example 2(71): 1-(4-Methylbenzyl)-3-(3-thiethanylamino)methyl]-1H-indole-2-carboxylic acid

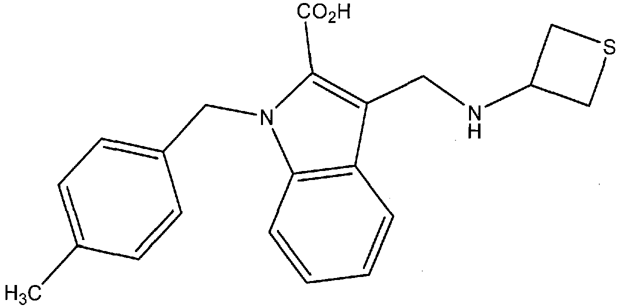

LCMS (B) Retention time (min): 0.96;
MS (ESI, Pos.): 367 (M+H)⁺.
¹H-NMR (DMSO-d₆): δ 2.21, 3.10-3.17, 3.47-3.55, 4.17, 4.35-4.45, 5.94, 6.99-7.05, 7.09, 7.18, 7.44, 7.71, 11.42.

### Example 2(72): 1-(2-Chlorobenzyl)-3-[(cyclopropylamino)methyl]-1H-indole-2-carboxylic acid

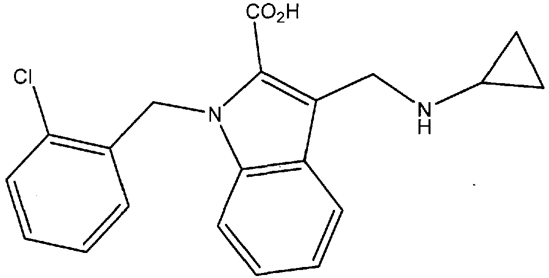

LCMS (B) Retention time (min): 0.97;
MS (ESI, Pos.): 355 (M+H)⁺.
¹H-NMR (DMSO-d₆): δ 0.71-0.83, 2.62-2.69, 4.42, 6.05, 6.13, 7.05, 7.14, 7.17-7.23, 7.26, 7.47, 7.82, 11.05.

### Example 2(73): 3-[(Cyclobutylamino)methyl]-1-[3-(1-pyrrolidinyl)benzyl]-1H-indole-2-carboxylic acid

LCMS (B) Retention time (min): 0.98;
MS (ESI, Pos.): 404 (M+H)⁺.
¹H-NMR (DMSO-d₆): δ 1.67-1.84, 1.86-1.95, 2.10-2.20, 3.07-3.14, 3.53-3.64, 4.19, 5.94, 6.30, 6.33, 6.40, 6.97, 7.08, 7.16, 7.44, 7.70, 11.16.

### Example 2(74): 3-[(Cyclobutylamino)methyl]-1-(2-thienylmethyl)-1H-indole-2-carboxylic acid

LCMS (B) Retention time (min): 0.92;
MS (ESI, Pos.): 341 (M+H)⁺.
¹H-NMR (CDCl₃): δ 1.45-1.86, 2.01-2.13, 2.33-2.49, 3.41-3.56, 4.41, 6.15, 6.84, 7.01, 7.07, 7.17, 7.30, 7.48, 7.58, 11.25.

### Example 2(75): 3-[(Cyclobutylamino)methyl]-1-[2-(methylthio)benzyl]-1H-indole-2-carboxylic acid

LCMS (B) Retention time (min): 0.96;
MS (ESI, Pos.): 381 (M+H)⁺.
¹H-NMR (DMSO-d₆): δ 1.71-1.88, 2.10-2.34, 2.58, 3.60-3.70, 4.23, 6.00, 6.07, 6.89, 7.08-7.23, 7.33, 7.76, 11.10.

### Example 2(76): 3-[(Cyclobutylamino)methyl]-1-[(5-methyl-3-pyridinyl]methyl]-1H-indole-2-carboxylic acid

LCMS (B) Retention time (min): 0.73;
MS (ESI, Pos.): 350 (M+H)⁺.
¹H-NMR (CDCl₃): δ 1.60-1.85, 1.98-2.19, 2.21, 2.25-2.39, 3.45-3.58, 4.33, 5.93, 7.16, 7.22-7.33, 7.58, 8.18, 8.25, 11.05.

### Example 2(77): 3-[(Cyclobutylamino)methyl]-1-[(4-cyclopropyl-1,3-thiazol-2-yl)methyl]-1H-indole-2-carboxylic acid

LCMS (B) Retention time (min): 0.92;
MS (ESI, Pos.): 382 (M+H)⁺.
¹H-NMR (CDCl₃): δ 0.76-1.08, 1.58-1.88, 1.90-2.17, 2.26-2.43, 3.44-3.59, 4.40, 6.14, 6.51, 7.19, 7.31, 7.53, 7.57, 10.33.

### Example 2(78): 3-[(Cyclobutylammo)methyl]-1-{-{4-[(trifluoromethyl)thio]benzyl}-1H-indole-2-carboxylic acid

LCMS (B) Retention time (min): 1.09;
MS (ESI, Pos.): 435 (M+H)⁺.
¹H-NMR (DMSO-d₆): δ 1.68-1.87, 2.08-2.22, 3.57-3.69, 4.20, 6.09, 7.11, 7.19, 7.23, 7.42, 7.60, 7.74, 11.06.

### Example 2(79): 3-[(Cyclobutylamino)methyl]-1-[3-(3-phenylpropoxy)benzyl]-1H-indole-2-carboxylic acid

LCMS (A) Retention time (min): 1.00;
MS (ESI, Pos.): 469 (M+H)⁺.
¹H-NMR (DMSO-d₆): δ 1.68-1.84, 1.92-1.99, 2.11-2.19, 2.69, 3.57-3.65, 3.87, 4.20, 5.99, 6.68-6.71, 6.74, 7.08-7.22, 7.27, 7.43, 7.72, 11.08.

### Example 2(80): 3-[(Cyclobutylamino)methyl]-1-(3-nitrobenzyl)-1H-indole-2-carboxylic acid

LCMS (B) Retention time (min): 0.94;
MS (ESI, Pos.): 380 (M+H)⁺.
¹H-NMR (DMSO-d₆): δ 1.69-1.86, 2.10-2.22, 3.57-3.69, 4.23, 6.14, 7.13, 7.21, 7.48, 7.54-7.64, 7.76, 7.98, 8.07, 11.05.

### Example 2(81): 3-[(Cyclobutylamino)methyl]-1-[(2-isopropyl-1,3-thiazol-4-yl)methyl]-1H-indole-2-carboxylic acid

LCMS (B) Retention time (min): 0.93;
MS (ESI, Pos.): 384 (M+H)⁺.
¹H-NMR (CDCl₃): δ 1.34, 1.55-1.87, 1.90-2.16, 2.25-2.42, 3.25, 3.43-3.56, 4.37, 6.06, 6.56, 7.16, 7.27, 7.49, 7.57, 10.76.

### Example 2(82): 3-[(Cyclobutylamino)methyl]-1-{4-[(3-methyl-3-oxetanyl)methoxy]benzyl}-1H-indole-2-carboxylic acid

LCMS (A) Retention time (min): 0.81;
MS (ESI, Pos.): 435 (M+H)⁺.
¹H-NMR (DMSO-d₆): δ 1.36, 1.52-1.91, 1.98-2.14, 2.25-2.44, 3.39-3.55, 3.86, 4.33-4.44, 4.54, 5.92, 6.74, 7.10, 7.12-7.38, 7.56, 11.07.

### Reference Example 5: Ethyl 1-[4-(benzyloxy)benzyl]-3-[(cyclobutylamino)methyl]-1H-indole-2-carboxylate

To a solution of the compound (1.0 g) synthesized in Reference Example 2 in toluene (10 mL), 4-benzyloxybenzyl alcohol (1.6 g) and cyanomethylene tributyl phosphorane (1.8 g) were added, and the mixture was stirred at 60°C overnight. The reaction mixture was cooled to room temperature and then concentrated under reduced pressure, and the resulting residue was purified by silica gel column chromatography (hexane : ethyl acetate = 1 : 1 → 0 : 1) to obtain the title compound (1.1 g) having the following physical property values.
TLC: Rf 0.25 (ethyl acetate : methanol = 19 : 1);
LCMS (A) Retention time (min): 0.94;
MS (ESI, Pos.): 469 (M+H)⁺.
¹H-NMR (CDCl₃): δ 1.37, 1.55-1.81, 2.08-2.19, 3.31-3.42, 4.14, 4.38, 4.99, 5.70, 6.81-6.87, 6.92-6.99, 7.15-7.22, 7.27-7.42, 7.78.

### Reference Example 6: Ethyl 3-[(cyclobutylamino)methyl]-1-(4-hydroxybenzyl)-1H-indole-2-carboxylate

To a solution of the compound (1.1 g) produced in Reference Example 5 in methanol (50 mL), 10% palladium-activated carbon (50% wet, 200 mg) was added, and the mixture was stirred under a hydrogen atmosphere at room temperature for 4 hours. The reaction mixture was filtered through Celite (trade name), and then the filtrate was concentrated. Methanol (50 mL) and 10% palladium-activated carbon (50% wet, 200 mg) were added to the resulting residue, and the mixture was stirred under a hydrogen atmosphere at room temperature for 2 hours. The reaction mixture was filtered through Celite (trade name), and then the filtrate was concentrated, and the resulting residue was purified by silica gel column chromatography (hexane : ethyl acetate = 1 : 1 → 0 : 1) to obtain the title compound (487 mg) having the following physical property values.
TLC: Rf 0.42 (chloroform : methanol = 9 : 1);
¹H-NMR (CDCl₃): δ 1.31, 1.58-1.90, 2.16-2.28, 3.36-3.49, 4.07, 4.31, 5.59, 6.26-6.34, 6.54-6.62, 7.03-7.10, 7.18-7.34, 7.72.

### Example 3: 3-[(Cyclobutylamino)methyl]-1-(4-hydroxybenzyl)-1H-indole-2-carboxylic acid

Using the compound produced in Reference Example 6, the same procedure as in Example 2 was carried out to obtain the title compound having the following physical property values.
LCMS (A) Retention time (min): 0.71;
MS (ESI, Pos.): 351 (M+H)⁺.
¹H-NMR (DMSO-d₆): δ 1.71-1.84, 2.11-2.23, 3.66-3.75, 4.34, 5.80, 6.61, 6.95, 7.17, 7.29, 7.58, 7.80, 9.29, 9.55.

### Example 3(1) to Example 3(2)

The same procedure as in Reference Example 5 to Reference Example 6 to Example 3 was carried out by using a corresponding alcohol compound in place of 4-benzyloxybenzyl alcohol to obtain the following compounds.

### Example 3(1): 3-[(Cyclobutylamino)methyl]-1-(2-hydroxybenzyl)-1H-indole-2-carboxylic acid

LCMS (A) Retention time (min): 0.74;
MS (ESI, Pos.): 351 (M+H)⁺.
¹H-NMR (DMSO-d₆): δ 1.68-1.83, 2.07-2.20, 3.57-3.65, 4.18, 5.78, 6.64, 6.71, 6.95, 7.02, 7.07, 7.14, 7.38, 7.70, 10.32, 11.17.

### Example 3(2): 3-[(Cyclobutylamino)methyl]-1-(3-hydroxybenzyl)-1H-indole-2-carboxylic acid

LCMS (A) Retention time (min): 0.72;
MS (ESI, Pos.): 351 (M+H)⁺.
¹H-NMR (DMSO-d₆): δ 1.69-1.83, 2.09-2.20, 3.56-3.64, 4.16, 5.92, 6.48, 6.52-6.57, 7.00, 7.08, 7.16, 7.39, 7.69, 9.26, 11.08.

### Reference Example 7: Ethyl 1-[2-(benzyloxy)benzyl]-3-formyl-1H-indole-2-carboxylate

To a solution of the compound (1.5 g) synthesized in Reference Example 1 in THF (30 mL), 2-benzyloxybenzyl alcohol (3.0 g) and triphenylphosphine (3.6 g) were added. The reaction mixture was cooled in an ice bath, diethyl azodicarboxylate (2.2 M toluene solution, 6.3 mL) was added thereto, and the mixture was stirred at room temperature overnight. The reaction mixture was concentrated under reduced pressure, and the resulting residue was purified by silica gel column chromatography (hexane : ethyl acetate = 19 : 1 → 9 : 1 → 4 : 1) to obtain the title compound (1.9 g) having the following physical property values.
TLC: Rf 0.33 (hexane : ethyl acetate = 4 : 1);
LCMS (A) Retention time (min): 1.24;
MS (ESI, Pos.): 436 (M+Na)⁺.

### Reference Example 8: Ethyl 1-[2-(benzyloxy)benzyl]-3-[(cyclobutylamino)methyl]-1H-indole-2-carboxylate

Using the compound produced in Reference Example 7, the same procedure as in Reference Example 4 was carried out to obtain the title compound having the following physical property values.
TLC: Rf 0.31 (ethyl acetate : methanol = 9 : 1);
LCMS (A) Retention time (min): 0.96;
MS (ESI, Pos.): 398 (M-*c*-BuNH₂+H)⁺.
¹H-NMR (CDCl₃): δ 1.29, 1.54-1.80, 2.09-2.20, 3.32-3.44, 4.17, 4.32, 5.18, 5.82, 6.29-6.35, 6.66-6.75, 6.95, 7.11-7.53, 7.77-7.82.

### Reference Example 9: Ethyl 1-[2-(benzyloxy)benzyl]-3-{[cyclobutyl(trifluoroacetyl)amino]methyl}-1H-indole-2-carboxylate

To a solution of the compound (97 mg) synthesized in Reference Example 8 in a methylene chloride (hereinafter, abbreviated as DCM) (30 mL), trifluoroacetic anhydride (0.043 mL) and triethylamine (0.087 mL) were added, and the mixture was stirred at room temperature for 3 hours. A saturated aqueous sodium bicarbonate solution and ethyl acetate were added to the reaction mixture, and the mixture was extracted. The organic layer was dried over sodium sulfate, and then concentrated under reduced pressure, and the resulting residue was purified by silica gel column chromatography (hexane : ethyl acetate = 1 : 1 → 1 : 9) to obtain the title compound (71 mg) having the following physical property values.
TLC: Rf 0.23 (hexane : ethyl acetate = 9 : 1);
LCMS (A) Retention time (min): 1.38;
MS (ESI, Pos.): 587 (M+Na)⁺.
¹H-NMR (CDCl₃): δ 1.26, 1.43-1.70, 1.91-2.41, 4.21-4.65, 4.33, 5.18, 5.29-5.53, 5.81, 6.30, 6.73, 6.96, 7.05-7.30, 7.32-7.60.

### Reference Example 10: Ethyl 3-{[cyclobutyl(trifluoroacetyl)amino]methyl}-1-(2-hydroxybenzyl)-1H-indole-2-carboxylate

To a solution of the compound (71 mg) produced in Reference Example 9 in methanol (5 mL), 10% palladium-activated carbon (50% wet, 20 mg) was added, and the mixture was stirred under a hydrogen atmosphere at room temperature for 3 hours. The reaction mixture was filtered through Celite (trade name), and then the filtrate was concentrated. The resulting residue was purified by silica gel column chromatography (hexane : ethyl acetate = 9 : 1 → 7 : 3→ 1 : 1) to obtain the title compound (43 mg) having the following physical property values.
TLC: Rf 0.33 (hexane : ethyl acetate = 1 : 1);
LCMS (A) Retention time (min): 1.18;
MS (ESI, Pos.): 497 (M+Na)⁺.
¹H-NMR (CDCl₃): δ 1.38, 1.45-1.70, 1.89-2.34, 4.18-4.64, 4.44, 5.24-5.48, 5.70, 6.30, 6.69-6.85, 7.05-7.18, 7.24-7.58.

### Example 4: 3-[(Cyclobutylamino)methyl]-1-(2-ethoxybenzyl)-1H-indole-2-carboxylic acid

To a solution of the compound (20 mg) produced in Reference Example 10 in DMF (1 mL), potassium carbonate (18 mg) and iodoethane (9.9 mg) were added, and the mixture was stirred at room temperature overnight. Ethyl acetate and water were added to the reaction mixture, and the mixture was extracted. The organic layer was dried over sodium sulfate and then concentrated under reduced pressure, methanol (0.5 mL), THF (0.5 mL) and 5 N sodium hydroxide (0.4 mL) were added to the resulting residue, and the mixture was stirred at 60°C for 3 hours. The reaction mixture was cooled to room temperature, then 2 N hydrochloric acid (1 mL) and ethyl acetate were added thereto, and the mixture was extracted. The organic layer was dried over sodium sulfate and then concentrated under reduced pressure, and the resulting residue was purified by HPLC (column: YMC Triart C18 30 mm × 75 mm, 5 µm; flow rate: 40 mL/min; mobile phase A: 0.1% aqueous TFA solution; mobile phase B: 0.1% TFA acetonitrile solution: gradient (ratios of mobile phase (A) : mobile phase (B) are described): 0 to 0.50 min: (90% : 10%); 0.50 to 9.00 min: (90% : 10%) to (20% : 80%); 9.01 to 11.00 min: (0% : 100%)) to obtain the title compound (7.1 mg) having the following physical property values.
LCMS (A) Retention time (min): 0.87;
MS (ESI, Pos.): 379 (M+H)⁺.
¹H-NMR (CDCl₃): δ 1.39, 1.73-1.85, 2.14-2.25, 3.72-3.81, 4.13, 4.45, 5.86, 6.24, 6.68, 7.00, 7.16, 7.22, 7.31, 7.42, 7.88, 9.07.

### Example 4(1): 1-[2-(4-Carboxybutoxy)benzyl]-3-[(cyclobutylamino)methyl]-1H-indole-2-carboxylic acid

The same procedure as in Example 4 was carried out using methyl 5-bromovalerate in place of iodoethane to obtain the title compound having the following physical property.
LCMS (A) Retention time (min): 0.81;
MS (ESI, Pos.): 451 (M+H)⁺.
¹H-NMR (CDCl₃): δ 1.70-1.85, 2.13-2.25, 2.34, 2.53, 3.70-3.79, 4.08, 4.42, 5.87, 6.20, 6.67, 7.01, 7.15, 7.21, 7.29, 7.38, 7.86, 9.35, 12.16.

### Reference Example 11: Ethyl 1-(4-methylbenzyl)-1H-indole-2-carboxylate

To a solution of ethyl indole-2-carboxylate (3.8 g) in DMF (50 mL), sodium hydride (60%, 840 mg) was added, and the mixture was stirred at room temperature for 30 minutes. 1-(Bromomethyl)-4-methylbenzene (3.9 g) was added to the reaction mixture, and the mixture was stirred at room temperature for 2 hours. A saturated aqueous ammonium chloride solution and ethyl acetate were added to the reaction mixture, and the mixture was extracted, and sequentially washed with a saturated aqueous sodium bicarbonate solution and saturated saline solution. The organic layer was dried over sodium sulfate, and then concentrated under reduced pressure to obtain the title compound (5.8 g) having the following physical property values.
TLC: Rf 0.73 (hexane : ethyl acetate = 4 : 1);
¹H-NMR (CDCl₃): δ 1.36, 2.27, 4.33, 5.81, 6.95, 7.05, 7.15, 7.25-7.41, 7.70.

### Reference Example 12: Ethyl 3-acetyl-1-(4-methylbenzyl)-1H-indole-2-carboxylate

To a solution of the compound (800 mg) produced in Reference Example 11 in acetonitrile (20 mL), acetic acid (0.4 mL), polyphosphoric acid (100 mg) and trifluoroacetic anhydride (0.95 mL) were added, and the mixture was stirred at room temperature overnight. Tert-butyl methyl ether was added to the reaction mixture, and the mixture was neutralized by adding 1 N sodium hydroxide, and then extracted. The organic layer was washed with saturated saline solution, then dried over sodium sulfate, and concentrated under reduced pressure. The resulting residue was purified by silica gel column chromatography (hexane : ethyl acetate = 19 : 1 → 9 : 1 → 4 : 1) to obtain the title compound (620 mg) having the following physical property values.
TLC: Rf 0.36 (hexane : ethyl acetate = 4 : 1);
¹H-NMR (CDCl₃): δ 1.30, 2.29, 2.62, 4.38, 5.48, 7.00, 7.08, 7.24-7.37, 8.04-8.11.

### Reference Example 13: Ethyl 3-[1-(cyclobutylamino)ethyl]-1-(4-methylbenzyl)-1H-indole-2-carboxy late

To a solution of the compound (200 mg) produced in Reference Example 12 in DCE (3 mL), tetraethyl orthotitanate (0.25 mL) and cyclobutylamine (59 mg) were added, and the mixture was stirred at 60°C overnight. The reaction mixture was cooled to room temperature, sodium borohydride (45 mg) and methanol (0.75 mL) were added thereto, and the mixture was stirred at room temperature for 4 hours. Methanol was added to the reaction mixture, the mixture was filtered through Celite (trade name), and the filtrate was concentrated under reduced pressure. The resulting residue was purified by silica gel column chromatography (hexane : ethyl acetate = 4 : 1 → 1 : 1 → 0 : 1 → ethyl acetate : methanol = 9 : 1) to obtain the title compound (130 mg) having the following physical property values.
TLC: Rf 0.12 (ethyl acetate);
¹H-NMR (CDCl₃): δ 1.33, 1.42-1.92, 1.55, 2.09-2.23, 2.28, 3.13-3.27, 4,33, 4.75, 5.65, 6.90, 7.05, 7.08-7.17, 7.22-7.35, 8.05.

### Example 5: 3-[1-(Cyclobutylamino)ethyl]-1-(4-methylbenzyl)-1H-indole-2-carboxylic acid

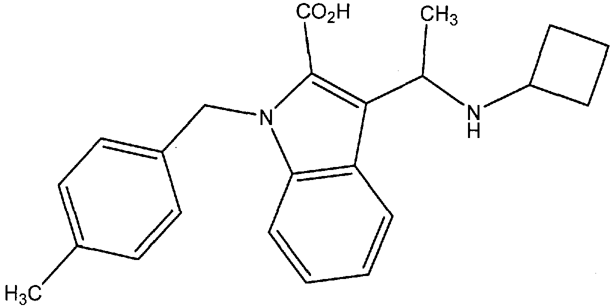

Using the compound produced in Reference Example 13, the same procedure as in Example 2 was carried out to obtain the title compound having the following physical property values.
LCMS (A) Retention time (min): 0.85;
MS (ESI, Pos.): 292 (M-*c*-BuNH₂+H)⁺.
¹H-NMR (DMSO-d₆): δ 1.51, 1.52-1.75, 1.78-1.91, 1.94-2.27, 4.53-4.67, 5.68, 6.16, 6.91-7.09, 7.14, 7.38, 7.66, 9.13, 13.93.

### Example 5(1): 1-Benzyl-3-[1-(cyclobutylamino)ethyl]-1H-indole-2-carboxylic acid

The same procedure as in Reference Example 11 → Reference Example 12 → Reference Example 13 → Example 5 was carried out using benzyl bromide in place of 1-(bromomethyl)-4-methylbenzene to obtain the title compound having the following physical property.
LCMS (A) Retention time (min): 0.83;
MS (ESI, Pos.): 349 (M+H)⁺.
¹H-NMR (CDCl₃): δ 1.35-1.94, 2.03-2.27, 2.38-2.59, 3.35-3.58, 4.61-4.76, 5.87, 6.31, 7.06-7.44, 7.58, 10.80, 12.72.

### Reference Example 14: Ethyl 4-bromo-5-methyl-1H-pyrrole-2-carboxylate

N-Bromosuccinimide (2.9 g) was added to a solution of ethyl 5-methyl-1H-pyrrole-2-carboxylate (2.5 g) in DCM (35 mL) under ice bath, and the reaction mixture was stirred under ice bath for one and a half hours. The reaction mixture was poured into 2 N sodium hydroxide (100 mL), ethyl acetate, a saturated aqueous sodium bicarbonate solution and a saturated aqueous sodium thiosulfate solution were added thereto, and the mixture was extracted. The organic layer was washed with saturated saline solution, dried over sodium sulfate, and then concentrated under reduced pressure to obtain the title compound (3.8 g) having the following physical property values.
TLC: Rf 0.51 (hexane : ethyl acetate = 4 : 1);
¹H-NMR (CDCl₃): δ 1.34, 2.27, 4.29, 6.83, 9.05.

### Reference Example 15: Ethyl 4-bromo-3-iodo-5-methyl-1H-pyrrole-2-carboxylate

To a solution of the compound (2.5 g) produced in Reference Example 14 in acetone (35 mL), 1,3-diiodo-5,5-dimethylhydantoin (2.9 g) was added, and the reaction mixture was stirred at room temperature for 2 hours. Furthermore, 1,3-diiodo-5,5-dimethylhydantoin (800 mg) was added thereto, and the mixture was stirred at room temperature for 2 hours. Ethyl acetate, a saturated aqueous sodium bicarbonate solution and a saturated aqueous sodium thiosulfate solution were added to the reaction mixture, and the mixture was extracted. The organic layer was washed with saturated saline solution, dried over sodium sulfate, and then concentrated under reduced pressure to obtain the title compound (3.9 g) having the following physical property values.
TLC: Rf 0.47 (hexane : ethyl acetate = 4 : 1);
¹H-NMR (CDCl₃): δ 1.39, 2.36, 4.35, 9.24.

### Reference Example 16: Ethyl 4-bromo-3-iodo-5-methyl-1-(4-methylbenzyl)-1H-pyrrole-2-carboxylate

To a solution of the compound (2.0 g) produced in Reference Example 15 in DMF (20 mL), sodium hydride (60%, 246 mg) was added under ice bath, and the reaction mixture was stirred under ice bath for 10 minutes and at room temperature for 30 minutes. 1-(Bromomethyl)-4-methylbenzene (1.1 g) was added thereto under ice bath, and the mixture was stirred under ice bath for 10 minutes and at room temperature for 2 hours. Furthermore, 1-(bromomethyl)-4-methylbenzene (150 mg) and sodium hydride (60%, 34 mg) were added thereto, and the mixture was stirred at room temperature for 2 hours. Ethyl acetate, a saturated aqueous ammonium chloride solution and saturated saline solution were added to the reaction mixture, and the mixture was extracted. The organic layer was dried over sodium sulfate, and then concentrated under reduced pressure. The resulting residue was purified by silica gel column chromatography (hexane : ethyl acetate = 19 : 1 → 9 : 1 → 4 : 1) to obtain the title compound (2.0 g) having the following physical property values.
TLC: Rf 0.70 (hexane : ethyl acetate = 4 : 1);
¹H-NMR (CDCl₃): δ 1.33, 2.26, 2.30, 4.27, 5.58, 6.82, 7.09.

### Reference Example 17: Ethyl 4-bromo-5-methyl-1-(4-methylbenzyl)-3-vinyl-1H-pyrrole-2-carboxylate

To a solution of the compound (1.0 g) produced in Reference Example 16 in toluene (12 mL), tributyl(vinyl)tin (0.95 mL) and bis(triphenylphosphine)palladium(II) dichloride (152 mg) were added, and the reaction mixture was stirred at 95°C for 7 hours. The reaction mixture was cooled to room temperature and then concentrated under reduced pressure. The resulting residue was purified by silica gel column chromatography (hexane : ethyl acetate = 19 : 1 → 9 : 1) to obtain the title compound (700 mg) having the following physical property values.
LCMS (A) Retention time (min): 1.30
MS (ESI, Pos.): 362 (M+H)⁺.
¹H-NMR (CDCl₃): δ 1.27, 2.20, 2.30, 4.23, 5.40, 5.55, 5.97, 6.83, 7.02-7.14.

### Reference Example 18: Ethyl 4-bromo-3-formyl-5-methyl-1-(4-methylbenzyl)-1H-pyrrole-2-carboxylate

To a solution of the compound (700 mg) produced in Reference Example 17 in THF (15 mL) and water (5 mL), sodium periodate (1.2 g) and osmium(VIII) oxide (4% aqueous solution, 6 drops) were added, and the reaction mixture was stirred at room temperature overnight. Furthermore, sodium periodate (827 mg) and osmium(VIII) oxide (4% aqueous solution, 3 drops) were added thereto, and the mixture was stirred at room temperature for 5 hours. Ethyl acetate and a saturated aqueous sodium bicarbonate solution were added to the reaction mixture, and the mixture was extracted. The organic layer was washed with saturated saline solution, dried over sodium sulfate, and then concentrated under reduced pressure. The resulting residue was purified by silica gel column chromatography (hexane : ethyl acetate = 9 : 1 → 4 : 1) to obtain the title compound (300 mg) having the following physical property values.
TLC: Rf 0.46 (hexane : ethyl acetate = 4 : 1);
¹H-NMR (CDCl₃): δ 1.32, 2.21, 2.31, 4.33, 5.59, 6.84, 7.11, 10.42.

### Reference Example 19: Ethyl 3-formyl-4-(4-methoxyphenyl)-5-methyl-1-(4-methylbenzyl)-1H-pyrrole-2-carboxylate

To a solution of the compound (19 mg) produced in Reference Example 18 in DMF (0.7 mL), water (0.17 mL), 4-methoxyphenylboronic acid (10 mg), potassium carbonate (11 mg) and tetrakis(triphenylphosphine)palladium(0) (9 mg) were added, and the mixture was stirred at 100°C for 30 minutes using microwaves. The reaction mixture was cooled to room temperature, then ethyl acetate and saturated saline solution were added thereto, and the mixture was extracted. The organic layer was dried over sodium sulfate, and then concentrated under reduced pressure to obtain the title compound having the following physical property values. The obtained title compound was used as it was for the next reaction.
LCMS (A) Retention time (min): 1.18
MS (ESI, Pos.): 414 (M+Na)⁺.

### Reference Example 20: Ethyl 3-[(cyclobutylamino)methyl]-4-(4-methoxyphenyl)-5-methyl-1-(4-methylbenzyl)-1H-pyrrole-2-carboxylate

Using the compound produced in Reference Example 19, the same procedure as in Reference Example 4 was carried out to obtain the title compound having the following physical property values.
LCMS (A) Retention time (min): 1.01
MS (ESI, Pos.): 447 (M+H)⁺.

### Example 6: 3-[(Cyclobutylamino)methyl]-4-(4-methoxyphenyl)-5-methyl-1-(4-methylbenzyl)-1H-pyrrole-2-carboxylic acid

Using the compound produced in Reference Example 20, the same procedure as in Example 2 was carried out to obtain the title compound having the following physical property values.
LCMS (A) Retention time (min): 0.93;
MS (ESI, Pos.): 419 (M+H)⁺.
¹H-NMR (DMSO-d₆): δ 1.62-1.80, 1.86-2.12, 3.34-3.50, 3.62, 3.75, 5.77, 6.88-6.98, 7.03-7.13, 11.50.

### Example 6(1) to Example 6(7)

The same procedure as in Reference Example 19 to Reference Example 20 to Example 6 was carried out by using a corresponding boronic acid compound in place of 4-methoxyphenylboronic acid to obtain the following compounds.

### Example 6(1): 3-[(Cyclobutylamino)methyl]-5-methyl-1-(4-methylbenzyl)-4-phenyl-1H-pyrrole-2-carboxylic acid

LCMS (A) Retention time (min): 0.93;
MS (ESI, Pos.): 389 (M+H)⁺.
¹H-NMR (DMSO-d₆): δ 1.60-1.80, 1.86-2.10, 2.23, 3.36-3.52, 3.64, 5.79, 6.93, 7.08, 7.15-7.22, 7.23-7.31, 7.34-7.43, 11.54.

### Example 6(2): 3-[(Cyclobutylamino)methyl]-4-(3-fluorophenyl)-5-methyl-1-(4-methylbenzyl)-1pyrrole-2-carboxylic acid

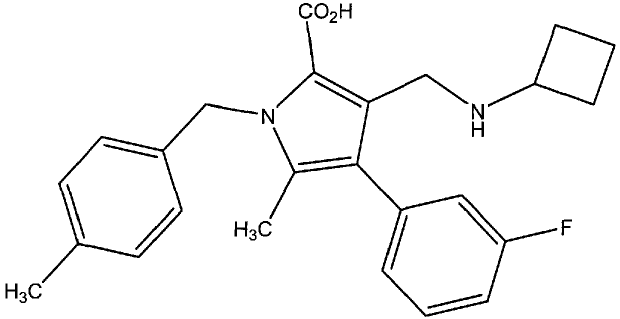

LCMS (A) Retention time (min): 0.94;
MS (ESI, Pos.): 407 (M+H)⁺.
¹H-NMR (DMSO-d₆): δ 1.64-1.79, 1.94-2.09, 2.24, 3.43-3.51, 3.69, 5.78, 6.94, 7.02-7.16, 7.41-7.47, 11.23.

### Example 6(3): 3-[(Cyclobutylamino)methyl]-4-(2-fluorophenyl)-5-methyl-1-(4-methylbenzyl)-1H-pyrrole-2-carboxylic acid

LCMS (A) Retention time (min): 0.93;
MS (ESI, Pos.): 407 (M+H)⁺.
¹H-NMR (DMSO-d₆): δ 1.59-1.76, 1.90-2.02, 2.25, 3.37-3.46, 3.57-3.74, 5.70-5.85, 6.93, 7.10, 7.19-7.32, 7.34-7.43.

### Example 6(4): 3-[(Cyclobutylamino)methyl]-5-methyl-1-4-methylbenzyl)-4-(3-thienyl)-1H-pyrrole-2-carboxylic acid

LCMS (A) Retention time (min): 0.92;
MS (ESI, Pos.): 395 (M+H)⁺.
¹H-NMR (DMSO-d₆): δ 1.53-1.73, 1.74-1.90, 1.92-2.12, 2.22, 3.35-3.45, 3.56, 5.78, 6.92, 7.02-7.12, 7.21, 7.53.

### Example 6(5): 3-[(Cyclobutylamino)methyl]-5-methyl-1-(4-methylbenzyl)-4-(2-thienyl)-1H-pyrrole-2-carboxylic acid

LCMS (A) Retention time (min): 0.92;
MS (ESI, Pos.): 395 (M+H)⁺.
¹H-NMR (DMSO-d₆): δ 1.63-1.79, 1.89-2.13, 2.23, 3.37-3.52, 3.72, 5.79, 6.88, 6.93, 7.04-7.12, 7.52, 11.45.

### Example 6(6): 3-[(Cyclobutylamino)methyl]-4-isopropenyl-5-methyl-1-(4-methylbenzyl)-1H-pyrrole-2-carboxylic acid

LCMS (A) Retention time (min): 0.88;
MS (ESI, Neg.): 351 (M-H)⁻.
¹H-NMR (CD₃OD): δ 1.80-1.97, 2.05, 2.10-2.34, 3.60-3.74, 3.91, 4.76-4.80, 5.28-5.32, 5.73, 6.86, 7.04.

### Example 6(7): 3-[(Cyclobutylamino)methyl]-5-methyl-1-(4-methylbenzyl)-4-vinyl-1H-pyrrole-2-carboxylic acid

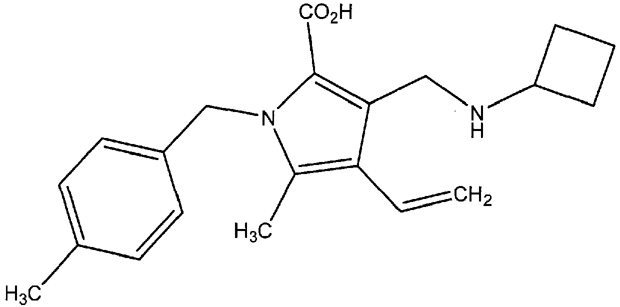

LCMS (A) Retention time (min): 0.83;
MS (ESI, Neg.): 337 (M-H)⁻.
¹H-NMR (CDCl₃): δ 1.80-1.97, 2.14, 2.15-2.37, 3.63-3.77, 4.05, 5.18, 5.28, 5.76, 6.65, 6.85, 7.05.

### Reference Example 21: Ethyl 4,5,6,7-tetrahydro-1H-indole-2-carboxylate

To a solution of 4,5,6,7-tetrahydro-1H-indole (3.0 g) in DCE (50 mL) was added trichloroacetyl chloride (1.4 g), and the mixture was stirred at 85°C for 2 hours. The reaction mixture was cooled to room temperature and then concentrated under reduced pressure, and then ethanol (40 mL) and sodium ethoxide (about 20% ethanol solution, 0.85 mL) were added thereto. The reaction mixture was cooled to room temperature and stirred overnight, and then concentrated under reduced pressure, and the resulting residue was purified by silica gel column chromatography (hexane : ethyl acetate = 9 : 1 → 4 : 1 → 2 : 1) to obtain the title compound (500 mg) having the following physical property value.

¹H-NMR (CDCl₃): δ 1.33, 1.67-1.87, 2.44-2.64, 4.28, 6.66, 8.61.

### Reference Example 22: Ethyl 3-formyl-4,5,6,7-tetrahydro-1H-indole-2-carboxylate

Using the compound produced in Reference Example 21, the same procedure as in Reference Example 1 was carried out to obtain the title compound having the following physical property value.

¹H-NMR (CDCl₃): δ 1.31, 1.65-1.83, 2.31, 2.41-2.50, 2.78-2.88, 4.31, 5.51, 6.84, 7.10, 10.52.

### Reference Example 23: Ethyl 3-formyl-1-(4-methylbenzyl)-4,5,6,7-tetrahydro-1H-indole-2-carboxylate

To a solution of the compound (50 mg) produced in Reference Example 22 in DMF (1 mL), cesium carbonate (147 mg) and 1-(bromomethyl)-4-methylbenzene (63 mg) were added, and the mixture was stirred at 100°C for 30 minutes using microwaves. Water and ethyl acetate were added to the reaction mixture, and the mixture was extracted, and then washed with saturated saline solution. The organic layer was dried over sodium sulfate, and then concentrated under reduced pressure, and the resulting residue was purified by silica gel column chromatography (hexane : ethyl acetate = 10 : 0 → 7 : 3) to obtain the title compound (69 mg) having the following physical property value.

¹H-NMR (CDCl₃): δ 1.31, 1.66-1.84, 2.31, 2.41-2.50, 2.79-2.86, 4.31, 5.51, 6.84, 7.10, 10.52.

### Reference Example 24: Ethyl 3-[(cyclobutylamino)methyl]-1-(4-methylbenzyl)-4,5,6,7-tetrahydro-1H-indole-2-carboxylate

Using the compound produced in Reference Example 23, the same procedure as in Reference Example 4 was carried out to obtain the title compound having the following physical property values.
LCMS (A) Retention time (min): 0.91;
MS (ESI, Pos.): 381 (M+H)⁺.

### Example 7: 3-[(Cyclobutylamino)methyl]-1-(4-methylbenzyl)-4,5,6,7-tetrahydro-1H-indole-2-carboxylic acid

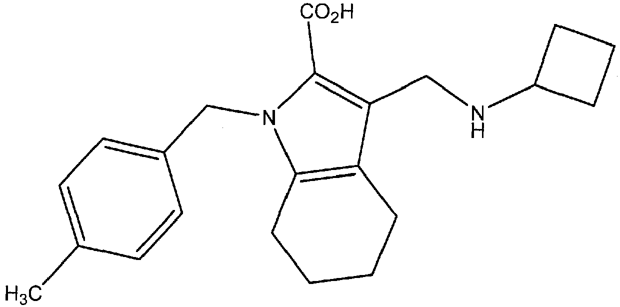

Using the compound produced in Reference Example 24, the same procedure as in Example 2 was carried out to obtain the title compound having the following physical property values.
LCMS (A) Retention time (min): 0.87;
MS (ESI, Pos.): 353 (M+H)⁺.
¹H-NMR (DMSO-d₆): δ 1.50-1.83, 1.92-2.18, 2.22, 2.22-2.38, 3.40-3.52, 3.62, 5.60, 6.87, 7.04, 11.52.

### Reference Example 25: Ethyl 5-bromo-3-formyl-1H-indole-2-carboxylate

The same procedure as in Reference Example 1 was carried out using ethyl 5-bromoindole-2-carboxylate to obtain the title compound having the following physical property values.
LCMS (B) Retention time (min): 1.16;
MS (ESI, Pos.): 296 (M+H)⁺.
¹H-NMR (CDCl₃): δ1.48, 4.54, 7.35, 7.52, 8.67, 9.28, 10.71.

### Reference Example 26: Ethyl 5-bromo-3-formyl-1-(4-methylbenzyl)-1H-indole-2-carboxylate

Using the compound produced in Reference Example 25, the same procedure as in Reference Example 16 was carried out to obtain the title compound having the following physical property values.
TLC: Rf 0.53 (hexane : ethyl acetate = 4 : 1);
¹H-NMR (CDCl₃): δ 1.40, 2.29, 4.46, 5.75, 6.93, 7.08, 7.26, 7.46, 8.72, 10.59.

### Reference Example 27: Ethyl 3-formyl-1-(4-methylbenzyl)-5-(methylsulfonyl)-1H-indole-2-carboxylate

To a solution of the compound (140 mg) produced in Reference Example 26 in N-methylpyrrolidone (hereinafter, abbreviated as NMP) (2 mL), sodium methanesulfinate (143 mg) and copper(I) iodide (266 mg) were added, and the mixture was stirred at 130°C for 1 hour using microwaves. Ethyl acetate and a saturated aqueous ammonium chloride solution were added thereto, and the reaction mixture was filtered through Celite (trade name) and then extracted. The organic layer was dried over sodium sulfate, and then concentrated under reduced pressure. The resulting residue was purified by silica gel column chromatography (hexane : ethyl acetate = 9 : 1 → 4 : 1 → 2 : 1) to obtain the title compound (58 mg) having the following physical property values.
TLC: Rf 0.53 (hexane : ethyl acetate = 1 : 1);
¹H-NMR (CDCl₃): δ 1.41, 2.30, 3.11, 4.49, 5.82, 6.95, 7.10, 7.55, 7.93, 9.17, 10.63.

### Reference Example 28: Ethyl 3-[(cyclobutylamino)methyl]-1-(4-methylbenzyl)-5-(methylsulfonyl)-1H-indole-2-carboxylate

Using the compound produced in Reference Example 27, the same procedure as in Reference Example 4 was carried out to obtain the title compound having the following physical property values.
LCMS (B) Retention time (min): 1.04;
MS (ESI, Pos.): 455 (M+H)⁺.

### Example 8: 3-[(Cyclobulylamino)methyl]-1-(4-methylbenzyl)-5-(methylsulfonyl)-1H-indole-2-carboxylic acid

Using the compound produced in Reference Example 28, the same procedure as in Example 2 was carried out to obtain the title compound having the following physical property values.
LCMS (B) Retention time (min): 0.91;
MS (ESI, Pos.): 427 (M+H)⁺.
¹H-NMR (DMSO-d₆): δ 1.72-1.88, 2.12-2.24, 3.17, 3.63-3.73, 4.28, 6.04, 7.02-7.07, 7.64-7.71, 8.38, 10.82.

### Example 8(1) to Example 8(2)

The same procedure as in Reference Example 25 to Reference Example 26 to Reference Example 27 to Reference Example 28 to Example 8 was carried out by using a corresponding indole compound in place of ethyl 5-bromoindole-2-carboxylate to obtain the following compounds.

### Example 8(1): 3-[(Cyclobutylamino)methyl]-1-(4-methylbenzyl)-7-(methylsulfonyl)-1H-indole-2-carboxylic acid

LCMS (B) Retention time (min): 0.92;
MS (ESI, Pos.): 427 (M+H)⁺.
¹H-NMR (DMSO-d₆): δ 1.68-1.82, 2.05-2.16, 2.19, 3.01, 3.53-3.63, 4.23, 6.51, 6.57, 6.97, 7.36, 7.93, 8.21, 10.87.

### Example 8(2): 3-[(Cyclobutylamino)methyl]-1-(4-methylbenzyl)-4-(methylsulfonyl)-1H-indole-2-carboxylic acid

LCMS (B) Retention time (min): 0.93;
MS (ESI, Pos.): 427 (M+H)⁺.
¹H-NMR (CDCl₃): δ 1.50-1.85, 1.90-2.37, 3.04, 3.73-3.87, 4.79, 5.72, 6.80-6.92, 7.25, 7.53, 7.82, 10.47.

### Reference Example 29: Methyl 6-bromo-3-formyl-1H-indole-2-carboxylate

The same procedure as in Reference Example 1 was carried out using methyl 6-bromoindole-2-carboxylate to obtain the title compound having the following physical property values.
TLC: Rf 0.58 (hexane : ethyl acetate = 1 : 1);
¹H-NMR (DMSO-d₆): δ 4.00, 7.45, 7.72, 8.18, 10.58, 12.99.

### Reference Example 30: Methyl 6-bromo-3-formyl-1-(4-methylbenzyl)-1H-indole-2-carboxylate

Using the compound produced in Reference Example 29, the same procedure as in Reference Example 16 was carried out to obtain the title compound having the following physical property values.
TLC: Rf 0.60 (hexane : ethyl acetate = 2 : 1);
LCMS (B) Retention time (min): 1.34;
MS (ESI, Pos.): 386 (M+H)⁺.
¹H-NMR (CDCl₃): δ 2.31, 3.98, 5.73, 6.94, 7.10, 7.45, 7.57, 8.40, 10.58.

### Reference Example 31: Methyl 6-cyano-3-formyl-1-(4-methylbenzyl)-1H-indole-2-carboxylate

To a solution of the compound (120 mg) produced in Reference Example 30 in NMP (0.7 mL), copper(I) cyanide (83 mg) and tetrakis(triphenylphosphine)palladium(0) (36 mg) were added, and the mixture was stirred at 150°C for 30 minutes using microwaves. Ethyl acetate and a saturated aqueous ammonium chloride solution were added to the reaction mixture, and the mixture was extracted. The organic layer was dried over sodium sulfate, and then concentrated under reduced pressure. The resulting residue was purified by silica gel column chromatography (hexane : ethyl acetate = 9 : 1 → 4 : 1→ 2 : 1) to obtain the title compound (86 mg) having the following physical property values.
TLC: Rf 0.46 (hexane : ethyl acetate = 2 : 1);
LCMS (B) Retention time (min): 1.23;
MS (ESI, Pos.): 333 (M+H)⁺.
¹H-NMR (CDCl₃): δ 2.32, 4.03, 5.78, 6.95, 7.12, 7.56, 7.73, 8.64, 10.58.

### Reference Example 32: Methyl 6-cyano-3-[(cyclobutylamino)methyl]-1-(4-methylbenzyl)-1H-indole-2-carboxylate

Using the compound produced in Reference Example 31, the same procedure as in Reference Example 4 was carried out to obtain the title compound having the following physical property values.
LCMS (B) Retention time (min): 1.06;
MS (ESI, Pos.): 388 (M+H)⁺.

### Example 9: 6-Cyano-3-[(cyclobutylamino)methyl]-1-(4-methylbenzyl)-1H-indole-2-carboxylic acid

Using the compound produced in Reference Example 32, the same procedure as in Example 2 was carried out to obtain the title compound having the following physical property values.
LCMS (B) Retention time (min): 0.97;
MS (ESI, Pos.): 374 (M+H)⁺.
¹H-NMR (DMSO-d₆): δ 1.69-1.85, 2.10-2.19, 2.21, 3.59-3.67, 4.23, 6.02, 7.02-7.11, 7.43, 7.91, 8.09, 10.74.

### Example 9(1): 7-Cyano-3-[(cyclobutylamino)methyl]-1-(4-methylbenzyl)-1H-indole-2-carboxylic acid

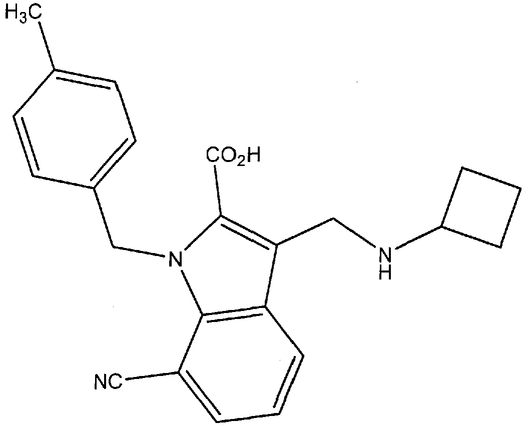

The same procedure as in Reference Example 29 to Reference Example 30 to Reference Example 31 to Reference Example 32 to Example 9 was carried out by using a corresponding indole compound in place of methyl 6-bromoindole-2-carboxylate to obtain the following compounds.
LCMS (B) Retention time (min): 0.96;
MS (ESI, Pos.): 374 (M+H)⁺.
¹H-NMR (DMSO-d₆): δ 1.69-1.88, 2.10-2.20, 2.22, 3.59-3.70, 4.25, 6.35, 6.80, 7.04, 7.27, 7.68, 8.16, 10.82.

### Reference Example 33: Ethyl 6-bromo-4H-pyrrolo[2,3-d][1,3]thiazole-5-carboxylate

The same procedure as in Reference Example 14 was carried out using ethyl 4H-pyrrolo[2,3-d][1,3]thiazole-5-carboxylate to obtain the title compound having the following physical property values.
LCMS (B) Retention time (min): 1.08;
MS (ESI, Pos.): 277 (M+H)⁺.
¹H-NMR (DMSO-d₆): δ 1.33, 4.32, 9.16, 13.19.

### Reference Example 34: Ethyl 6-bromo-4-(4-methylbenzyl)-4H-pyrrolo[2,3-d][1,3]thiazole-5-carboxylate

Using the compound produced in Reference Example 33, the same procedure as in Reference Example 3 was carried out to obtain the title compound having the following physical property values.
LCMS (B) Retention time (min): 1.40;
MS (ESI, Pos.): 379 (M+H)⁺.
¹H-NMR (CDCl₃): δ 1.37, 2.28, 4.35, 5.86, 7.06, 7.12, 8.74.

### Reference Example 35: Ethyl 4-(4-methylbenzyl)-6-vinyl-4H-pyrrolo[2,3-d][1,3]thiazole-5-carboxylate

Using the compound produced in Reference Example 34, the same procedure as in Reference Example 17 was carried out to obtain the title compound having the following physical property values.
LCMS (B) Retention time (min): 1.43;
MS (ESI, Pos.): 327 (M+H)⁺.
¹H-NMR (DMSO-d₆): δ 1.26, 2.23, 4.29, 5.49, 5.53, 5.77, 6.99, 7.08, 7.46, 9.27.

### Reference Example 36: Ethyl 6-formyl-4-(4-methylbenzyl)-4H-pyrrolo[2,3-d][1,3]thiazole-5-carboxylate

Using the compound produced in Reference Example 35, the same procedure as in Reference Example 18 was carried out to obtain the title compound having the following physical property values.
LCMS (B) Retention time (min): 1.29;
MS (ESI, Pos.): 329 (M+H)⁺.
¹H-NMR (DMSO-d₆): δ 1.31, 2.24, 4.39, 5.85, 7.08-7.14, 9.31, 10.38.

### Reference Example 37: Ethyl 6-[(cyclobutylamino)methyl]-4-(4-methylbenzyl)-4H-pyrrolo[2,3-d][1,3]thiazole-5-carboxylate

Using the compound produced in Reference Example 36, the same procedure as in Reference Example 4 was carried out to obtain the title compound having the following physical property values.
LCMS (B) Retention time (min): 1.07;
MS (ESI, Pos.): 384 (M+H)⁺.

### Example 10: 6-[(Cyclobutylamino)methyl]-4-(4-methylbenzyl)-4H-pyrrolo[2,3-d][1,3]thiazole-5-carboxylic acid

Using the compound produced in Reference Example 37, the same procedure as in Example 2 was carried out to obtain the title compound having the following physical property values.
LCMS (B) Retention time (min): 0.88;
MS (ESI, Pos.): 356 (M+H)⁺.
¹H-NMR (DMSO-d₆): δ 1.72-1.85, 2.08-2.26, 3.65-3.80, 4.35, 5.83, 7.04-7.12, 8.84, 9.20.

### Example 10(1) to Example 10(2)

The same procedure as in Reference Example 33 to Reference Example 34 to Reference Example 35 to Reference Example 36 to Reference Example 37 to Example 10 was carried out by using a corresponding pyrrole compound in place of ethyl 4H-pyrrolo[2,3-d][1,3]thiazole-5-carboxylate to obtain the following compounds.

### Example 10(1): 3-[(Cyclobutylamino)methyl]-1-(4-methylbenzyl)-1H-pyrrolo[3,2-c]pyridine-2-carboxylic acid

LCMS (A) Retention time (min): 0.56;
MS (ESI, Pos.): 350 (M+H)⁺.
¹H-NMR (DMSO-d₆): δ 1.66-1.84, 2.07-2.28, 3.55-3.70, 4.24, 5.93, 6.99-7.07, 7.43, 8.18, 8.98, 10.91.

### Example 10(2): 5-[(Cyclobutylamino)methyl]-7-(4-methylbenzyl)-7H-pyrrolo[2,3-dlpyrimidine-6-carboxylic acid

LCMS (B) Retention time (min): 0.81;
MS (ESI, Pos.): 351 (M+H)⁺.
¹H-NMR (DMSO-d₆): δ 1.70-1.85, 2.10-2.19, 2.21, 3.60-3.72, 4.26, 5.97, 7.03, 7.09, 8.14, 8.87, 9.23, 10.75.

### Reference Example 38: Ethyl 5-bromo-3-(cyclobutylamino)methyl]-1-(4-methylbenzyl)-1H-indole-2-carboxylate

Using the compound produced in Reference Example 26, the same procedure as in Reference Example 4 was carried out to obtain the title compound having the following physical property values.
LCMS (B) Retention time (min): 1.13;
MS (ESI, Pos.): 455 (M+H)⁺.
¹H-NMR (CDCl₃): δ 1.35, 1.60-1.93, 1.96, 2.09-2.20, 2.28, 3.38-3.50, 4.15, 4.38, 4.72, 5.70, 6.86, 7.05, 7.20, 7.38, 7.93.

### Reference Example 39: Ethyl 3-[(cyclobutylamino)methyl]-5-(dimethylphosphoryl)-1-(4-methylbenzyl)-1H-indole-2-carboxylate

To a solution of the compound (30 mg) produced in Reference Example 38 in DMF (0.45 mL), dimethylphosphine oxide (15 mg), potassium carbonate (27 mg) and bis(tri-tert-butylphosphine)palladium(0) (3.4 mg) were added, and the mixture was stirred at 100°C for 1 hour. The reaction mixture was cooled to room temperature, the resulting solid was filtered, and the obtained filtrate was concentrated under reduced pressure to obtain the title compound having the following physical property values. The obtained title compound was used as it was for the next reaction.
LCMS (B) Retention time (min): 0.97;
MS (ESI, Pos.): 453 (M+H)⁺.

### Example 11: 3-[(Cyclobutylamino)methyl]-5-(dimethylphosphoryl)-1-(4-methylbenzyl)-1H-indole-2-carboxylic acid

Using the compound produced in Reference Example 39, the same procedure as in Example 2 was carried out to obtain the title compound having the following physical property values.
LCMS (B) Retention time (min): 0.84;
MS (ESI, Pos.): 425 (M+H)⁺.
¹H-NMR (DMSO-d₆): δ 1.67, 1.76-1.85, 2.15-2.26, 3.75-3.84, 4.48, 5.90, 6.96, 7.06, 7.66, 7.72, 8.32, 9.01.

### Reference Example 40: Ethyl 7-bromo-3-formyl-1H-indole-2-carboxylate

The same procedure as in Reference Example 1 was carried out using ethyl 7-bromoindole-2-carboxylate to obtain the title compound having the following physical property values.
TLC: Rf 0.79 (hexane : ethyl acetate = 1 : 1);

### Reference Example 41: Ethyl 7-bromo-3-formyl-1-(4-methylbenzyl)-1H-indole-2-carboxylate

Using the compound produced in Reference Example 40, the same procedure as in Reference Example 11 was carried out to obtain the title compound having the following physical property values.
TLC: Rf 0.74 (hexane : ethyl acetate = 2 : 1);
LCMS (B) Retention time (min): 1.39;
MS (ESI, Pos.): 400 (M+H)⁺.
¹H-NMR (CDCl₃): δ 1.33, 2.29, 4.41, 6.28, 6.82, 7.07, 7.19, 7.59, 8.57, 10.50.

### Reference Example 42: Ethyl 3-formyl-1-(4-methylbenzyl)-7-[(trimethylsilyl)ethynyl]-1H-indole-2-carboxylate

To a solution of the compound (73 mg) produced in Reference Example 41 in DMF (0.9 mL), triethylamine (0.076 mL), trimethylsilylacetylene (27 mg), copper(I) iodide (3.5 mg), and bis(triphenylphosphine)palladium(II) dichloride (13 mg) were added, and the mixture was stirred at 70°C for 4 hours. The reaction mixture was cooled to room temperature, then ethyl acetate and a saturated aqueous ammonium chloride solution were added thereto, and the mixture was extracted. The organic layer was dried over sodium sulfate, and then concentrated under reduced pressure. The resulting residue was purified by silica gel column chromatography (hexane : ethyl acetate = 10 : 0 → 19 : 1→ 9 : 1) to obtain the title compound (25 mg) having the following physical property values.
TLC: Rf 0.43 (hexane : ethyl acetate = 4 : 1);
LCMS (B) Retention time (min): 1.52;
MS (ESI, Pos.): 418 (M+H)⁺.

### Reference Example 43: Ethyl 3-[(cyclobutylamino)methyl]-1-(4-methylbenzyl)-7-[(trimethylsilylethynyl]-1H-indole-2-carboxylate

Using the compound produced in Reference Example 42, the same procedure as in Reference Example 4 was carried out to obtain the title compound having the following physical property values.
LCMS (B) Retention time (min): 1.30;
MS (ESI, Pos.): 473 (M+H)⁺.

### Example 12: 3-[(Cyclobutylamino)methyl]-7-ethynyl-1-(4-methylbenzyl)-1H-indole-2-carboxylic acid

Using the compound produced in Reference Example 43, the same procedure as in Example 2 was carried out to obtain the title compound having the following physical property values.
LCMS (B) Retention time (min): 0.99;
MS (ESI, Pos.): 373 (M+H)⁺.
¹H-NMR (CDCl₃): δ 1.48-1.75, 1.86-1.99, 2.12-2.28, 3.29-3.40, 4.25, 6.54, 6.92, 6.96, 7.09, 7.45, 7.56, 11.05.

### Example 13: 3-[(Cyclobutylamino)methyl]-7-ethyl-1-(4-methylbenzyl)-1H-indole-2-carboxylic acid

To a solution of the compound (8 mg) produced in Example 12 in ethanol (1.5 mL), 10% palladium-activated carbon (50% wet, 4 mg) was added, and the mixture was stirred under a hydrogen atmosphere at room temperature for 1 hour. The reaction mixture was filtered through Celite (trade name), and then the filtrate was concentrated to obtain the title compound (8 mg) having the following physical property values.
LCMS (B) Retention time (min): 1.02;
MS (ESI, Pos.): 377 (M+H)⁺.
¹H-NMR (CDCl₃): δ 1.25, 1.42-1.66, 1.88-2.01, 2.07-2.27, 2.81-2.94, 3.27-3.40, 4.26, 6.22, 6.70, 6.94, 7.03, 7.10, 7.40.

### Reference Example 44: Ethyl 3-[(E)-(hydroxyimino)methyl]-1-(4-methylbenzyl)-1H-indole-2-carboxylate

To a solution of the compound (943 mg) produced in Reference Example 3 in ethanol (9.4 mL), pyridine (0.48 mL) and hydroxylamine hydrochloride (408 mg) were added, and the mixture was stirred at 65°C for 30 minutes. The reaction mixture was cooled to room temperature and then concentrated under reduced pressure, and water was added to the resulting residue. The resulting solid was filtered and washed with 2 N hydrochloric acid and water to obtain the title compound (1.0 g) having the following physical property values.
LCMS (B) Retention time (min): 1.26;
MS (ESI, Pos.): 337 (M+H)⁺.

### Reference Example 45: Ethyl 3-(aminomethyl)-1-(4-methylbenzyl)-1H-indole-2-carboxylate

Acetic acid (9.4 mL) and zinc (768 mg) were added to the compound (1.0 g) produced in Reference Example 44, and the mixture was stirred at 70°C for 1 hour. The reaction mixture was cooled to room temperature, then added with DCM, and neutralized with 5 N sodium hydroxide, and the mixture was extracted. The organic layer was dried over sodium sulfate, and then concentrated under reduced pressure. Hexane and ethyl acetate were added to the resulting residue, and slurry washing was performed. The resulting solid was filtered and dried under reduced pressure to obtain the title compound (981 mg) having the following physical property values.
LCMS (B) Retention time (min): 1.03;
MS (ESI, Pos.): 306 (M-NH3+H)⁺.
¹H-NMR (CDCl₃): δ 1.32, 2.28, 4.34, 4.35, 5.70, 6.89, 7.04, 7.18, 7.30-7.35, 7.79.

### Reference Example 46: Ethyl 3-{[(1-deuterio)cyclobutylamino]methyl}-1-(4-methylbenzyl)-1H-indole-2-carboxylate

Acetic acid-d4 (0.28 mL) was added to a solution of sodium borodeuteride (66 mg) in acetonitrile (3 mL) under ice bath. After stirring at room temperature for 30 minutes, cyclobutanone (65 mg), diisopropylethylamine (0.21 mL) and the compound (300 mg) produced in Reference Example 45 were added thereto, and the mixture was stirred at room temperature for 30 minutes. DCE (3 mL) was added thereto, and the mixture was further stirred at room temperature overnight. Then, water and ethyl acetate were added thereto, and the mixture was extracted. The organic layer was dried over sodium sulfate, and then concentrated under reduced pressure. The resulting residue was purified by HPLC (column: YMC Triart C18 50 mm × 100 mm, 5 µm; flow rate: 100 mL/min; mobile phase A: 0.1% aqueous TFA solution; mobile phase B: 0.1% TFA acetonitrile solution: gradient (ratios of mobile phase (A) : mobile phase (B) are described): 0 to 0.50 min: (90% : 10%); 0.50 to 12.00 min: (90% : 10%) to (0% : 100%); 12.01 to 18.00 min: (0% : 100%)) to obtain the title compound (134 mg) having the following physical property value.

¹H-NMR (CDCl₃): δ 1.36, 1.55-1.77, 2.08-2.17, 2.27, 4.15, 4.37, 5.73, 6.90, 7.03, 7.18, 7.27-7.35, 7.79.

### Example 14: 3-{[(1-Deuterio)cyclobutylamino]methyl}-1-(4-methylbenzyl)-1H-indole-2-carboxylic acid

Using the compound produced in Reference Example 46, the same procedure as in Example 2 was carried out to obtain the title compound having the following physical property values.
LCMS (B) Retention time (min): 1.01;
MS (ESI, Pos.): 350 (M+H)⁺.
¹H-NMR (DMSO-d₆): δ 1.68-1.86, 2.08-2.20, 2.21, 4.17, 5.96, 7.00-7.05, 7.08, 7.16, 7.42, 7.70, 11.09.

### Reference Example 47: Ethyl 3-(deuterio)formyl-1H-indole-2-carboxylate

Phosphorus oxychloride (0.99 mL) was added to a solution of N,N-dimethylformamide-d7 (0.82 mL) in DCE (5 mL), and the mixture was stirred at room temperature for 10 minutes. Then, a solution of ethyl indole-2-carboxylate (1.0 g) in DCE (5 mL) was added thereto, and the reaction mixture was stirred at 60°C for 3 hours. The reaction mixture was cooled to room temperature, and then heavy water (1 mL), ethyl acetate, saturated saline solution, and a saturated aqueous sodium bicarbonate solution were added thereto. The resulting solid was filtered and dried under reduced pressure to obtain the title compound (590 mg) having the following physical property values.
TLC: Rf 0.24 (hexane : ethyl acetate = 2 : 1);
¹H-NMR (DMSO-d₆): δ 1.41, 4.46, 7.31, 7.40, 7.58, 8.25, 12.81.

### Reference Example 48: Ethyl 3-(deuterio)formyl-1-(4-methylbenzyl)-1H-indole-2-carboxylate

Using the compound produced in Reference Example 47, the same procedure as in Reference Example 3 was carried out to obtain the title compound having the following physical property values.
TLC: Rf 0.39 (hexane : ethyl acetate = 4 : 1);
LCMS (B) Retention time (min): 1.31;
MS (ESI, Pos.): 323 (M+H)⁺.
¹H-NMR (DMSO-d₆): δ 1.31, 2.23, 4.42, 5.80, 7.00, 7.10, 7.36, 7.41, 7.69, 8.32,.

### Reference Example 49: Ethyl 3-[(cyclobutylamino)(dideuterio)methyl]-1-(4-methylbenzyl)-1H-indole-2-carboxylate

To a solution of the compound (50 mg) produced in Reference Example 48 in DCE (1 mL), cyclobutylamine (17 mg) and acetic acid-d4 (0.1 mL) were added, and the mixture was stirred at 45°C for 4 hours. The reaction mixture was cooled to room temperature, then a solution of sodium deuterated boron (10 mg) in acetic acid-d4 (0.1 mL) was added thereto, and the mixture was stirred at room temperature overnight. Ethyl acetate and a saturated aqueous sodium bicarbonate solution were added to the reaction mixture, and the mixture was extracted. The organic layer was dried over sodium sulfate, and then concentrated under reduced pressure to obtain the title compound having the following physical property values. The obtained title compound was used as it was for the next reaction.
LCMS (B) Retention time (min): 1.11;
MS (ESI, Pos.): 379 (M+H)⁺.

### Example 15: 3-[(Cyclobutylamino)(dideuterio)methyl]-1-(4-methylbenzyl)-1H-indole-2-carboxylic acid

Using the compound produced in Reference Example 49, the same procedure as in Example 2 was carried out to obtain the title compound having the following physical property values.
LCMS (B) Retention time (min): 1.02;
MS (ESI, Pos.): 351 (M+H)⁺.
¹H-NMR (DMSO-d₆): δ 1.70-1.87, 2.12-2.25, 3.62-3.74, 5.92, 7.00, 7.05, 7.16, 7.26, 7.50, 7.80, 10.06.

### Reference Example 50: Ethyl 3-[(E)-(hydroxyimino)(deuterio)methyl]-1-(4-methylbenzyl)-1H-indole-2-carboxylate

Using the compound produced in Reference Example 48, the same procedure as in Reference Example 44 was carried out to obtain the title compound having the following physical property values.
TLC: Rf 0.20 (hexane : ethyl acetate = 4 : 1);
¹H-NMR (DMSO-d₆): δ 1.28, 2.22, 4.35, 5.76, 6.92, 7.07, 7.23, 7.37, 7.61, 8.25, 11.21.

### Reference Example 51: Ethyl 3-[amino(dideuterio)methyl]-1-(4-methylbenzyl)-1H-indole-2-carboxy late

Using the compound produced in Reference Example 50, and acetic acid-d4 in place of acetic acid, the same procedure as in Reference Example 45 was carried out to obtain the title compound having the following physical property values.
LCMS (B) Retention time (min): 1.01;
MS (ESI, Pos.): 308 (M-NH₃+H)⁺.

### Reference Example 52: Ethyl 3-{[(1-deuterio)cyclobulylamino](dideuterio)methyl}-1-(4-methylbenzyl)-1H-indole-2-carboxylate

Using the compound produced in Reference Example 51, the same procedure as in Reference Example 46 was carried out to obtain the title compound having the following physical property values.
LCMS (B) Retention time (min): 1.10;
MS (ESI, Pos.): 380 (M+H)⁺.

### Example 16: 3-{[(1-Deuterio)cyclobutylamino](dideuterio)methyl}-1-(4-methylbenzyl)-1H-indole-2-carboxylic acid

Using the compound produced in Reference Example 52, the same procedure as in Example 2 was carried out to obtain the title compound having the following physical property values.
LCMS (B) Retention time (min): 1.02;
MS (ESI, Pos.): 352 (M+H)⁺.
¹H-NMR (CDCl₃): δ 1.53-2.12, 2.22, 2.29-2.43, 5.88, 6.95-7.03, 7.17, 7.23-7.29, 7.34, 7.56, 10.28.

### Example 17: 3-[(Cyclobutylamino)methyl]-4-isopropyl-5-methyl-1-(4-methylbenzyl)-1H-pyrrole-2-carboxylic acid

Using the compound produced in Example 6(6), the same procedure as in Example 13 was carried out to obtain the title compound having the following physical property values.
LCMS (A) Retention time (min): 0.88;
MS (ESI, Neg.): 353 (M-H)⁻.
¹H-NMR (CD₃OD): δ 1.26, 1.81-1.99, 2.10, 2.12-2.37, 2.92-3.05, 3.62-3.78, 3.98, 5.72, 6.81, 7.03.

### Example 18: (ξ)-3-[1-(Cyclobutylamino)ethyl]-1-(4-methylbenzyl)-1H-indole-2-carboxylic acid

The compound (21 mg) produced in Example 5 was subjected to optical resolution using SFC (column used: Daicel CHIRALPAK IC (10 mm × 250 mm) manufactured by Daicel Corporation, mobile phase: CO₂: (0.1% diethylamine/ethanol) = 8 : 2, flow rate: 30 mL/min, pressure: 100 bar, wavelength: 220 nm, column temperature: 35°C). A second peak (retention time: about 15.00 minutes) under the above optical resolution conditions was obtained as the title compound (8 mg) having the following physical property values.
SFC Retention time (min): 15.00;
MS (ESI, Pos.): 363 (M+H)⁺.
¹H-NMR (CD₃OD): δ 1.61-1.92, 1.95-2.12, 2.15-2.27, 3.46-3.59, 4.74, 5.69, 6.10, 6.94-7.04, 7.13, 7.24, 7.43, 7.64.

### Reference Example 53: Ethyl 3-({[(benzyloxy)carbonyl](cyclobutyl)amino}methyl)-1-(4-methylbenzyl)-1H-indole-2-carboxylate

To a solution of the compound (4.2 g) produced in Reference Example 4 in acetonitrile (25 mL), benzyl chloroformate (2.3 g) and diisopropylethylamine (3.9 mL) were added thereto, and the mixture was stirred at room temperature for 4 hours. Ethyl acetate and a saturated aqueous ammonium chloride solution were added to the reaction mixture, and the mixture was extracted. The organic layer was washed with saturated saline solution, dried over sodium sulfate, and then concentrated under reduced pressure to obtain the title compound having the following physical property values. The obtained title compound was used as it was for the next reaction.
LCMS (B) Retention time (min): 1.49;
MS (ESI, Pos.): 511 (M+H)⁺.

### Reference Example 54: 3-({[(Benzyloxy)carbonyl](cyclobutyl)amino}methyl)-1-(4-methylbenzyl)-1H-indole-2-carboxylic acid

Using the compound produced in Reference Example 53, the same procedure as in Example 2 was carried out to obtain the title compound (4.6 g) having the following physical property values.
LCMS (A) Retention time (min): 1.36;
MS (ESI, Pos.): 483 (M+H)⁺.

### Reference Example 55: Benzyl cyclobutyl({1-(4-methylbenzyl)-2-[(methylsulfonyl)carbamoyl]-1H-indol-3-yl}methyl)carbamate

To a solution of the compound (60 mg) produced in Reference Example 54 in THF (1 mL), 1,1'-carbonyldiimidazole (30 mg) was added, and the mixture was stirred at 60°C for 3 hours. The reaction mixture was cooled to room temperature, then 1,8-diazabicyclo[5.4.0]undec-7-ene (47 mg) and methanesulfonamide (18 mg) were added thereto, and the mixture was stirred at 55°C for 2 hours. The reaction mixture was concentrated under reduced pressure, and the resulting residue was purified by HPLC (column: YMC Triart C18 50 mm × 100 mm, 5 µm; flow rate: 100 mL/min; mobile phase A: 0.1% aqueous TFA solution; mobile phase B: 0.1% TFA acetonitrile solution: gradient (ratios of mobile phase (A) : mobile phase (B) are described): 0 to 0.50 min: (90% : 10%); 0.50 to 12.00 min: (90% : 10%) to (0% : 100%); 12.01 to 18.00 min: (0% : 100%)) to obtain the title compound (44 mg) having the following physical property values.
LCMS (A) Retention time (min): 1.39;
MS (ESI, Pos.): 560 (M+H)⁺.

### Example 19: 3-[(Cyclobutylamino)methyl]-1-(4-methylbenzyl)-N-(methylsulfonyl)-1H-indole-2-carboxamide

To a solution of the compound (22 mg) produced in Reference Example 55 in methanol (1.5 mL), 10% palladium-activated carbon (50% wet, 25 mg) and ammonium formate (25 mg) were added, and the mixture was stirred at 50°C for 4 hours. The reaction mixture was filtered through Celite (trade name), then the filtrate was concentrated, and the resulting residue was purified by HPLC (column: YMC Triart C18 50 mm ×100 mm, 5 µm; flow rate: 100 mL/min; mobile phase A: 0.1% aqueous TFA solution; mobile phase B: 0.1% TFA acetonitrile solution: gradient (ratios of mobile phase (A) : mobile phase (B) are described): 0 to 0.50 min: (90% : 10%); 0.50 to 12.00 min: (90% : 10%) to (0% : 100%); 12.01 to 18.00 min: (0% : 100%)) to obtain the title compound (9.6 mg) having the following physical property values.
LCMS (B) Retention time (min): 1.03;
MS (ESI, Pos.): 426 (M+H)⁺.
¹H-NMR (DMSO-d₆): δ 1.69-1.85, 2.10-2.36, 3.00, 3.67-3.80, 4.26, 5.87, 7.01-7.08, 7.14, 7.23, 7.50, 7.79, 9.98.

### Example 19(1) to Example 19(6)

The same procedure as in Reference Example 55 to Example 19 was carried out by using a corresponding sulfonamide compound in place of methanesulfonamide to obtain the following compounds.

### Example 19(1): 3-[(Cyclobutylamino)methyl]-N-(cyclopropylsulfonyl)-1-(4-methylbenzyl)-1H-indole-2-carboxamide

LCMS (B) Retention time (min): 1.06;
MS (ESI, Pos.): 452 (M+H)⁺.
¹H-NMR (DMSO-d₆): δ 0.79-0.97, 1.68-1.83, 2.08-2.30, 2.90-3.013.63-3.70, 4.23, 5.89, 7.01-7.10, 7.14, 7.22, 7.51, 7.77, 10.02.

### Example 19(2): 3-((Cyclobutylamino-(4-methylbenzyl)-N-(4-pyridinylsulfonyl)-1H-indole-2-carboxamide

LCMS (B) Retention time (min): 1.03
MS (ESI, Pos.): 489 (M+H)⁺.
¹H-NMR (DMSO-d₆): δ 1.74-1.89, 2.14-2.37, 3.76-3.89, 4.33, 5.78, 6.88, 6.97, 7.15, 7.24, 7.52, 7.76-7.81, 9.70.

### Example 19(3): 3-[(Cyclobutylamino)methyl]-N-(dimethylsulfamoyl)-1-(4-methylbenzyl)-1H-indole-2-carboxamide

LCMS (B) Retention time (min): 1.06
MS (ESI, Pos.): 455 (M+H)⁺.
¹H-NMR (DMSO-d₆): δ 1.69-1.85, 2.09-2.30, 2.64, 3.64-3.75, 4.25, 5.92, 7.02, 7.05, 7.14, 7.22, 7.48, 7.78, 10.13.

### Example 19(4): 3-[(Cyclobutylamino)methyl]-1-(4-methylbenzyl)-N-(1,3-thiazol-2-ylsulfonyl)-1H-indole-2-carboxamide

LCMS (B) Retention time (min): 1.09
MS (ESI, Pos.): 495 (M+H)⁺.
¹H-NMR (DMSO-d₆): δ 1.73-1.86, 2.15-2.24, 2.29-2.40, 3.78-3.87, 4.36, 5.78, 6.96, 7.01, 7.16, 7.24, 7.51, 7.80, 7.91, 7.95, 9.62.

### Example 19(5): 3-[(Cyclobutylamino-1-(4-methylbenzyl)-N-(4-morpholinylsulfonyl)-1H-indole-2-carboxamide

LCMS (B) Retention time (min): 1.05
MS (ESI, Pos.): 497 (M+H)⁺.
¹H-NMR (DMSO-d₆): δ 1.69-1.84, 2.10-2.28, 3.00-3.11, 3.51-3.59, 3.64-3.77, 4.26, 5.92, 7.01, 7.04, 7.14, 7.23, 7.50, 7.79, 9.98.

### Example 19(6): 3-[(Cyclobutylamino)methyl]-1-(4-methylbenzyl)-N-(2-thienylsulfonyl)-1H-indole-2-carboxamide

LCMS (B) Retention time (min): 1.13
MS (ESI, Pos.): 494 (M+H)⁺.
¹H-NMR (DMSO-d₆): δ 1.72-1.89, 2.13-2.38, 3.72-3.85, 4.27, 5.83, 6.96, 6.99, 7.07, 7.14, 7.23, 7.52, 7.60, 7.73, 7.78, 9.84.

### Reference Example 56: Benzyl {[2-carbamoyl-1-(4-methylbenzyl)-1H-indol-3-yl]methyl}cyclobutylcarbamate

To a solution of the compound (800 mg) produced in Reference Example 54 in THF (5 mL), 1-chloro-N,N,2-trimethylpropenylamine (886 mg) was added, and the mixture was stirred at room temperature for 1 hour. Ammonia (0.5 mol/L 1,4-dioxane solution, 30 mL) was added to the reaction mixture, and the mixture was stirred at room temperature for 1 hour, and then methanol was added thereto. The reaction mixture was concentrated under reduced pressure, and the resulting residue was purified by silica gel column chromatography (hexane : ethyl acetate = 9 : 1 → 4 : 1 → 2 : 1) to obtain the title compound (773 mg) having the following physical property values.
LCMS (B) Retention time (min): 1.31;
MS (ESI, Pos.): 482 (M+H)⁺.
¹H-NMR (DMSO-d₆): δ 1.35-1.53, 1.82-1.94, 2.00-2.14, 2.20, 3.91-4.03, 4.87, 5.16, 5.51, 6.93, 6.98, 7.03, 7.15, 7.29-7.47, 7.86, 8.08.

### Example 20: 3-[(Cyclobutylamino)methyl]-1-(4-methylbenzyl)-1H-indole-2-carboxamide

Using the compound (20 mg) produced in Reference Example 56, the same procedure as in Example 19 was carried out to obtain the title compound (0.9 mg) having the following physical property values.
LCMS (B) Retention time (min): 0.90
MS (ESI, Pos.): 348 (M+H)⁺.
¹H-NMR (DMSO-d₆): δ 1.49-1.69, 1.72-1.83, 2.03-2.12, 2.22, 3.10-3.20, 3.88, 5.79, 6.98, 7.05, 7.10, 7.20, 7.46, 7.60, 7.69, 9.94.

### Example 20(1): 3-[(Cyclobutylamino)methyl]-N-methoxy-1-(4-methylbenzyl)-1H-indole-2-carboxamide

The same procedure as in Reference Example 56 to Example 20 was carried out by using a corresponding amino compound in place of ammonia to obtain the title compound having the following physical property.
LCMS (B) Retention time (min): 0.89
MS (ESI, Pos.): 378 (M+H)⁺.
¹H-NMR (DMSO-d₆): δ 1.54-1.75, 1.79-1.94, 2.03-2.17, 2.22, 3.20-3.31, 3.91, 5.74, 7.00, 7.06, 7.12, 7.23, 7.50, 7.71, 8.17.

### Reference Example 57: Benzyl {[2-cyano-1-(4-methylbenzyl)-1H-indol-3-yl]methyl}cyclobutylcarbamate

To a solution of the compound (700 mg) produced in Reference Example 56 in DCM (6 mL), pyridine (0.94 mL) and trifluoroacetic anhydride (0.81 mL) were added, and the mixture was stirred at room temperature for 2 hours. Ethyl acetate and 0.5 N hydrochloric acid were added to the reaction mixture, and the mixture was extracted. The organic layer was washed with 0.5 N hydrochloric acid and saturated saline solution and dried over sodium sulfate, and then concentrated under reduced pressure, and the resulting residue was purified by silica gel column chromatography (hexane : ethyl acetate = 9 : 1 → 4 : 1) to obtain the title compound (495 mg) having the following physical property values.
LCMS (B) Retention time (min): 1.43;
MS (ESI, Pos.): 464 (M+H)⁺.
¹H-NMR (CDCl₃): δ 1.48-1.65, 2.04-2.22, 2.29, 4.23-4.40, 4.91, 5.22, 5.38, 7.01, 7.05-7.14, 7.25-7.42, 7.56-7.68.

### Reference Example 58: Benzyl cyclobutyl{[1-(4-methylbenzyl)-2-(1H-tetrazol-5-yl)-1H-indol-3-yl]methyl}carbamate

To a solution of the compound (235 mg) produced in Reference Example 57 in DMF (2.5 mL), ammonium chloride (136 mg) and sodium azide (165 mg) were added, and the mixture was stirred at 120°C for 2 hours using microwaves. Ethyl acetate and 0.5 N hydrochloric acid were added to the reaction mixture, and the mixture was extracted. The organic layer was washed with saturated saline solution, dried over sodium sulfate, and then concentrated under reduced pressure to obtain the title compound (250 mg) having the following physical property values.
LCMS (B) Retention time (min): 1.43;
MS (ESI, Pos.): 507 (M+H)⁺.

### Example 21: N-{[1-(4-Methylbenzyl)-2-(1H-tetrazol-5-yl)-1H-indol-3-yl]methyl}cyclobutanamine

Using the compound (50 mg) produced in Reference Example 58, the same procedure as in Example 19 was carried out to obtain the title compound (12 mg) having the following physical property values.
LCMS (B) Retention time (min): 1.01
MS (ESI, Pos.): 373 (M+H)⁺.
¹H-NMR (DMSO-d₆): δ 1.72-1.91, 2.14-2.36, 3.74-3.86, 4.37, 6.12, 6.94, 6.99, 7.11-7.20, 7.45, 7.76, 9.69.

### Reference Example 59: Benzyl cyclobutyl{[2-(N'-hydroxycarbamimidoyl)-1-(4-methylbenzyl)-1H-indol-3-yl]methyl}carbamate

To a solution of the compound (105 mg) produced in Reference Example 57 in ethanol (1 mL), hydroxylamine hydrochloride (157 mg) and triethylamine (0.32 mL) were added, and the mixture was stirred at 75°C for 5 hours. The reaction mixture was cooled to room temperature, then ethyl acetate and saturated saline solution were added, and the mixture was extracted. The organic layer was dried over sodium sulfate, and then concentrated under reduced pressure, and the resulting residue was purified by silica gel column chromatography (hexane : ethyl acetate = 9 : 1→- 4 : 1 → 2 : 1) to obtain the title compound (16 mg) having the following physical property values.
LCMS (B) Retention time (min): 1.12;
MS (ESI, Pos.): 519 (M+Na)⁺.

### Reference Example 60: Benzyl cyclobutyl{[1-(4-methylbenzyl)-2-(5-oxo-4,5-dihydro-1,2,4-oxadiazol-3-yl)-1H-indol-3-yl]methyl}carbamate

To a solution of the compound (16 mg) produced in Reference Example 59 in DMSO (0.2 mL) and 1,4-dioxane (0.3 mL), 1,1'-carbonyldiimidazole (7.7 mg) and 1,8-diazabicyclo[5.4.0]undec-7-ene (12 mg) were added, and the mixture was stirred at 80°C for 1 hour. The reaction mixture was cooled to room temperature, then ethyl acetate and a saturated aqueous ammonium chloride solution were added thereto, and the mixture was extracted. The organic layer was dried over sodium sulfate, and then concentrated under reduced pressure to obtain the title compound having the following physical property values. The obtained title compound was used as it was for the next reaction.
LCMS (B) Retention time (min): 1.35;
MS (ESI, Pos.): 545 (M+Na)⁺.

### Example 22: 3-{3-[(Cyclobutylamino)methyl]-1-(4-methylbenzyl)-1H-indol-2-yl}-1,2,4-oxadiazol-5(4H)-one

Using the compound produced in Reference Example 60, the same procedure as in Example 19 was carried out to obtain the title compound (5.4 mg) having the following physical property values.
LCMS (B) Retention time (min): 1.01
MS (ESI, Pos.): 389 (M+H)⁺.
¹H-NMR (DMSO-d₆): δ 1.70-1.92, 2.11-2.28, 3.65-3.75, 4.33, 5.89, 6.96, 7.04, 7.15, 7.22, 7.46, 7.78.

### Reference Example 61: 2-Chloro-1-(4-methylbenzyl)-1H-indole-3-carbaldehyde

To a solution of 2-chloro-1H-indole-3-carbaldehyde (500 mg) in DMF (5 mL), potassium carbonate (770 mg) and 1-(bromomethyl)-4-methylbenzene (541 mg) were added, and the mixture was stirred at room temperature for 1 hour. Water was added to the reaction mixture, and the obtained solid was filtered and dried to obtain the title compound having the following physical property values. The obtained title compound was used as it was for the next reaction.
LCMS (B) Retention time (min): 1.27;
MS (ESI, Pos.): 284 (M+H)⁺.

### Reference Example 62: 2-Mercapto-1-( 4-methylbenzyl)-1H-indole-3-carbaldehyde

To a solution of the compound produced in Reference Example 61 in methanol (12 mL), an aqueous sodium hydrogen sulfide (334 mg) solution (3 mL) was added, and the mixture was stirred at room temperature for 5 hours. DMF (10 mL) was added to the reaction mixture, and the mixture was stirred at room temperature overnight, then sodium hydrogen sulfide (334 mg) was added thereto. The reaction mixture was stirred at 50°C for 30 minutes and cooled to room temperature, then ethyl acetate, water, and hydrochloric acid were added thereto, and the mixture was extracted. The organic layer was washed with saturated saline solution, dried over sodium sulfate, and then concentrated under reduced pressure to obtain the title compound having the following physical property values. The obtained title compound was used as it was for the next reaction.
LCMS (B) Retention time (min): 1.32;
MS (ESI, Pos.): 282 (M+H)⁺.

### Reference Example 63: 3-Formyl-1-(4-methylbenzyl)-1H-indole-2-sulfonic acid

To a solution of the compound produced in Reference Example 62 in acetonitrile (20 mL), acetic acid (7.5 mL) and 30% aqueous hydrogen peroxide (3.5 mL) were added, and the mixture was stirred at room temperature overnight. Ethyl acetate and water were added to the reaction mixture, and the mixture was filtered through Celite (trade name) and then extracted. The organic layer was dried over sodium sulfate, and then concentrated under reduced pressure. The resulting residue was purified by HPLC (column: YMC Triart C18 50 mm × 100 mm, 5 µm; flow rate: 100 mL/min; mobile phase A: 0.1% aqueous TFA solution; mobile phase B: 0.1% TFA acetonitrile solution: gradient (ratios of mobile phase (A) : mobile phase (B) are described): 0 to 0.50 min: (70% : 30%); 0.50 to 12.00 min: (70% : 30%) to (40% : 60%); 12.01 to 18.00 min: (0% : 100%)) to obtain the title compound (22 mg) having the following physical property values.
LCMS (B) Retention time (min): 0.92;
MS (ESI, Pos.): 330 (M+H)⁺.

### Example 23: 3-[(Cyclobutylamino)methyl]-1-(4-methylbenzyl)-1H-indole-2-sulfonic acid

Using the compound (5 mg) produced in Reference Example 63, the same procedure as in Reference Example 4 was carried out to obtain the title compound (0.6 mg) having the following physical property values.
LCMS (B) Retention time (min): 0.99
MS (ESI, Pos.): 385 (M+H)⁺.
¹H-NMR (DMSO-d₆): δ 1.68-1.83, 2.12-2.27, 3.69-3.79, 4.40, 5.74, 7.04, 7.09-7.18, 7.72, 8.50.

### Biological Example 1: LPA3 Agonist activity measurement experiment using lysophosphatidic acid receptor subtype expressing cells

### [Operation]

(Method A) Using RH7777 cells derived from rat hepatocellular carcinoma as a host, each cell line (Multispan, Inc., Cat No. C1048-6, C1050-6, and C1053-6) stably expressing a human LPA3 receptor was subjected to an experiment. The cells cultured until reaching subconfluence were detached, and suspended in a medium (DMEM containing 10% FBS, 1% penicillin/streptomycin, 30 µg/mL puromycin) so as to be 4 × 10⁵ cells/mL. The cells were seeded in a 96 well optical bottom plate (Asahi Techno Glass, MT4940-010) at 100 µL/well, and cultured under conditions of 5% CO₂ and 37°C for 1 day.

On the day of measurement, the medium was removed, and a medium containing 0.2% FBS was added at 100 µL/well, and cultured for 4 to 6 hours. Thereafter, the medium was removed, and an assay buffer (Hank's Balanced Salt Solution containing 0.02 mol/L HEPES, 2.5 mmol/L probenecid, 0.1% BSA, 5 µmοl/L Fluo-4, Quenching Buffer, Pluronic F-127) was added at 100 µL/well, and the cells were incubated with 5% CO₂ at 37°C for about 1 hour. The plate was set in a fluorescent drug screening system (Hamamatsu Photonics K.K.), and after 30 seconds from the start of measurement, 25 µL of a cell-added solution containing only a test substance or dimethyl sulfoxide (hereinafter, it is abbreviated as DMSO.) was added thereto. After 3 minutes, 25 µL of a ligand was further added thereto, and then measurement was performed for 4 minutes. A fluorescence wavelength of 540 nm at an excitation wavelength of 480 nm was measured, and intracellular Ca²⁺ concentration was indicated by fluorescence intensity change.

Agonist action of the test substance was calculated as a ratio increase degree after addition of the test substance when a ratio increase value indicated by cells to which a cell-added solution containing only DMSO was added at the time of ligand addition was set to 100%. Further, EC₅₀ value was calculated as a 50% action concentration using Microsoft Excel, from an activity (%) at concentrations before and after agonist activity exceeds 50%.

(Method B) A cell line stably expressing a human LPA3 receptor using a cell obtained by knocking down an LPA1 receptor of CHO cells of Chinese hamster ovary as a host was subjected to an experiment. The cryopreserved cells were thawed and suspended in loading buffer (Hank's Balanced Salt Solution containing 0.02 mol/L HEPES, 2.5 mmol/L probenecid, 0.1% BSA, 5 µmol/L Fluo-4, Quenching Buffer, Pluronic F-127) so as to be 1 × 10⁵ cells/mL. The plate was allowed to stand under conditions of 5% CO₂ and 37°C for 1 hour, then the buffer was replaced with an assay buffer (Hank's Balanced Salt Solution containing 0.02 mol/L HEPES, 2.5 mmol/L probenecid, 0.1% BSA), and the cells were seeded in a 384 well optical bottom plate (384 Well microplate, low volume, Corning, 3540) at 10 µL/well.

A plate was set in a fluorescent drug screening system (Hamamatsu Photonics K.K.), and after 15 seconds from the start of the measurement, 10 µL of a positive control, the test substance or the cell-added solution containing only DMSO was added thereto, and the measurement was performed for 1 minute. A fluorescence wavelength of 540 nm at an excitation wavelength of 480 nm was measured, and intracellular Ca²⁺ concentration was indicated by fluorescence intensity change.

Agonist action of the test substance was calculated as a ratio increase degree after addition of the test substance when a ratio increase value indicated by the positive control or the cell-added solution containing was added was set to 100%. Further, EC₅₀ value was calculated as a 50% action concentration using Microsoft Excel, from an activity (%) at concentrations before and after agonist activity exceeds 50%.

### [Results]

The compound of the present invention showed an EC₅₀ value of 1 µmol/L or less in the LPA3 agonist activity measurement experiment of Method A or Method B described above, and showed potent LPA3 agonist activity.

For example, the EC₅₀ values were as follows: the compound produced in Example 1(3) showed 0.5 µmol/L (Method A) or 0.2 µmol/L (Method B), the compound produced in Example 1(4) showed 0.6 µmol/L (Method A) or 0.6 µmol/L (Method B), the compound produced in Example 2 showed 0.06 µmol/L (Method A), the compound produced in Example 2(6) showed 0.05 µmol/L (Method B), the compound produced in Example 2(8) showed 0.2 µmol/L (Method A), the compound produced in Example 2(18) showed 1.0 µmol/L (Method A), the compound produced in Example 2(34) showed 0.04 µmol/L (Method A), the compound produced in Example 2(41) showed 0.04 µmol/L (Method B), the compound produced in Example 2(46) showed 0.1 µmol/L (Method B), the compound produced in Example 2(49) showed 0.1 µmol/L (Method B), the compound produced in Example 2(53) showed 0.1 µmol/L (Method B), the compound produced in Example 2(60) showed 0.03 µmol/L (Method B), the compound produced in Example 2(70) showed 0.2 µmol/L (Method B), the compound produced in Example 2(71) showed 0.06 µmol/L (Method B), the compound produced in Example 2(72) showed 0.1 µmol/L (Method B), the compound produced in Example 2(74) showed 0.03 µmol/L (Method B), the compound produced in Example 2(75) showed 0.06 µmol/L (Method B), the compound produced in Example 5 showed 0.1 µmol/L (Method A), the compound produced in Example 6(2) showed 0.9 µmol/L (Method A), the compound produced in Example 6(7) showed 1.0 µmol/L (Method A), the compound produced in Example 7 showed 0.1 µmol/L (Method A), the compound produced in Example 9(1) showed 0.2 µmol/L (Method B), the compound produced in Example 19 showed 0.06 µmol/L (Method B), and the compound produced in Example 21 showed 0.04 µmol/L (Method B).

### Preparation Example 1:

Tablet containing 5 mg of 3-[(cyclobutylamino)methyl]-1-(3-cyclopropylbenzyl)-1H-indole-2-carboxylic acid

The following ingredients are mixed in a conventional manner and compressed to give 10,000 tablets each containing 5 mg of an active ingredient.
- 3-[(Cyclobutylamino)methyl]-1-(3-cyclopropylbenzyl)-1H-indole-2-carboxylic acid: 50 g
- Carboxymethyl cellulose calcium (disintegrant): 20 g
- Magnesium stearate (lubricant): 10 g
- Microcrystalline cellulose: 920 g

### Preparation Example 2:

Tablet containing 5 mg of 3-[(cyclobutylamino)methyl]-1-(4-methylbenzyl)-1H-indole-2-carboxylic acid

The following ingredients are mixed in a conventional manner and compressed to give 10,000 tablets each containing 5 mg of an active ingredient.
- 3-[(Cyclobutylamino)methyl]-1-(4-methylbenzyl)-1H-indole-2-carboxylic acid: 50 g
- Carboxymethyl cellulose calcium (disintegrant): 20 g
- Magnesium stearate (lubricant): 10 g
- Microcrystalline cellulose: 920 g

### Preparation Example 3:

### Injection containing 20 mg of 3-[(cyclobutylamino)methyl]-1-(4-methylbenzyl)-1H-pyrrolo[2,3-b]pyridine-2-carboxylic acid

After mixing the following ingredients in a conventional manner, the solution is sterilized in a conventional manner, and 5 mL aliquots are charged into ampules and lyophilized in a conventional manner to give 10,000 ampules each containing 20 mg of an active ingredient.
▪ 3-[(Cyclobutylamino)methyl]-1-(4-methylbenzyl)-1H-pyrrolo[2,3-b]pyridine-2-carboxylic acid: 200 g
▪ Mannitol: 20 g
▪ Distilled water: 50 L

### INDUSTRIAL APPLICABILITY

Since the compound of the present invention has an agonist activity for LPA3, a pharmaceutical containing the compound as an active ingredient is useful as a preventive and/or therapeutic agent for essential thrombocythemia, reactive thrombocytosis, and the like.

## Claims

1. A compound represented by general formula (IY): 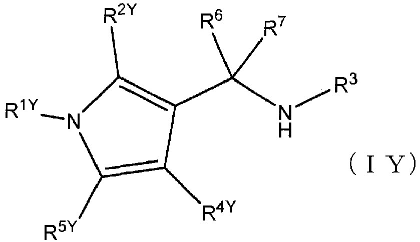 wherein R^{1Y} represents a C1 to 4 alkyl group or -CR⁸R⁹-ring 1, R⁸ and R⁹ each independently represent a hydrogen atom or a C1 to 4 alkyl group, ring 1 represents a 3 to 15-membered carbocyclic ring which may be substituted with 1 to 5 R^{13Y}s or a 3 to 15-membered heterocyclic ring which may be substituted with 1 to 5 R^{13Y}s, R^{13Y} represents halogen, a C1 to 4 alkyl group, a C1 to 4 alkoxy group, a C1 to 4 haloalkyl group, a C1 to 4 haloalkoxy group, a C3 to 6 cycloalkyl group, a hydroxyl group, a carboxyl group, an amino group, a (C1 to 4 alkyl)carbonylamino group, a (C1 to 4 alkyl)carbonyl group, a carbamoyl group, a (C1 to 4 alkyl)aminocarbonyl group, a di(C1 to 4 alkyl)aminocarbonyl group, a cyano group, a C1 to 4 alkylsulfonyl group, a C1 to 4 alkylthio group, a C1 to 4 haloalkylthio group, a nitro group, a 5 to 6-membered heterocyclic ring, or -O-(CH₂)r_{Y}-R¹⁷, R¹⁷ represents a carboxyl group, a C1 to 4 alkoxycarbonyl group, -CONHSO₂R^{10Y}, -CONHR¹⁹, a sulfo group (-SO₃H group), - SO₂NHCOR²⁰, a 5-membered ring having highly acidic hydrogen, a cyano group, a di-(C1 to 4 alkyl)-hydroxymethyl group, a 3 to 15-membered carbocyclic ring which may be substituted with 1 to 3 R¹⁸s, or a 3 to 15-membered heterocyclic ring which may be substituted with 1 to 3 R¹⁸s, R¹⁸ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, an amino group, a C1 to 4 alkoxycarbonyl group, or an oxo group, a plurality of R^{13Y}s may be the same or different, r_{Y} represents an integer of 1 to 5, a plurality of R¹⁸s may be the same or different, R^{2Y} represents a carboxyl group, a C1 to 4 alkoxycarbonyl group, -CONHSO₂R^{10Y}, -CONHR¹⁹, a sulfo group (-SO₃H group), -SO₂NHCOR²⁰, or a 5-membered ring having highly acidic hydrogen, R^{10Y} represents a C1 to 4 alkyl group, a C1 to 4 haloalkyl group, a C3 to 6 cycloalkyl group, a di-(C1 to 4 alkyl)amino group, a 5 to 6-membered carbocyclic ring which may be substituted with 1 to 3 R¹⁶s, or a 5 to 6-membered heterocyclic ring which may be substituted with 1 to 3 R¹⁶s, and R¹⁶ represents halogen, a C1 to 4 alkyl group, or a C1 to 4 alkoxy group, a plurality of R¹⁶s may be the same or different, R¹⁹ represents a hydrogen atom, a hydroxyl group, a C1 to 4 alkyl group, or a C1 to 4 alkoxy group, R²⁰ represents a C1 to 4 alkyl group, a C1 to 4 haloalkyl group, a C3 to 6 cycloalkyl group, a di-(C 1 to 4 alkyl)amino group, a 5 to 6-membered carbocyclic ring which may be substituted with 1 to 3 R²¹s, or a 5 to 6-membered heterocyclic ring which may be substituted with 1 to 3 R²¹s, R²¹ represents halogen, a C1 to 4 alkyl group, or a C1 to 4 alkoxy group, a plurality of R²¹s may be the same or different, R³ represents a 3 to 5-membered saturated carbocyclic ring which may be substituted with 1 to 5 R¹¹s or a 3 to 5-membered saturated heterocyclic ring which may be substituted with 1 to 5 R¹¹s, R¹¹ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, or an amino group, a plurality of R¹¹s may be the same or different, and two R¹¹s may be taken together to form C1 to 3 alkylene, and R^{4Y} and R^{5Y} each independently represent halogen, a C1 to 4 alkyl group, a C1 to 4 haloalkyl group, a C3 to 6 cycloalkyl group, a C2 to 4 alkenyl group, or a 5 to 6-membered carbocyclic ring which may be substituted with 1 to 3 R¹⁵s or a 5 to 6-membered heterocyclic ring which may be substituted with 1 to 3 R¹⁵s, R¹⁵ represents halogen, a C1 to 4 alkyl group, or a C1 to 4 alkoxy group, a plurality of R¹⁵s may be the same or different, and carbon atoms at position 4 and position 5 of the pyrrole ring, R^{4Y}, and R^{5Y} may be taken together to form a 5 to 6-membered carbocyclic ring which may be substituted with 1 to 3 R¹²s, or a 5 to 6-membered heterocyclic ring which may be substituted with 1 to 3 R¹²s, R¹² represents halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, a plurality of R¹²s may be the same or different, and R⁶ and R⁷ each independently represent a hydrogen atom, a C1 to 4 alkyl group, or a C1 to 4 haloalkyl group, and herein, each hydrogen atom may be a deuterium atom or a tritium atom; provided, however, 3-[(cyclopentylamino)methyl]-1-[(3-methylphenyl)methyl]-1H-indole-2-carboxylic acid, 3-[(cyclopentylamino)methyl] -1 - [(2-fluorophenyl)methyl] -6-methyl-1H-indole-2-carboxylic acid, 3-[(cyclopentylamino)methyl]-6-methoxy-1-[(4-methylphenyl)methyl]-1H-indole-2-carboxylic acid, 3-[(cyclopentylamino)methyl]-1-ethyl-6-methoxy-1H-indole-2-carboxylic acid, 1-[(2-chloro-4-fluorophenyl)methyl]-3-[(cyclopentylamino)methyl]-6-methoxy-1H-indole-2-carboxylic acid, 1-[(2-chloro-4-fluorophenyl)methyl]-3-[(cyclopropylamino)methyl]-6-methoxy-1H-indole-2-carboxylic acid, 3-[(cyclopentylamino)methyl]-6-methoxy-l-[(4-methoxyphenyl)methyl]-1H-indole-2-carboxylic acid, 3-[(cyclopropylamino)methyl]-6-methoxy-1-[(4-methoxyphenyl)methyl]-1H-indole-2-carboxylic acid, 3-[(cyclopentylamino)methyl]-1-[(4-fluorophenyl)methyl]-6-methoxy-1H-indole-2-carboxylic acid, 3-[(cyclopentylammo)methyl]-6-methoxy-1-methyl-1H-indole-2-carboxylic acid, 3-[(cyclopentylamino)methyl]-1-[(4-methoxyphenyl)methyl]-1H-indole-2-carboxylic acid, 3-[(cyclopentylamino)methyl]-1-ethyl-6-methyl-1H-indole-2-carboxylic acid, 3-[(cyclopentylamino)methyl]-6-methoxy-1-[(2-methylphenyl)methyl]-1H-indole-2-carboxylic acid, 3-[(cyclopentylamino)methyl]-6-methyl-1-[(4-methylphenyl)methyl]-1H-indole-2-carboxylic acid, 3-[(cyclopropylamino)methyl]-6-methyl-1-[(4-methylphenyl)methyl]-1H-indole-2-carboxylic acid, 3-[(cyclopentylamino)methyl]-1-[(2-fluorophenyl)methyl]-6-methoxy-1H-indole-2-carboxylic acid, 3-[(cyclopentylamino)methyl]-1-[(4-fluorophenyl)methyl]-6-methyl-1H-indole-2-carboxylic acid, 3 - [(cyclopropylamino)methyl] -1 - [(4-fluorophenyl)methyl] -6-methyl-1H-indole-2-carboxylic acid, 3 - [(cyclopentylamino)methyl] -1 - [(3 -fluorophenyl)methyl] -6-methoxy-1H-indole-2-carboxylic acid, 3-[(cyclopentylamino)methyl]-6-methyl-1-[(2-methylphenyl)methyl]-1H-indole-2-carboxylic acid, 3 - [(cyclopropylamino)methyl] -6-methyl-1-[(2-methylphenyl)methyl]-1H-indole-2-carboxylic acid, 3-[(cyclopentylamino)methyl]-1 - [(3-fluorophenyl)methyl]-6-methyl-1H-indole-2-carboxylic acid, 3-[(cyclopropylamino)methyl]-1-[(3-fluorophenyl)methyl]-6-methyl-1H-indole-2-carboxylic acid, 3-[(cyclopentylamino)methyl]-1-[(4-methoxyphenyl)methyl]-6-methyl-1H-indole-2-carboxylicacid, 3-[(cyclopentylamino)methyl]-1-[(4-ethenylphenyl)methyl]-6-methyl-1H-indole-2-carboxylic acid, 3-[(cyclopentylamino)methyl]-1-methyl-1H-indole-2-carboxylic acid, 3-[(cyclopentylamino)methyl]-1-(phenylmethyl)-1H-indole-2-carboxylic acid, 3-[(cyclopentylamino)methyl]-1-[(2-methylphenyl)methyl]-1H-indole-2-carboxylic acid, 3-[(cyclopentylamino)methyl]-1-[(4-methylphenyl)methyl]-1H-indole-2-carboxylic acid, 3-[(cyclopentylamino)methyl]-1-[(4-fluorophenyl)methyl]-1H-indole-2-carboxylic acid, 3-[(cyclopentylamino)methyl]-1-[(2,5-dimethylphenyl)methyl]-1H-indole-2-carboxylic acid, 3-[(cyclopentylamino)methyl]-1-[(4-ethenylphenyl)methyl]-1H-indole-2-carboxylic acid, 3-[(cyclopropylamino)methyl]-1-(phenylmethyl)-1H-indole-2-carboxylic acid, 3-[(cyclopropylamino)methyl]-1-[(2-methylphenyl)methyl]-1H-indole-2-carboxylic acid, 3-[(cyclopropylamino)methyl]-1-[(3-methylphenyl)methyl]-1H-indole-2-carboxylic acid, 3-[(cyclopropylamino)methyl]-1-[(4-methylphenyl)methyl]-1H-indole-2-carboxylic acid, 3-[(cyclopropylamino)methyl]-1-[(4-ethenylphenyl)methyl]-1H-indole-2-carboxylic acid, 1-[(2-chlorophenyl)methyl]-3-[(cyclopentylamino)methyl]-1H-indole-2-carboxylic acid, 1-[(2-chlorophenyl)methyl]-3-[(cyclopropylamino)methyl]-1H-indole-2-carboxylic acid, 1-[(3-chlorophenyl)methyl]-3-[(cyclopentylamino)methyl]-1H-indole-2-carboxylic acid, 1-[(4-chlorophenyl)methyl]-3-[(cyclopentylamino)methyl]-1H-indole-2-carboxylic acid, 1-[(2-chloro-4-fluorophenyl)methyl]-3-[(cyclopentylamino)methyl]-1H-indole-2-carboxylic acid, 1-[(2-chloro-4-fluorophenyl)methyl]-3-[(cyclopropylamino)methyl]-1H-indole-2 carboxylic acid, and 3-[(cyclopentylamino)methyl]-1,6-dimethyl-1H-indole-2-carboxylic acid are excluded, or a salt thereof.

2. The compound according to claim 1, represented by general formula (I): 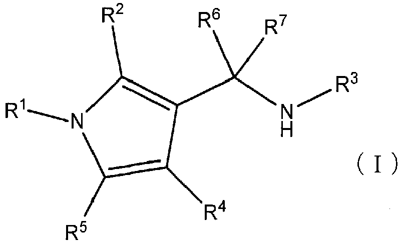 wherein R¹ represents a C1 to 4 alkyl group or -CR⁸R⁹-ring 1, R⁸ and R⁹ each independently represent a hydrogen atom or a C1 to 4 alkyl group, ring 1 represents a 3 to 15-membered carbocyclic ring which may be substituted with 1 to 5 R¹³s or a 3 to 15-membered heterocyclic ring which may be substituted with 1 to 5 R¹³s, R¹³ represents halogen, a C1 to 4 alkyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or - O-(CH₂)r-COOH group, a plurality of R¹³s may be the same or different, r represents an integer of 2 to 5, R² represents a carboxyl group, a C1 to 4 alkoxycarbonyl group, or - CONHSO₂R¹⁰, R¹⁰ represents a C1 to 4 alkyl group, a C3 to 6 cycloalkyl group, a 5 to 6-membered carbocyclic ring or 5 to 6-membered heterocyclic ring which may be substituted with 1 to 3 R¹⁶s, R¹⁶ represents halogen, a C1 to 4 alkyl group or a C1 to 4 alkoxy group, a plurality of R¹⁶s may be the same or different, R³ represents a 3 to 5-membered saturated carbocyclic ring which may be substituted with 1 to 5 R¹¹s, or a 3 to 5-membered saturated heterocyclic ring which may be substituted with 1 to 5 R¹¹s, R¹¹ represents halogen, a C1 to 4 alkyl group, a C1 to 4 alkoxy group, a C1 to 4 alkylthio group, a C1 to 4 haloalkyl group, a C1 to 4 haloalkoxy group, a hydroxyl group, a cyano group, a C1 to 4 alkylsulfonyl group, or an amino group, a plurality of R¹¹s may be the same or different, two R¹¹s may be taken together to form C1 to 3 alkylene, R⁴ and R⁵ each independently represent halogen, a C1 to 4 alkyl group, a C2 to 4 alkenyl group, or a 5 to 6-membered carbocyclic ring which may be substituted with 1 to 3 R¹⁵s or a 5 to 6-membered heterocyclic ring which may be substituted with 1 to 3 R¹⁵s, R¹⁵ represents halogen, a C1 to 4 alkyl group, or a C1 to 4 alkoxy group, a plurality of R¹⁵s may be the same or different, and carbon atoms at position 4 and position 5 of the pyrrole ring, R⁴, and R⁵ may be taken together to form a 5 to 6-membered carbocyclic ring which may be substituted with 1 to 3 R¹²s, or a 5 to 6-membered heterocyclic ring which may be substituted with 1 to 3 R¹²s, R¹² represents halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, a plurality of R¹²s may be the same or different, and R⁶ and R⁷ each independently represent a hydrogen atom, a C1 to 4 alkyl group, or a C1 to 4 haloalkyl group, and herein, each hydrogen atom may be a deuterium atom or a tritium atom; provided, however, 3-[(cyclopentylamino)methyl]-1-[(3-methylphenyl)methyl]-1H-indole-2-carboxylic acid, 3-[(cyclopentylamino)methyl]-1-[(2-fluorophenyl)methyl]-6-methyl-1H-indole-2-carboxylic acid, 3-[(cyclopentylamino)methyl]-6-methoxy-1-[(4-methylphenyl)methyl]-1H-indole-2-carboxylic acid, 3-[(cyclopentylamino)methyl]-1-ethyl-6-methoxy-1H-indole-2-carboxylic acid, 1-[(2-chloro-4-fluorophenyl)methyl]-3-[(cyclopentylamino)methyl]-6-methoxy-1H-indole-2-carboxylic acid, 1-[(2-chloro-4-fluorophenyl)methyl]-3-[(cyclopropylamino)methyl]-6-methoxy-1H-indole-2-carboxylic acid, 3 - [(cyclopentylamino)methyl] -6-methoxy-1 - [(4-methoxyphenyl)methyl] -1H-indole-2-carboxylic acid, 3-[(cyclopropylamino)methyl]-6-methoxy-1-[(4-methoxyphenyl)methyl]-1H-indole-2-carboxylic acid, 3 - [(cyclopentylamino)methyl]-1 - [(4-fluorophenyl)methyl]-6-methoxy-1H-indole-2-carboxylic acid, 3-[(cyclopentylamino)methyl]-6-methoxy-1-methyl-1H-indole-2-carboxylic acid, 3-[(cyclopentylamino)methyl]-1-[(4-methoxyphenyl)methyl]-1H-indole-2-carboxylic acid, 3-[(cyclopentylamino)methyl]-1-ethyl-6-methyl-1H-indole-2-carboxylic acid, 3-[(cyclopentylamino)methyl]-6-methoxy-1-[(2-methylphenyl)methyl]-1H-indole-2-carboxylic acid, 3 - [(cyclopentylamino)methyl] -6-methyl-1 - [(4-methylphenyl)methyl] -1H-indole-2-carboxylic acid, 3 - [(cyclopropylamino)methyl] -6-methyl-1 - [(4-methylphenyl)methyl] -1H-indole-2-carboxylic acid, 3-[(cyclopentylamino)methyl]-1-[(2-fluorophenyl)methyl]-6-methoxy-1H-indole-2-carboxylic acid, 3-[(cyclopentylamino)methyl]-1-[(4-fluorophenyl)methyl]-6-methyl-1H-indole-2-carboxylic acid, 3-[(cyclopropylamino)methyl]-1-[(4-fluorophenyl)methyl]-6-methyl-1H-indole-2-carboxylic acid, 3-[(cyclopentylamino)methyl]-1-[(3-fluorophenyl)methyl]-6-methoxy-1H-indole-2-carboxylic acid, 3-[(cyclopentylamino)methyl]-6-methyl-1-[(2-methylphenyl)methyl]-1H-indole-2-carboxylic acid, 3-[(cyclopropylammo)methyl]-6-methyl-1-[(2-methylphenyl)methyl]-1H-indole-2-carboxylic acid, 3 - [(cyclopentylamino)methyl] -1 - [(3 -fluorophenyl)methyl] -6-methyl-1H-indole-2-carboxylic acid, 3 - [(cyclopropylamino)methyl] -1 - [(3 - fluorophenyl)methyl]-6-methyl-1H-indole-2-carboxylic acid, 3-[(cyclopentylamino)methyl]-1-[(4-methoxyphenyl)methyl]-6-methyl-1H-indole-2-carboxylic acid, 3-[(cyclopentylamino)methyl]-1-[(4-ethenylphenyl)methyl]-6-methyl-1H-indole-2-carboxylic acid, 3-[(cyclopentylamino)methyl]-1-methyl-1H-indole-2-carboxylic acid, 3-[(cyclopentylamino)methyl]-1-(phenylmethyl)-1H-indole-2-carboxylic acid, 3-[(cyclopentylamino)methyl]-1-[(2-methylphenyl)methyl]-1H-indole-2-carboxylic acid, 3-[(cyclopentylamino)methyl]-1-[(4-methylphenyl)methyl]-1H-indole-2-carboxylic acid, 3-[(cyclopentylamino)methyl]-1-[(4-fluorophenyl)methyl]-1H-indole-2-carboxylic acid, 3-[(cyclopentylamino)methyl]-1-[(2,5-dimethylphenyl)methyl]-1H-indole-2-carboxylic acid, 3-[(cyclopentylamino)methyl]-1-[(4-ethenylphenyl)methyl]-1H-indole-2-carboxylic acid, 3-[(cyclopropylamino)methyl]-1-(phenylmethyl)-1H-indole-2-carboxylic acid, 3-[(cyclopropylamino)methyl]-1-[(2-methylphenyl)methyl]-1H-indole-2-carboxylic acid, 3-[(cyclopropylamino)methyl]-1-[(3-methylphenyl)methyl]-1H-indole-2-carboxylic acid, 3-[(cyclopropylamino)methyl]-1-[(4-methylphenyl)methyl]-1H-indole-2-carboxylic acid, 3-[(cyclopropylamino)methyl]-1-[(4-ethenylphenyl)methyl]-1H-indole-2-carboxylic acid, 1-[(2-chlorophenyl)methyl]-3-[(cyclopentylamino)methyl]-1H-indole-2-carboxylic acid, 1-[(2-chlorophenyl)methyl]-3-[(cyclopropylamino)methyl]-1H-indole-2-carboxylic acid, 1-[(3-chlorophenyl)methyl]-3-[(cyclopentylamino)methyl]-1H-indole-2-carboxylic acid, 1-[(4-chlorophenyl)methyl]-3-[(cyclopentylamino)methyl]-1H-indole-2-carboxylic acid, 1-[(2-chloro-4-fluorophenyl)methyl]-3-[(cyclopentylamino)methyl]-1H-indole-2-carboxylic acid, 1-[(2-chloro-4-fluorophenyl)methyl]-3-[(cyclopropylamino)methyl]-1H-indole-2 carboxylic acid, and 3-[(cyclopentylamino)methyl]-1,6-dimethyl-1H-indole-2-carboxylic acid are excluded, or a salt thereof.

3. The compound according to claim 1 or 2, or a salt thereof, wherein R³ is a cyclobutyl ring which may be substituted with 1 to 5 R¹¹s.

4. The compound according to any one of claims 1 to 3, represented by general formula (I-1): 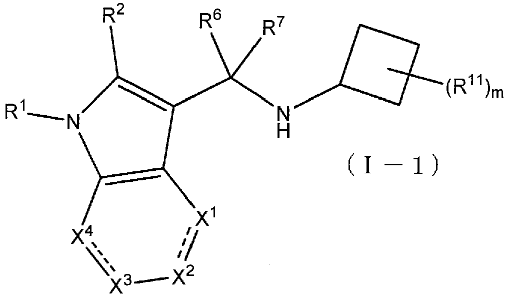
wherein X¹ represents CR¹⁴⁻¹, NR¹⁴⁻¹, or a nitrogen atom, X² represents CR¹⁴⁻², NR¹⁴⁻², or a nitrogen atom, X³ represents CR¹⁴⁻³, NR¹⁴⁻³, or a nitrogen atom, X⁴ represents CR¹⁴⁻⁴ NR¹⁴⁻⁴ or a nitrogen atom, R¹⁴⁻¹, R¹⁴⁻², R¹⁴⁻³, and R¹⁴⁻⁴ each independently represent a hydrogen atom, halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, and m represents an integer of 0 to 5, 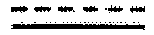
represents a single bond or a double bond, and other symbols represent the same meaning as in claim 1 or claim 2, or a salt thereof.

5. The compound according to any one of claims 1 to 4, represented by general formula (I-1-3): 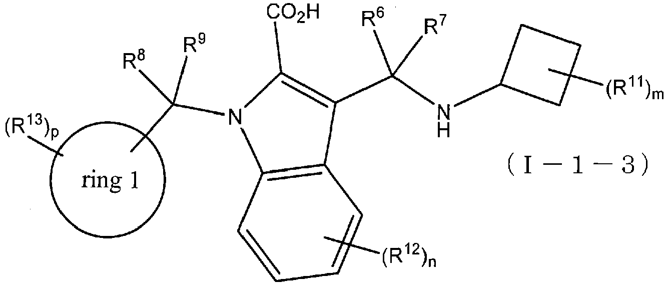 wherein p represents an integer of 0 to 5, n represents an integer of 0 to 3, and other symbols represent the same meaning as in claim 1, 2 or 4, or a salt thereof.

6. The compound according to any one of claims 1 to 5, represented by general formula (1-1-1): 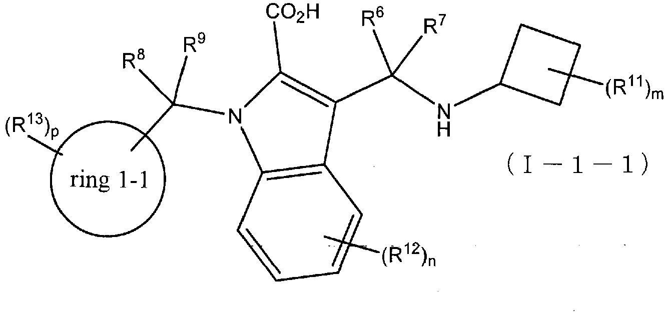 wherein ring 1-1 represents a 5 to 6-membered carbocyclic ring or a 5 to 6-membered heterocyclic ring, and other symbols represent the same meaning as in claim 1, 2, 4 or 5, or a salt thereof.

7. The compound according to claim 6, wherein ring 1-1 is a 5 to 6-membered carbocyclic ring, or a salt thereof.

8. The compound according to any one of claims 1 to 3, represented by general formula (1-2): 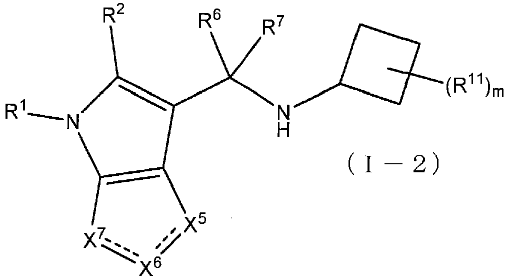 wherein X⁵ represents CR¹⁴⁻⁵, NR¹⁴⁻⁵ , a nitrogen atom, or a sulfur atom which may be oxidized, X⁶ represents CR¹⁴⁻⁶, NR¹⁴⁻⁶, or a nitrogen atom, X⁷ represents CR¹⁴⁻⁷, NR¹⁴⁻⁷, a nitrogen atom, or a sulfur atom which may be oxidized, R¹⁴⁻⁵, R¹⁴⁻⁶, and R¹⁴⁻⁷ each independently represent a hydrogen atom, halogen, a C1 to 4 alkyl group, a C2 to 4 alkenyl group, a C2 to 4 alkynyl group, a C1 to 4 alkoxy group, a C1 to 4 haloalkyl group, a C1 to 4 haloalkoxy group, a C3 to 6 cycloalkyl group, a hydroxyl group, a cyano group, a C1 to 4 alkylsulfonyl group, or a C1 to 4 dialkylphosphoryl group, m represents an integer of 0 to 5, and other symbols represent the same meaning as in claim 1 or claim 2; provided that, 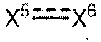 and 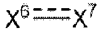 do not simultaneously represent a double bond, or a salt thereof.

9. The compound according to any one of claims 1 to 3, represented by general formula (1-3): 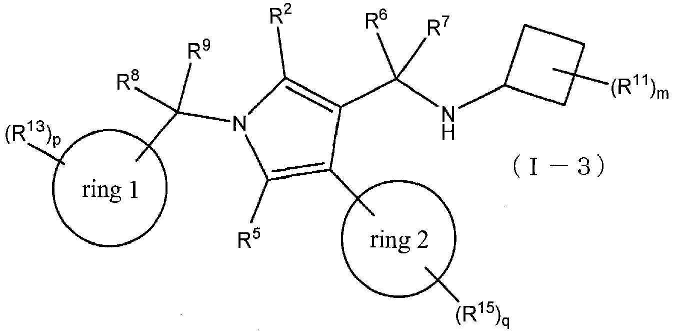 wherein ring 2 represents a 5 to 6-membered carbocyclic ring or a 5 to 6-membered heterocyclic ring, p represents an integer of 0 to 5, q represents an integer of 0 to 3, m represents an integer of 0 to 5, and other symbols represent the same meaning as in claim 1 or claim 2, or a salt thereof.

10. The compound according to any one of claims 1 to 3, represented by general formula (1-4): 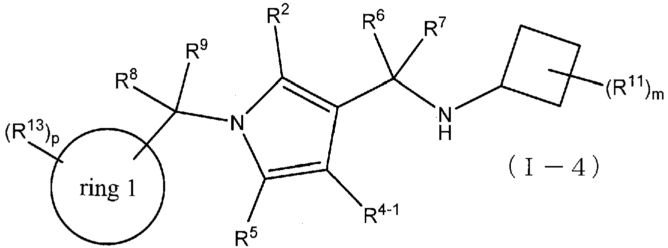 wherein R⁴⁻¹ represents halogen, a C1 to 4 alkyl group, or a C2 to 4 alkenyl group, p represents an integer of 0 to 5, m represents an integer of 0 to 5, and other symbols represent the same meaning as in claim 1 or claim 2, or a salt thereof.

11. The compound according to claim 1 or 2, wherein the compound is (1) 3-[(cyclobutylamino)methyl]-1-(cyclopropylmethyl)-1H-indole-2-carboxylic acid,
(2) 3-[(cyclobutylamino)methyl]-1-[(2,2-difluorocyclopropyl)methyl]-1H-indole-2-carboxylic acid,
(3) 3-[(cyclobutylamino)methyl]-1-ethyl-1H-indole-2-carboxylic acid,
(4) 3-[(cyclobutylamino)methyl]-1-(4-methylbenzyl)-1H-indole-2-carboxylic acid,
(5) 1-(2-chlorobenzyl)-3-[(cyclobutylamino)methyl]-1H-indole-2-carboxylic acid,
(6) 5-bromo-3-[(cyclobutylamino)methyl]-4-methyl-1-(4-methylbenzyl)-1H-pyrrole-2-carboxylic acid,
(7) 3-[(cyclobutylamino)methyl]-1-(3-cyclopropylbenzyl)-1H-indole-2-carboxylic acid,
(8) 1-(4-methylbenzyl)-3-{ [(2-methylcyclobutyl)amino]methyl} -1H-indole-2-carboxylic acid,
(9) 3-[(cyclobutylamino)methyl]-1-(4-methylbenzyl)-1H-pyrrolo[2,3-b]pyridine-2-carboxylic acid,
(10) 3-[(cyclobutylamino)methyl]-1-[2-(difluoromethoxy)benzyl]-1H-indole-2-carboxylic acid,
(11) 6-[(cyclobutylamino)methyl]-4-(4-methylbenzyl)-4H-thieno[3,2-b]pyrrole-5-carboxylic acid,
(12) 1-(4-methylbenzyl)-3-[(3-thiethanylamino)methyl]-1H-indole-2-carboxylic acid,
(13) 3 - [1 -(cyclobutylamino)ethyl] -1 -(4-methylbenzyl)-1H-indole-2-carboxylic acid,
(14) 3 - [(cyclobutylamino)methyl] -4-(3 -fluorophenyl)-5 -methyl-1 -(4-methylbenzyl)-1H-pyrrole-2-carboxylic acid,
(15) 3-[(cyclobutylamino)methyl]-5-methyl-1-(4-methylbenzyl)-4-vinyl-1H-pyrrole-2-carboxylic acid,
(16) 3-[(cyclobutylamino)methyl]-1-(4-methylbenzyl)-4,5,6,7-tetrahydro-1H-indole-2-carboxylic acid, or
(17) 7-cyano-3 - [(cyclobutylamino)methyl] -1-(4-methylbenzyl)-1H-indole-2-carboxylic acid, or a salt thereof.

12. A pharmaceutical composition comprising the compound represented by general formula (IY) or the salt thereof according to claim 1 and a pharmaceutically acceptable carrier.

13. The pharmaceutical composition according to claim 12, which is an LPA3 agonist.

14. The pharmaceutical composition according to claim 12 or 13, which is a preventive and/or therapeutic agent for an LPA3-related disease.

15. The pharmaceutical composition according to claim 14, wherein the LPA3-related disease is essential thrombocythemia, reactive thrombocytosis, aplastic anemia, myelodysplastic syndrome, or sepsis.

16. A method for preventing and/or treating an LPA3-related disease, comprising administering an effective amount of the compound represented by general formula (IY) or the salt thereof according to claim 1 to a mammal.

17. The compound represented by general formula (IY) or the salt thereof according to claim 1, which is used for prevention and/or treatment of an LPA3-related disease.

18. A use of the compound represented by general formula (IY) or the salt thereof according to claim 1, for producing a preventive and/or therapeutic agent for an LPA3-related disease.
